# EUROPEAN PATENT APPLICATION

(11) **EP 4 502 151 A1**
(43) Date of publication of application: **05.02.2025**
(21) Application number: 23781079.1
(22) Date of filing: 31.03.2023
(51) Int. Cl.: C12N 7/00, A01N 63/40, A01P 3/00, C12Q 1/04, C12Q 1/70, C07K 14/01, C12N 15/34, C12R 1/64

(54) **GENUS XANTHOMONAS BACTERIOLYTIC BACTERIOPHAGE**

(30) Priority: 31.03.2022 JP 2022060819; 31.03.2022 JP 2022060249; 31.03.2022 JP 2022059936; 31.03.2022 JP 2022060143; 31.03.2022 JP 2022060441; 31.03.2022 JP 2022061075; 31.03.2022 JP 2022060887; 31.03.2022 JP 2022060822; 31.03.2022 JP 2022060909; 29.09.2022 JP 2022156882; 29.09.2022 JP 2022156972; 29.09.2022 JP 2022157086; 16.03.2023 JP 2023041699; 16.03.2023 JP 2023041776; 16.03.2023 JP 2023041784; 16.03.2023 JP 2023041827
(71) Applicant: Kaneka Corporation, Osaka-shi, Osaka 530-8288 (JP); Rakuno Gakuen University, Ebetsu-shi, Hokkaido 069-8501 (JP); Mitsui & Co., Ltd., Chiyoda-Ku Tokyo 100-8631 (JP)
(72) Inventor: YOSHIDA Shinichi, Iwata-shi, Shizuoka 438-0802 (JP); DOJUN Nobuhiko, Iwata-shi, Shizuoka 438-0802 (JP); KITANO Mitsuaki, Takasago-shi, Hyogo 676-8688 (JP); IWANO Hidetomo, Ebetsu-shi, Hokkaido 069-8501 (JP); KUDO Hitoshi, Tokyo 100-8631 (JP)
(74) Representative: Hoffmann Eitle
(86) International application number: PCT/JP2023/013610
(87) International publication number: WO 2023/191071

(57) **Abstract**

To control plant diseases caused by *Xanthomonas* spp., a novel bacteriophage exhibiting bacteriolysis activity specifically on *Xanthomonas* spp. was isolated. Thus, developed and provided is a plant disease control composition containing the bacteriophage as an active ingredient. Provided are a bacteriolytic agent comprising a bacteriophage that has a novel genomic DNA sequence and exhibits bacteriolysis activity specifically on *Xanthomonas* spp., and a plant disease control composition containing the same as an active ingredient.

## Description

### Technical Field

The present invention relates to a bacteriolytic agent composed of a bacteriophage, a composition for plant disease control comprising the same, and a method for controlling plant disease.

### Background Art

A bacteriophage (herein often abbreviated simply as a "phage") is a generic term for viruses that infect only bacteria. Many phages, also called bacteriolytic phages, are specifically attached to target bacteria as a host, then inject their own DNA, and are selfamplified utilizing the translational mechanism of the bacteria. Furthermore, the bacteria are lysed, and consequently, the amplified phages are diffused, and an infection into new target bacteria is repeated (Non-Patent Literature 1).

Many of the bacteria of the *Xanthomonas* genus are known to be pathogenic bacteria for diseases of plants, for example, various crops. In common customary control, a copper fungicide or an antibiotic has been used. However, there are many problems, such as a drug effect, drug poisoning, and as well as possible causes for the disruption of a bacterial lawn balance. Hence, recent interest has focused on a method using a phage as a new control method (Non-Patent Literature 2).

An example of a phage bacteriolytic to bacteria of the *Xanthomonas* genus is reported, for example, in Patent Literature 1 to 3 and Non-Patent Literature 2. However, the host range of the phages is extremely narrow. Hence, it is still important to search for a new phage and to find a phage having higher bacteriolytic ability.

### Citation List

### Patent Literature

Patent Literature 1: JP2016-32435A
Patent Literature 2: JP2021-102635A
Patent Literature 3: WO2017/113029

### Non-Patent Literature

Non-Patent Literature 1: Sharma S. et al., FoliaMicrobiol., 2017, 62:17-55
Non-Patent Literature 2: Nakayinga R. et al., BMC Microbiology, 2021, 21:291

### Summary of Invention

### Technical Problem

To solve the above-described problems, the present inventors have aimed their attention at a bacteriophage. Differently from a conventional copper fungicide or antibiotic, a phage, which is a virus, is a natural product, and the manifestation of its drug poisoning has hitherto not been reported. Additionally, a phage has a very high specificity to a host, and thus, only targets bacteria of a specific genus or species, having an extremely limited influence on a bacterial lawn balance. Additionally, a phage is harmless to not only an animal such as a human but also a plant, and is very safe.

Accordingly, to inhibit damage causable to crops by a plant disease caused by bacteria of the *Xanthomonas* genus, a novel phage that exhibits bacteriolytic ability against bacteria of the *Xanthomonas* genus is isolated. The further object is to provide a composition comprising the phage as an active component, and to use the composition for disease control, the detection of pathogenic bacteria, and the like.

### Solution to Problem

The present inventors have isolated a novel phage from natural dirty water and soil, using a method for detecting a bacteriolytic plaque formed on a soft agar medium having bacteria of the *Xanthomonas* genus cultured thereon, have evaluated the bacteriolytic ability of the phage against various bacteria of the *Xanthomonas* genus, and have analyzed the genomic sequence of the phage. Consequently, the phage has been revealed as having a novel genomic DNA sequence. The present invention has been completed on the basis of the above-described results of research and development, and specifically provides the following.
(1-1) A bacteriolytic agent consisting of a bacteriophage comprising, in the genomic DNA, a gene encoding a tail fiber protein having a recognizing ability for target bacteria, wherein the protein consists of the amino acid sequence of any one of the following (a) to (c): (a) the amino acid sequence of SEQ ID NO: 1; (b) the amino acid sequence having addition, deletion, and/or substitution of one or a plurality of amino acids in the amino acid sequence of SEQ ID NO: 1; (c) an amino acid sequence having a sequence identity of 90% or more to the amino acid sequence of SEQ ID NO: 1.
(1-2) The bacteriolytic agent according to (1-1), wherein the amino acid sequence of SEQ ID NO: 1 is the amino acid sequence of any one of SEQ ID NOs: 2 to 4.
(1-3) The bacteriolytic agent according to (1-1) or (1-2), wherein the gene consists of the nucleotide sequence of any one of the following (d) to (f): (d) the nucleotide sequence of any one of SEQ ID NOs: 5 to 7; (e) the nucleotide sequence having addition, deletion, and/or substitution of one or a plurality of nucleotides in the nucleotide sequence of any one of SEQ ID NOs: 5 to 7; (f) a nucleotide sequence having a sequence identity of 90% or more to the nucleotide sequence of any one of SEQ ID NOs: 5 to 7.
(1-4) The bacteriolytic agent according to any of (1-1) to (1-3), wherein the nucleotide sequence of the genomic DNA consists of the nucleotide sequence of any one of the following (g) to (k): (g) the nucleotide sequence of any one of SEQ ID NOs: 8 to 10; (h) the nucleotide sequence having addition, deletion, and/or substitution of one or a plurality of nucleotides in the nucleotide sequence other than the nucleotide sequence of the gene according to any of (1-1) to (1-3) within the nucleotide sequence of any one of SEQ ID NOs: 8 to 10; (i) a nucleotide sequence having a sequence identity of 80% or more to the nucleotide sequence other than the nucleotide sequence of the gene according to any of (1-1) to (1-3) within the nucleotide sequence of any one of SEQ ID NOs: 8 to 10; (j) the nucleotide sequence having addition, deletion, and/or substitution of one or a plurality of nucleotides in the nucleotide sequence of any one of SEQ ID NOs: 8 to 10; (k) a nucleotide sequence having a sequence identity of 90% or more to the nucleotide sequence of any one of SEQ ID NOs: 8 to 10.
(1-5) The bacteriolytic agent according to any of (1-1) to (1-4), wherein the bacteriolytic agent exhibits bacteriolytic ability against bacteria of the *Xanthomonas* genus.
(1-6) The bacteriolytic agent according to (1-5), wherein the bacteria of the *Xanthomonas* genus are at least one kind of bacteria selected from the group consisting of *Xanthomonas arboricola, Xanthomonas campestris, Xanthomonas citri, Xanthomonas oryzae,* and *Xanthomonas cucurbitae.*
(2-1) A bacteriolytic agent consisting of a bacteriophage comprising, in the genomic DNA, a gene encoding a tail fiber protein having a recognizing ability for target bacteria, wherein the protein consists of the amino acid sequence of any one of the following (a) and (b): (a) the amino acid sequence of SEQ ID NO: 11 or 45; (b) the amino acid sequence having substitution of one amino acid other than at positions 278 and 350 in the amino acid sequence of SEQ ID NO: 11.
(2-2) The bacteriolytic agent according to (2-1), wherein the gene consists of the nucleotide sequence of SEQ ID NO: 12 or 46.
(2-3) The bacteriolytic agent according to (2-1) or (2-2), wherein the nucleotide sequence of the genomic DNA consists of the nucleotide sequence of any one of the following (a) to (e): (a) the nucleotide sequence of SEQ ID NO: 13, 47, or 48; (b) the nucleotide sequence having addition, deletion, and/or substitution of one or a plurality of nucleotides in the nucleotide sequence other than the nucleotide sequence of the gene according to (2-1) or (2-2) within the nucleotide sequence of SEQ ID NO: 13, 47, or 48; (c) a nucleotide sequence having a sequence identity of 98.5% or more to the nucleotide sequence other than the nucleotide sequence of the gene according to (2-1) or (2-2) within the nucleotide sequence of SEQ ID NO: 13, 47, or 48; (d) the nucleotide sequence having addition, deletion, and/or substitution of one or a plurality of nucleotides in the nucleotide sequence of SEQ ID NO: 13, 47, or 48; (e) a nucleotide sequence having a sequence identity of 98.5% or more to the nucleotide sequence of SEQ ID NO: 13, 47, or 48.
(2-4) The bacteriolytic agent according to any of (2-1) to (2-3), wherein the bacteriolytic agent exhibits bacteriolytic ability against bacteria of the *Xanthomonas* genus.
(2-5) The bacteriolytic agent according to (2-4), wherein the bacteria of the *Xanthomonas* genus are at least one kind of bacteria selected from the group consisting of *Xanthomonas arboricola, Xanthomonas campestris, Xanthomonas citri, Xanthomonas oryzae,* and *Xanthomonas cucurbitae.*
(3-1) A bacteriolytic agent consisting of a bacteriophage having a genomic DNA sequence comprising the nucleotide sequence of any one of the following (a) to (c): (a) the nucleotide sequence of SEQ ID NO: 14; (b) the nucleotide sequence having addition, deletion, and/or substitution of one or a plurality of nucleotides in the nucleotide sequence of SEQ ID NO: 14; (c) a nucleotide sequence having a sequence identity of 90% or more to the nucleotide sequence of SEQ ID NO: 14.
(3-2) The bacteriolytic agent according to (3-1), wherein the bacteriolytic agent exhibits bacteriolytic ability against bacteria of the *Xanthomonas* genus.
(3-3) The bacteriolytic agent according to (3-2), wherein the bacteria of the *Xanthomonas* genus are *Xanthomonas arboricola.*
(4-1) A bacteriolytic agent consisting of a bacteriophage comprising, in the genomic DNA, a gene encoding a tail fiber protein having a recognizing ability for target bacteria, wherein the protein consists of the amino acid sequence of any one of the following (a) to (c): (a) the amino acid sequence of SEQ ID NO: 15; (b) the amino acid sequence having addition, deletion, and/or substitution of one or a plurality of amino acids in the amino acid sequence of SEQ ID NO: 15; (c) an amino acid sequence having a sequence identity of 90% or more to the amino acid sequence of SEQ ID NO: 15.
(4-2) The bacteriolytic agent according to (4-1), wherein the gene consists of the nucleotide sequence of any one of the following (d) to (f): (d) the nucleotide sequence of SEQ ID NO: 16; (e) the nucleotide sequence having addition, deletion, and/or substitution of one or a plurality of nucleotides in the nucleotide sequence of SEQ ID NO: 16; (f) a nucleotide sequence having a sequence identity of 90% or more to the nucleotide sequence of SEQ ID NO: 16.
(4-3) The bacteriolytic agent according to (4-1) or (4-2), wherein the nucleotide sequence of the genomic DNA consists of the nucleotide sequence of any one of the following (g) to (k): (g) the nucleotide sequence of SEQ ID NO: 17; (h) the nucleotide sequence having addition, deletion, and/or substitution of one or a plurality of nucleotides in the nucleotide sequence other than the nucleotide sequence of the gene according to (4-1) or (4-2) within the nucleotide sequence of SEQ ID NO: 17; (i) a nucleotide sequence having a sequence identity of 80% or more to the nucleotide sequence other than the nucleotide sequence of the gene according to (4-1) or (4-2) within the nucleotide sequence of SEQ ID NO: 17; (j) the nucleotide sequence having addition, deletion, and/or substitution of one or a plurality of nucleotides in the nucleotide sequence of SEQ ID NO: 17; (k) a nucleotide sequence having a sequence identity of 90% or more to the nucleotide sequence of SEQ ID NO: 17.
(4-4) The bacteriolytic agent according to any of (4-1) to (4-3), wherein the bacteriolytic agent exhibits bacteriolytic ability against bacteria of the *Xanthomonas* genus.
(4-5) The bacteriolytic agent according to (4-4), wherein the bacteria of the *Xanthomonas* genus are *Xanthomonas arboricola.*
(5-1) A bacteriolytic agent comprising a bacteriophage having a genomic DNA sequence comprising the nucleotide sequence of any one of the following (a) to (c): (a) the nucleotide sequence of SEQ ID NO: 18 or 19; (b) the nucleotide sequence having addition, deletion, and/or substitution of one or a plurality of nucleotides in the nucleotide sequence of SEQ ID NO: 18 or 19; (c) a nucleotide sequence having a sequence identity of 90% or more to the nucleotide sequence of SEQ ID NO: 18 or 19.
(5-2) The bacteriolytic agent according to (5-1), wherein the bacteriolytic agent exhibits bacteriolytic ability against bacteria of the *Xanthomonas* genus.
(5-3) The bacteriolytic agent according to (5-2), wherein the bacteria of the *Xanthomonas* genus are *Xanthomonas arboricola.*
(6-1) A bacteriolytic agent consisting of a bacteriophage comprising, in the genomic DNA, a gene encoding a protein consisting of the amino acid sequence of any one of the following (a) to (c): (a) the amino acid sequence of SEQ ID NO: 21; (b) the amino acid sequence having addition, deletion, and/or substitution of one or a plurality of amino acids in the amino acid sequence of SEQ ID NO: 21; (c) an amino acid sequence having a sequence identity of 90% or more to the amino acid sequence of SEQ ID NO: 21.
(6-2) The bacteriolytic agent according to (6-1), wherein the gene consists of the nucleotide sequence of any one of the following (d) to (f): (d) the nucleotide sequence of SEQ ID NO: 22; (e) the nucleotide sequence having addition, deletion, and/or substitution of one or a plurality of nucleotides in the nucleotide sequence of SEQ ID NO: 22; (f) a nucleotide sequence having a sequence identity of 90% or more to the nucleotide sequence of SEQ ID NO: 22.
(6-3) The bacteriolytic agent according to (6-1) or (6-2), wherein the nucleotide sequence of the genomic DNA consists of the nucleotide sequence of any one of the following (g) to (k): (g) the nucleotide sequence of SEQ ID NO: 23; (h) the nucleotide sequence having addition, deletion, and/or substitution of one or a plurality of nucleotides in the nucleotide sequence other than the nucleotide sequence of the gene according to (6-1) or (6-2) within the nucleotide sequence of SEQ ID NO: 23; (i) a nucleotide sequence having a sequence identity of 95% or more to the nucleotide sequence other than the gene according to (6-1) or (6-2) within the nucleotide sequence of SEQ ID NO: 23; (j) the nucleotide sequence having addition, deletion, and/or substitution of one or a plurality of nucleotides in the nucleotide sequence of SEQ ID NO: 23; (k) a nucleotide sequence having a sequence identity of 95% or more to the nucleotide sequence of SEQ ID NO: 23.
(6-4) The bacteriolytic agent according to any of (6-1) to (6-3), wherein the bacteriolytic agent exhibits bacteriolytic ability against bacteria of the *Xanthomonas* genus.
(6-5) The bacteriolytic agent according to (6-4), wherein the bacteria of the *Xanthomonas* genus are *Xanthomonas arboricola.*
(7-1) A bacteriolytic agent consisting of a bacteriophage comprising, in the genomic DNA, the following: a gene encoding a tail tubular protein A consisting of the amino acid sequence of any one of the following (a) to (c) and having a recognizing ability for a target bacteria; and a gene encoding a tail tubular protein B consisting of the amino acid sequence of any one of the following (d) to (f) and having a recognizing ability for a target bacteria: (a) the amino acid sequence of SEQ ID NO: 24, 49, or 60; (b) the amino acid sequence having addition, deletion, and/or substitution of one or a plurality of amino acids in the amino acid sequence of SEQ ID NO: 24 or 49; (c) an amino acid sequence having a sequence identity of 97% or more to the amino acid sequence of SEQ ID NO: 24 or 49; (d) the amino acid sequence of SEQ ID NO: 57, 50, or 61; (e) the amino acid sequence having addition, deletion, and/or substitution of one or a plurality of amino acids in the amino acid sequence of SEQ ID NO: 57 or 50; (f) an amino acid sequence having a sequence identity of 97% or more to the amino acid sequence of SEQ ID NO: 57 or 50.
(7-2) The bacteriolytic agent according to (7-1), wherein the gene encoding the tail tubular protein A consists of the nucleotide sequence of any one of the following (g) to (i): (g) the nucleotide sequence of SEQ ID NO: 26 or 51; (h) the nucleotide sequence having addition, deletion, and/or substitution of one or a plurality of nucleotides in the nucleotide sequence of SEQ ID NO: 26 or 51; (i) a nucleotide sequence having a sequence identity of 97% or more to the nucleotide sequence of SEQ ID NO: 26 or 51.
(7-3) The bacteriolytic agent according to (7-1) or (7-2), wherein the gene encoding the tail tubular protein B consists of the nucleotide sequence of any one of the following (j) to (l): (j) the nucleotide sequence of SEQ ID NO: 27 or 52; (k) the nucleotide sequence having addition, deletion, and/or substitution of one or a plurality of nucleotides in the nucleotide sequence of SEQ ID NO: 27 or 52; (l) a nucleotide sequence having a sequence identity of 97% or more to the nucleotide sequence of SEQ ID NO: 27 or 52.
(7-4) The bacteriolytic agent according to any of (7-1) to (7-3), wherein the nucleotide sequence of the genomic DNA consists of the nucleotide sequence of any one of the following (m) to (q): (m) the nucleotide sequence of SEQ ID NO: 28, 29, or 53; (n) the nucleotide sequence having addition, deletion, and/or substitution of one or a plurality of nucleotides in the nucleotide sequence other than the nucleotide sequence of the gene according to any of (7-1) to (7-3) within the nucleotide sequence of SEQ ID NO: 28, 29, or 53; (o) a nucleotide sequence having a sequence identity of 95% or more to the nucleotide sequence other than the gene according to any of (7-1) to (7-3) within the nucleotide sequence of SEQ ID NO: 28, 29, or 53; (p) the nucleotide sequence having addition, deletion, and/or substitution of one or a plurality of nucleotides in the nucleotide sequence of SEQ ID NO: 28, 29, or 53; (q) a nucleotide sequence having a sequence identity of 95% or more to the nucleotide sequence of SEQ ID NO: 28, 29, or 53.
(7-5) The bacteriolytic agent according to any of (7-1) to (7-4), wherein the bacteriolytic agent exhibits bacteriolytic ability against bacteria of the *Xanthomonas* genus.
(7-6) The bacteriolytic agent according to (7-5), wherein the bacteria of the *Xanthomonas* genus are *Xanthomonas arboricola* or *Xanthomonas campestris.*
(8-1) A bacteriolytic agent consisting of a bacteriophage having a genomic DNA sequence comprising the nucleotide sequence of any one of the following (a) to (c): (a) the nucleotide sequence of any one of SEQ ID NOs: 32 to 36; (b) the nucleotide sequence having addition, deletion, and/or substitution of one or a plurality of nucleotides in the nucleotide sequence of any one of SEQ ID NOs: 32 to 36; (c) a nucleotide sequence having a sequence identity of 90% or more to the nucleotide sequence of any one of SEQ ID NOs: 32 to 36.
(8-2) The bacteriolytic agent according to (8-1), wherein the bacteriolytic agent exhibits bacteriolytic ability against bacteria of the *Xanthomonas* genus.
(8-3) The bacteriolytic agent according to (8-2), wherein the bacteria of the *Xanthomonas* genus are *Xanthomonas arboricola.*
(9-1) A bacteriolytic agent consisting of a bacteriophage comprising, in the genomic DNA, the following: a gene encoding a tail tubular protein A consisting of the amino acid sequence of any one of the following (a) to (c) and having a recognizing ability for a target bacteria; and a gene encoding a tail tubular protein B consisting of the amino acid sequence of any one of the following (d) to (f) and having a recognizing ability for a target bacteria: (a) the amino acid sequence of SEQ ID NO: 37; (b) the amino acid sequence having addition, deletion, and/or substitution of one or a plurality of amino acids in the amino acid sequence of SEQ ID NO: 37; (c) an amino acid sequence having a sequence identity of 92% or more to the amino acid sequence of SEQ ID NO: 37; (d) the amino acid sequence of SEQ ID NO: 38, 54, or 59; (e) the amino acid sequence having addition, deletion, and/or substitution of one or a plurality of amino acids in the amino acid sequence of SEQ ID NO: 38 or 54; (f) an amino acid sequence having a sequence identity of 92% or more to the amino acid sequence of SEQ ID NO: 38 or 54.
(9-2) The bacteriolytic agent according to (9-1), wherein the gene encoding the tail tubular protein A consists of the nucleotide sequence of any one of the following (g) to (i): (g) the nucleotide sequence of SEQ ID NO: 39; (h) the nucleotide sequence having addition, deletion, and/or substitution of one or a plurality of nucleotides in the nucleotide sequence of SEQ ID NO: 39; (i) a nucleotide sequence having a sequence identity of 90% or more to the nucleotide sequence of SEQ ID NO: 39.
(9-3) The bacteriolytic agent according to (9-1) or (9-2), wherein the gene encoding the tail tubular protein B consists of the nucleotide sequence of any one of the following (j) to (l): (j) the nucleotide sequence of SEQ ID NO: 40 or 55; (k) the nucleotide sequence having addition, deletion, and/or substitution of one or a plurality of nucleotides in the nucleotide sequence of SEQ ID NO: 40 or 55; (l) a nucleotide sequence having a sequence identity of 90% or more to the nucleotide sequence of SEQ ID NO: 40 or 55.
(9-4) The bacteriolytic agent according to any of (9-1) to (9-3), wherein the nucleotide sequence of the genomic DNA consists of the nucleotide sequence of any one of the following (m) to (q): (m) the nucleotide sequence of SEQ ID NO: 41 or 56; (n) the nucleotide sequence having addition, deletion, and/or substitution of one or a plurality of nucleotides in the nucleotide sequence other than the nucleotide sequence of the gene according to any of (9-1) to (9-3) within the nucleotide sequence of SEQ ID NO: 41 or 56; (o) a nucleotide sequence having a sequence identity of 80% or more to the nucleotide sequence other than the nucleotide sequence of the gene according to any of (9-1) to (9-3) within the nucleotide sequence of SEQ ID NO: 41 or 56; (p) the nucleotide sequence having addition, deletion, and/or substitution of one or a plurality of nucleotides in the nucleotide sequence of SEQ ID NO: 41 or 56; (q) a nucleotide sequence having a sequence identity of 90% or more to the nucleotide sequence of SEQ ID NO: 41 or 56.
(9-5) The bacteriolytic agent according to any of (9-1) to (9-4), wherein the bacteriolytic agent exhibits bacteriolytic ability against bacteria of the *Xanthomonas* genus.
(9-6) The bacteriolytic agent according to (9-5), wherein the bacteria of the *Xanthomonas* genus are at least one kind of bacteria selected from the group consisting of *Xanthomonas arboricola, Xanthomonas campestris,* and *Xanthomonas citri.*
(10-1) A bacteriolytic agent consisting of a bacteriophage comprising, in the genomic DNA, a gene encoding a tail fiber protein having a recognizing ability for target bacteria, wherein the protein consists of the amino acid sequence of any one of the following (a) to (c): (a) the amino acid sequence of SEQ ID NO: 42; (b) the amino acid sequence having addition, deletion, and/or substitution of one or a plurality of amino acids in the amino acid sequence of SEQ ID NO: 42; (c) an amino acid sequence having a sequence identity of 90% or more to the amino acid sequence of SEQ ID NO: 42.
(10-2) The bacteriolytic agent according to (10-1), wherein the gene consists of the nucleotide sequence of any one of the following (d) to (f): (d) the nucleotide sequence of SEQ ID NO: 43; (e) the nucleotide sequence having addition, deletion, and/or substitution of one or a plurality of nucleotides in the nucleotide sequence of SEQ ID NO: 43; (f) a nucleotide sequence having a sequence identity of 90% or more to the nucleotide sequence of SEQ ID NO: 43.
(10-3) The bacteriolytic agent according to (10-1) or (10-2), wherein the nucleotide sequence of the genomic DNA consists of the nucleotide sequence of any one of the following (g) to (k): (g) the nucleotide sequence of SEQ ID NO: 44; (h) the nucleotide sequence having addition, deletion, and/or substitution of one or a plurality of nucleotides in the nucleotide sequence other than the nucleotide sequence of the gene according to (10-1) or (10-2) within the nucleotide sequence of SEQ ID NO: 44; (i) a nucleotide sequence having a sequence identity of 80% or more to the nucleotide sequence other than the nucleotide sequence of the gene according to (10-1) or (10-2) within the nucleotide sequence of SEQ ID NO: 44; (j) the nucleotide sequence having addition, deletion, and/or substitution of one or a plurality of nucleotides in the nucleotide sequence of SEQ ID NO: 44; (k) a nucleotide sequence having a sequence identity of 90% or more to the nucleotide sequence of SEQ ID NO: 44.
(10-4) The bacteriolytic agent according to any of (10-1) to (10-3), wherein the bacteriolytic agent exhibits bacteriolytic ability against bacteria of the *Xanthomonas* genus.
(10-5) The bacteriolytic agent according to (10-4), wherein the bacteria of the *Xanthomonas* genus are at least one kind of bacteria selected from the group consisting of *Xanthomonas arboricola, Xanthomonas campestris,* and *Xanthomonas citri.*
[1-1] A bacteriolytic agent consisting of a bacteriophage comprising, in the genomic DNA, a gene encoding a tail fiber protein having a recognizing ability for target bacteria, wherein the protein consists of the amino acid sequence of any one of the following (a) to (d): (a) the amino acid sequence of any one selected from the group consisting of SEQ ID NOs: 11, 45, 42, and 1; (b) the amino acid sequence having addition, deletion, and/or substitution of one or a plurality of amino acids in the amino acid sequence of SEQ ID NO: 42 or 1; and (c) an amino acid sequence having a sequence identity of 90% or more to the amino acid sequence of SEQ ID NO: 42 or 1; or (d) the amino acid sequence having substitution of one amino acid other than at positions 278 and 350 in the amino acid sequence of SEQ ID NO: 11.
[1-2] A bacteriolytic agent consisting of a bacteriophage comprising, in the genomic DNA, the following: a gene consisting of the amino acid sequence of any one of the following (e) to (g); and a gene consisting of the amino acid sequence of any one of the following (h) to (j), wherein the genes are respectively a gene encoding a tail tubular protein A and a gene encoding a tail tubular protein B, both proteins having a recognizing ability for target bacteria: (e) the amino acid sequence of SEQ ID NO: 24, 49, or 60; (f) the amino acid sequence having addition, deletion, and/or substitution of one or a plurality of amino acids in the amino acid sequence of SEQ ID NO: 24 or 49; (g) an amino acid sequence having a sequence identity of 97% or more to the amino acid sequence of SEQ ID NO: 24 or 49; (h) the amino acid sequence of SEQ ID NO: 57, 50, or 61; (i) the amino acid sequence having addition, deletion, and/or substitution of one or a plurality of amino acids in the amino acid sequence of SEQ ID NO: 57 or 50; or (j) an amino acid sequence having a sequence identity of 97% or more to the amino acid sequence of SEQ ID NO: 57 or 50.
[1-3] A bacteriolytic agent consisting of a bacteriophage comprising, in the genomic DNA, the following: a gene consisting of the amino acid sequence of any one of the following (k) to (m); and a gene consisting of the amino acid sequence of any one of the following (n) to (p), wherein the genes are respectively a gene encoding a tail tubular protein A and a gene encoding a tail tubular protein B, both proteins having a recognizing ability for target bacteria: (k) the amino acid sequence of SEQ ID NO: 37; (l) the amino acid sequence having addition, deletion, and/or substitution of one or a plurality of amino acids in the amino acid sequence of SEQ ID NO: 37; (m) an amino acid sequence having a sequence identity of 92% or more to the amino acid sequence of SEQ ID NO: 37; (n) the amino acid sequence of SEQ ID NO: 38, 54, or 59; (o) the amino acid sequence having addition, deletion, and/or substitution of one or a plurality of amino acids in the amino acid sequence of SEQ ID NO: 38 or 54; or (p) an amino acid sequence having a sequence identity of 92% or more to the amino acid sequence of SEQ ID NO: 38 or 54.
[1-4] The bacteriolytic agent according to [1-1], wherein the amino acid sequence of SEQ ID NO: 1 is the amino acid sequence of any one of SEQ ID NOs: 2 to 4.
[1-5] The bacteriolytic agent according to [1-1], wherein the gene consists of the nucleotide sequence of any one of the following (1) to (3): (1) the nucleotide sequence of any one selected from the group consisting of SEQ ID NOs: 12, 46, 43, and 5 to 7; (2) the nucleotide sequence having addition, deletion, and/or substitution of one or a plurality of nucleotides in the nucleotide sequence of any one selected from the group consisting of SEQ ID NOs: 43 and 5 to 7; or (3) a nucleotide sequence having a sequence identity of 90% or more to the nucleotide sequence of any one selected from the group consisting of SEQ ID NOs: 43 and 5 to 7.
[1-6] The bacteriolytic agent according to [1-5], wherein the nucleotide sequence of the genomic DNA consists of the nucleotide sequence of any one of the following (1') to (7'): (1') the nucleotide sequence of any one selected from the group consisting of SEQ ID NOs: 13, 47, 48, 44, and 8 to 10; (2') the nucleotide sequence having addition, deletion, and/or substitution of one or a plurality of nucleotides in the nucleotide sequence other than the gene according to [1-5] within the nucleotide sequence of any one selected from the group consisting of SEQ ID NOs: 13, 47, 48, 44, and 8 to 10; (3') a nucleotide sequence having a sequence identity of 80% or more to the nucleotide sequence other than the gene according to [1-5] within the nucleotide sequence of any one selected from the group consisting of SEQ ID NOs: 44 and 8 to 10; (4') a nucleotide sequence having a sequence identity of 98.5% or more to the nucleotide sequence other than the gene according to [1-5] within the nucleotide sequence of SEQ ID NO: 13, 47, or 48; (5') the nucleotide sequence having addition, deletion, and/or substitution of one or a plurality of nucleotides in the nucleotide sequence of any one selected from the group consisting of SEQ ID NOs: 13, 47, 48, 44, and 8 to 10; or (6') a nucleotide sequence having a sequence identity of 90% or more to the nucleotide sequence of any one selected from the group consisting of SEQ ID NOs: 44 and 8 to 10; (7') a nucleotide sequence having a sequence identity of 98.5% or more to the nucleotide sequence of SEQ ID NO: 13, 47, or 48.
[1-7] The bacteriolytic agent according to [1-2], wherein the gene encoding the tail tubular protein A consists of the nucleotide sequence of any one of the following (4) to (6): (4) the nucleotide sequence of SEQ ID NO: 26 or 51; (5) the nucleotide sequence having addition, deletion, and/or substitution of one or a plurality of nucleotides in the nucleotide sequence of SEQ ID NO: 26 or 51; or (6) a nucleotide sequence having a sequence identity of 97% or more to the nucleotide sequence of SEQ ID NO: 26 or 51.
[1-8] The bacteriolytic agent according to [1-2] or [1-7], wherein the gene encoding the tail tubular protein B consists of the nucleotide sequence of any one of the following (7) to (9): (7) the nucleotide sequence of SEQ ID NO: 27 or 52; (8) the nucleotide sequence having addition, deletion, and/or substitution of one or a plurality of nucleotides in the nucleotide sequence of SEQ ID NO: 27 or 52; or (9) a nucleotide sequence having a sequence identity of 97% or more to the nucleotide sequence of SEQ ID NO: 27 or 52.
[1-9] The bacteriolytic agent according to [1-2], wherein the nucleotide sequence of the genomic DNA consists of the nucleotide sequence of any one of the following (8') to (12'): (8') the nucleotide sequence of SEQ ID NO: 28, 29, or 53; (9') the nucleotide sequence having addition, deletion, and/or substitution of one or a plurality of nucleotides in the nucleotide sequence other than the gene according to [1-2] within the nucleotide sequence of SEQ ID NO: 28, 29, or 53; (10') a nucleotide sequence having a sequence identity of 95% or more to the nucleotide sequence other than the gene according to [1-2] within the nucleotide sequence of SEQ ID NO: 28, 29, or 53; (11') the nucleotide sequence having addition, deletion, and/or substitution of one or a plurality of nucleotides in the nucleotide sequence of SEQ ID NO: 28, 29, or 53; or (12') a nucleotide sequence having a sequence identity of 95% or more to the nucleotide sequence of SEQ ID NO: 28, 29, or 53.
[1-10] The bacteriolytic agent according to [1-3], wherein the gene encoding the tail tubular protein A consists of the nucleotide sequence of any one of the following (10) to (12): (10) the nucleotide sequence of SEQ ID NO: 39; (11) the nucleotide sequence having addition, deletion, and/or substitution of one or a plurality of nucleotides in the nucleotide sequence of SEQ ID NO: 39; or (12) a nucleotide sequence having a sequence identity of 90% or more to the nucleotide sequence of SEQ ID NO: 39.
[1-11] The bacteriolytic agent according to [1-3] or [1-10], wherein the gene encoding the tail tubular protein B consists of the nucleotide sequence of any one of the following (13) to (15): (13) the nucleotide sequence of SEQ ID NO: 40 or 55; (14) the nucleotide sequence having addition, deletion, and/or substitution of one or a plurality of nucleotides in the nucleotide sequence of SEQ ID NO: 40 or 55; or (15) a nucleotide sequence having a sequence identity of 90% or more to the nucleotide sequence of SEQ ID NO: 40 or 55.
[1-12] The bacteriolytic agent according to [1-3], wherein the nucleotide sequence of the genomic DNA consists of the nucleotide sequence of any one of the following (13') to (17'): (13') the nucleotide sequence of SEQ ID NO: 41 or 56; (14') the nucleotide sequence having addition, deletion, and/or substitution of one or a plurality of nucleotides in the nucleotide sequence other than the gene according to [1-3] within the nucleotide sequence of SEQ ID NO: 41 or 56; (15') a nucleotide sequence having a sequence identity of 80% or more to the nucleotide sequence other than the gene according to [1-3] within the nucleotide sequence of SEQ ID NO: 41 or 56; (16') the nucleotide sequence having addition, deletion, and/or substitution of one or a plurality of nucleotides in the nucleotide sequence of SEQ ID NO: 41 or 56; or (17') a nucleotide sequence having a sequence identity of 90% or more to the nucleotide sequence of SEQ ID NO: 41 or 56.
[1-13] The bacteriolytic agent according to any of [1-1] to [1-12], wherein the bacteriolytic agent exhibits bacteriolytic ability against bacteria of the *Xanthomonas* genus.
[2-1] A bacteriolytic agent consisting of a bacteriophage comprising, in the genomic DNA, the following: a gene consisting of the amino acid sequence of any one of the following (a) to (c); and a gene consisting of the amino acid sequence of any one of the following (d) to (f), wherein the genes are respectively a gene encoding a tail tubular protein A and a gene encoding a tail tubular protein B, both proteins having a recognizing ability for target bacteria: (a) the amino acid sequence of SEQ ID NO: 24, 49, or 60; (b) the amino acid sequence having addition, deletion, and/or substitution of one or a plurality of amino acids in the amino acid sequence of SEQ ID NO: 24 or 49; (c) an amino acid sequence having a sequence identity of 97% or more to the amino acid sequence of SEQ ID NO: 24 or 49; (d) the amino acid sequence of SEQ ID NO: 57, 50, or 61; (e) the amino acid sequence having addition, deletion, and/or substitution of one or a plurality of amino acids in the amino acid sequence of SEQ ID NO: 57 or 50; or (f) an amino acid sequence having a sequence identity of 97% or more to the amino acid sequence of SEQ ID NO: 57 or 50.
[2-2] A bacteriolytic agent consisting of a bacteriophage comprising, in the genomic DNA, a gene encoding a tail fiber protein having a recognizing ability for target bacteria, wherein the protein consists of the amino acid sequence of any one of the following (g) to (j): (g) the amino acid sequence of any one selected from the group consisting of SEQ ID NOs: 1, 11, 45, 15, and 42; (h) the amino acid sequence having addition, deletion, and/or substitution of one or a plurality of amino acids in the amino acid sequence of any one selected from the group consisting of SEQ ID NOs: 1, 11, 15, and 42; (i) an amino acid sequence having a sequence identity of 90% or more to the amino acid sequence of any one selected from the group consisting of SEQ ID NOs: 1, 11, 15, and 42; or (j) the amino acid sequence having substitution of one amino acid other than at positions 278 and 350 in the amino acid sequence of SEQ ID NO: 11.
[2-3] A bacteriolytic agent consisting of a bacteriophage comprising, in the genomic DNA, the following: a gene consisting of the amino acid sequence of any one of the following (k) to (m); and a gene consisting of the amino acid sequence of any one of the following (n) to (p), wherein the genes are respectively a gene encoding a tail tubular protein A and a gene encoding a tail tubular protein B, both proteins having a recognizing ability for target bacteria: (k) the amino acid sequence of SEQ ID NO: 37; (l) the amino acid sequence having addition, deletion, and/or substitution of one or a plurality of amino acids in the amino acid sequence of SEQ ID NO: 37; (m) an amino acid sequence having a sequence identity of 92% or more to the amino acid sequence of SEQ ID NO: 37; (n) the amino acid sequence of SEQ ID NO: 38, 54, or 59; (o) the amino acid sequence having addition, deletion, and/or substitution of one or a plurality of amino acids in the amino acid sequence of SEQ ID NO: 38 or 54; or (p) an amino acid sequence having a sequence identity of 92% or more to the amino acid sequence of SEQ ID NO: 38 or 54.
[2-4] A bacteriolytic agent consisting of a bacteriophage comprising, in the genomic DNA, a gene encoding a protein consisting of the amino acid sequence of any one of the following (q) to (s): (q) the amino acid sequence of SEQ ID NO: 21; (r) the amino acid sequence having addition, deletion, and/or substitution of one or a plurality of amino acids in the amino acid sequence of SEQ ID NO: 21; or (s) an amino acid sequence having a sequence identity of 90% or more to the amino acid sequence of SEQ ID NO: 21.
[2-5] A bacteriolytic agent consisting of a bacteriophage having a genomic DNA sequence comprising the nucleotide sequence of any one of the following (1') to (3'): (1') the nucleotide sequence of any one selected from the group consisting of SEQ ID NOs: 14, 18, 19, and 32 to 36; (2') the nucleotide sequence having addition, deletion, and/or substitution of one or a plurality of nucleotides in the nucleotide sequence of any one selected from the group consisting of SEQ ID NOs: 14, 18, 19, and 32 to 36; or (3') a nucleotide sequence having a sequence identity of 90% or more to the nucleotide sequence of any one selected from the group consisting of SEQ ID NOs: 14, 18, 19, and 32 to 36.
[2-6] The bacteriolytic agent according to [2-1], wherein the nucleotide sequence of the genomic DNA consists of the nucleotide sequence of any one of the following (4') to (8'): (4') the nucleotide sequence of SEQ ID NO: 28, 29, or 53; (5') the nucleotide sequence having addition, deletion, and/or substitution of one or a plurality of nucleotides in the nucleotide sequence other than the gene according to [2-2] within the nucleotide sequence of SEQ ID NO: 28, 29, or 53; (6') a nucleotide sequence having a sequence identity of 95% or more to the nucleotide sequence other than the gene according to [2-2] within the nucleotide sequence of SEQ ID NO: 28, 29, or 53; (7') the nucleotide sequence having addition, deletion, and/or substitution of one or a plurality of nucleotides in the nucleotide sequence of SEQ ID NO: 28, 29, or 53; or (8') a nucleotide sequence having a sequence identity of 95% or more to the nucleotide sequence of SEQ ID NO: 28, 29, or 53.
[2-7] The bacteriolytic agent according to any of [2-1] to [2-6], wherein the bacteriolytic agent exhibits bacteriolytic ability against bacteria of the *Xanthomonas* genus.
[3-1] A bacteriolytic agent consisting of a bacteriophage having a genomic DNA sequence comprising the nucleotide sequence of any one of the following (1') to (3'): (1') the nucleotide sequence of any one selected from the group consisting of SEQ ID NOs: 32 to 36, 14, 18, and 19; (2') the nucleotide sequence having addition, deletion, and/or substitution of one or a plurality of nucleotides in the nucleotide sequence of any one selected from the group consisting of SEQ ID NOs: 32 to 36, 14, 18, and 19; or (3') a nucleotide sequence having a sequence identity of 90% or more to the nucleotide sequence of any one selected from the group consisting of SEQ ID NOs: 32 to 36, 14, 18, and 19.
[3-2] A bacteriolytic agent consisting of a bacteriophage comprising, in the genomic DNA, a gene encoding a tail fiber protein having a recognizing ability for target bacteria, wherein the protein consists of the amino acid sequence of any one of the following (a) to (c): (a) the amino acid sequence of SEQ ID NO: 15; (b) the amino acid sequence having addition, deletion, and/or substitution of one or a plurality of amino acids in the amino acid sequence of SEQ ID NO: 15; or (c) an amino acid sequence having a sequence identity of 90% or more to the amino acid sequence of SEQ ID NO: 15.
[3-3] A bacteriolytic agent consisting of a bacteriophage comprising, in the genomic DNA, a gene encoding a protein consisting of the amino acid sequence of any one of the following (d) to (f): (d) the amino acid sequence of SEQ ID NO: 21; (e) the amino acid sequence having addition, deletion, and/or substitution of one or a plurality of amino acids in the amino acid sequence of SEQ ID NO: 21; or (f) an amino acid sequence having a sequence identity of 90% or more to the amino acid sequence of SEQ ID NO: 21.
[3-4] The bacteriolytic agent according to [3-2], wherein the gene consists of the nucleotide sequence of any one of the following (1) to (3): (1) the nucleotide sequence of SEQ ID NO: 16; (2) the nucleotide sequence having addition, deletion, and/or substitution of one or a plurality of nucleotides in the nucleotide sequence of SEQ ID NO: 16; or (3) a nucleotide sequence having a sequence identity of 90% or more to the nucleotide sequence of SEQ ID NO: 16.
[3-5] The bacteriolytic agent according to [3-2], wherein the nucleotide sequence of the genomic DNA consists of the nucleotide sequence of any one of the following (4') to (8'): (4') the nucleotide sequence of SEQ ID NO: 17; (5') the nucleotide sequence having addition, deletion, and/or substitution of one or a plurality of nucleotides in the nucleotide sequence other than the gene according to [3-2] within the nucleotide sequence of SEQ ID NO: 17; (6') a nucleotide sequence having a sequence identity of 80% or more to the nucleotide sequence other than the gene according to [3-2] within the nucleotide sequence of SEQ ID NO: 17; (7') the nucleotide sequence having addition, deletion, and/or substitution of one or a plurality of nucleotides in the nucleotide sequence of SEQ ID NO: 17; or (8') a nucleotide sequence having a sequence identity of 90% or more to the nucleotide sequence of SEQ ID NO: 17.
[3-6] The bacteriolytic agent according to [3-3], wherein the gene consists of the nucleotide sequence of any of the following (4) to (6): (4) the nucleotide sequence of SEQ ID NO: 22; (5) the nucleotide sequence having addition, deletion, and/or substitution of one or a plurality of nucleotides in the nucleotide sequence of SEQ ID NO: 22; or (6) a nucleotide sequence having a sequence identity of 90% or more to the nucleotide sequence of SEQ ID NO: 22.
[3-7] The bacteriolytic agent according to [3-3], wherein the nucleotide sequence of the genomic DNA consists of the nucleotide sequence of any one of the following (9') to (13'): (9') the nucleotide sequence of SEQ ID NO: 23; (10') the nucleotide sequence having addition, deletion, and/or substitution of one or a plurality of nucleotides in the nucleotide sequence other than the gene according to [3-3] within the nucleotide sequence of SEQ ID NO: 23; (11') a nucleotide sequence having a sequence identity of 95% or more to the nucleotide sequence other than the gene according to [3-3] within the nucleotide sequence of SEQ ID NO: 23; (12') the nucleotide sequence having addition, deletion, and/or substitution of one or a plurality of nucleotides in the nucleotide sequence of SEQ ID NO: 23; or (13') a nucleotide sequence having a sequence identity of 95% or more to the nucleotide sequence of SEQ ID NO: 23.
[3-8] The bacteriolytic agent according to any of [3-1] to [3-7], wherein the bacteriolytic agent exhibits bacteriolytic ability against bacteria of the *Xanthomonas* genus.
[3-9] The bacteriolytic agent according to [3-8], wherein the bacteria of the *Xanthomonas* genus are *Xanthomonas arboricola.*
[3-10] The bacteriolytic agent according to [3-9], wherein the pathovar of the bacteria of the *Xanthomonas* genus is *pruni.*
[4-1] A bacteriolytic agent consisting of a bacteriophage comprising, in the genomic DNA, a gene encoding a tail fiber protein having a recognizing ability for target bacteria, wherein the protein consists of the amino acid sequence of any one selected from the group consisting of SEQ ID NOs: 42, 11, 45, and 15.
[4-2] The bacteriolytic agent according to [4-1], wherein the gene consists of the nucleotide sequence of any one selected from the group consisting of SEQ ID NOs: 43, 12, 46, and 16.
[4-3] The bacteriolytic agent according to [4-1], wherein the nucleotide sequence of the genomic DNA consists of the nucleotide sequence of any one selected from the group consisting of SEQ ID NOs: 44, 13, 47, 48, and 17.
[4-4] A bacteriolytic agent consisting of a bacteriophage comprising, in the genomic DNA, a gene encoding a tail fiber protein having a recognizing ability for target bacteria, wherein the protein consists of the amino acid sequence of SEQ ID NO: 1.
[4-5] The bacteriolytic agent according to [4-4], wherein the amino acid sequence of SEQ ID NO: 1 is the amino acid sequence of any one of SEQ ID NOs: 2 to 4.
[4-6] The bacteriolytic agent according to [4-4], wherein the gene consists of the nucleotide sequence of any one of SEQ ID NOs: 5 to 7.
[4-7] The bacteriolytic agent according to [4-4], wherein the nucleotide sequence of the genomic DNA consists of the nucleotide sequence of any one of SEQ ID NOs: 8 to 10.
[4-8] A bacteriolytic agent consisting of a bacteriophage having a genomic DNA sequence comprising the nucleotide sequence of any one selected from the group consisting of SEQ ID NOs: 14, 18, 19, and 32 to 36.
[4-9] A bacteriolytic agent consisting of a bacteriophage comprising, in the genomic DNA, a gene encoding a protein consisting of the amino acid sequence of SEQ ID NO: 21.
[4-10] The bacteriolytic agent according to [4-9], wherein the gene consists of the nucleotide sequence of SEQ ID NO: 22.
[4-11] The bacteriolytic agent according to [4-9], wherein the nucleotide sequence of the genomic DNA consists of the nucleotide sequence of SEQ ID NO: 23.
[4-12] A bacteriolytic agent consisting of a bacteriophage comprising, in the genomic DNA, the following: a gene encoding a tail tubular protein A consisting of the amino acid sequence of SEQ ID NO: 24, 49, or 37 and having a recognizing ability for target bacteria; and a gene encoding a tail tubular protein B consisting of the amino acid sequence of any one selected from the group consisting of SEQ ID NOs: 57, 50, 38, 54, and 59 and having a recognizing ability for target bacteria.
[4-13] The bacteriolytic agent according to [4-12], wherein the gene encoding the tail tubular protein A consists of the nucleotide sequence of SEQ ID NO: 26, 51, or 39.
[4-14] The bacteriolytic agent according to [4-12], wherein the gene encoding the tail tubular protein B consists of the nucleotide sequence of any one selected from the group consisting of SEQ ID NOs: 27, 52, 40, and 55.
[4-15] The bacteriolytic agent according to [4-12], wherein the nucleotide sequence of the genomic DNA consists of the nucleotide sequence of any one selected from the group consisting of SEQ ID NOs: 28, 29, 53, and 41.
[4-16] The bacteriolytic agent according to any of [4-1] to [4-15], wherein the bacteriolytic agent exhibits bacteriolytic ability against bacteria of the *Xanthomonas* genus.
<1> A composition comprising one or more of the bacteriolytic agents according to any of (1-1) to (10-5), [1-1] to [4-16] as an active component(s).
   <2> A composition comprising two or more of the bacteriolytic agents according to (1-1) to (10-5), [1-1] to [4-16] as active components.
   <3> A composition for plant disease control, comprising the composition according to <1> or <2>.
   <4> The composition for plant disease control according to <3>, wherein the plant disease is caused by bacteria of the *Xanthomonas* genus.
   <5> The composition for plant disease control according to <3> or <4>, comprising another bacteriophage having bacteriolytic ability against the bacteria of the *Xanthomonas* genus.
   <6> A method for controlling plant disease, comprising a contacting step of contacting the composition for plant disease control according to any of <3> to <5> to a target plant.
   <7> A method for identifying bacteria of the *Xanthomonas* genus, comprising: a culturing step of culturing subject bacteria isolated from a plant tissue affected by a plant disease, to obtain a culture preparation; a mixing step of mixing the culture preparation and the bacteriolytic agent according to any of (1-1) to (10-5), [1-1] to [4-16], to obtain a mixture; a mixture culturing step of culturing the mixture under predetermined conditions; and a determining step of determining that the subject bacteria is a bacteria of the *Xanthomonas* genus, when the subject bacteria are lysed after the mixture culturing step.
   <8> The method according to <7>, wherein, in the mixture culturing step, the mixture further comprises a soft agar containing liquid medium, and wherein the mixture is cultured on a solid medium.
   <9> The method according to <7>, wherein, in the culturing step, the culture preparation comprises a soft agar containing liquid medium, and wherein the culture preparation is cultured on a solid medium.
   <10> The method according to any of <7> to <9>, further comprising, before the culturing step, an isolating step of isolating subject bacteria from a plant tissue affected by a plant disease.

The present specification encompasses the disclosure of Japanese Patent Application Nos. 2022-059936, 2022-060143, 2022-060249, 2022-060441, 2022-060819, 2022-060822, 2022-060887, 2022-060909, 2022-061075, 2022-156882, 2022-156972, 2022-157086, 2023-041699, 2023-041776, 2023-041784, and 2023-041827 that serve as a basis for the priority of the present application.

### Advantageous Effects of Invention

A bacteriolytic agent of the present invention and a composition comprising the same as an active component can lyse specific target bacteria.

A composition for plant disease control according to the present invention can prevent and inhibit a disease caused by specific target bacteria.

### Brief Description of Drawing

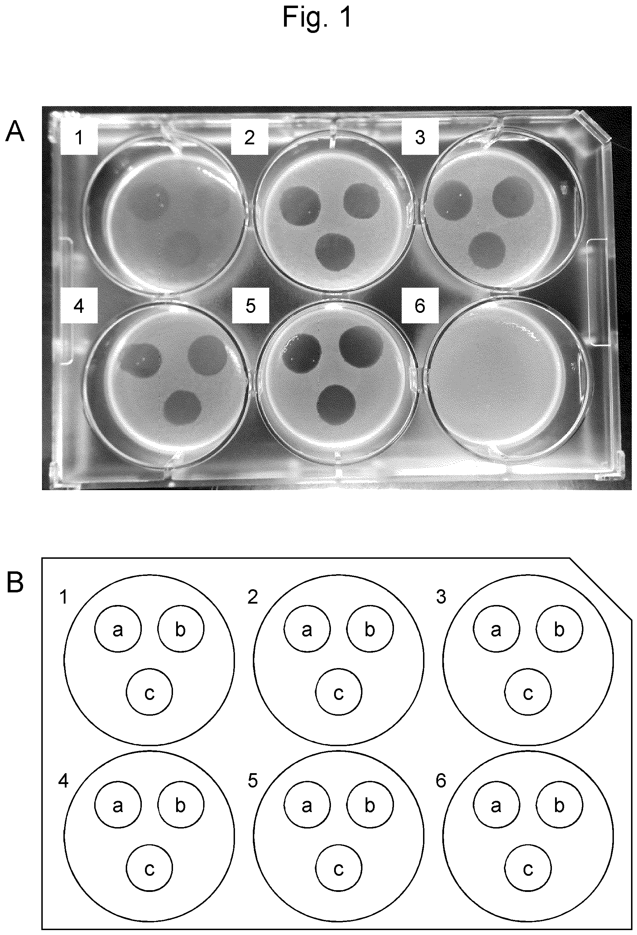

[Figure 1] Figure 1 shows the bacteriolytic ability of the 1st bacteriophage obtained in Example 1. Figure 1A shows a static culture with agar plates on which the bacteria of the *Xanthomonas* genus and *Pseudomonas fluorescens* as a control, which are shown in Table 2, were spread and cultured, and onto which a refined solution of the 1st phage was then dropped. Figure 1B is a diagram of the plates corresponding to Figure 1A, and shows the positions of the refined solutions of phage dropped onto the respective plates. In Figures 1A and 1B, 1 indicates a plate on which the bacteria with the ID MAFF No. 211191 was spread, 2 indicates a plate on which the bacteria with the ID MAFF No. 311351 was spread, 3 indicates a plate on which the bacteria with the ID MAFF No. 301256 was spread, 4 indicates a plate on which the bacteria with the ID MAFF No. 106765 was spread, 5 indicates a plate on which the bacteria with the ID MAFF No. 301078 was spread, and 6 indicates a plate on which *Pseudomonas fluorescens* with the ID NCIMB-ID 10460 as a control was spread. In Figure 1B, a indicates the position of a refined solution of a phage having the genomic DNA sequence of SEQ ID NO: 8, b indicates the position of a refined solution of a phage having the genomic DNA sequence of SEQ ID NO: 9, and c indicates the position of a refined solution of a phage having the genomic DNA sequence of SEQ ID NO: 10.

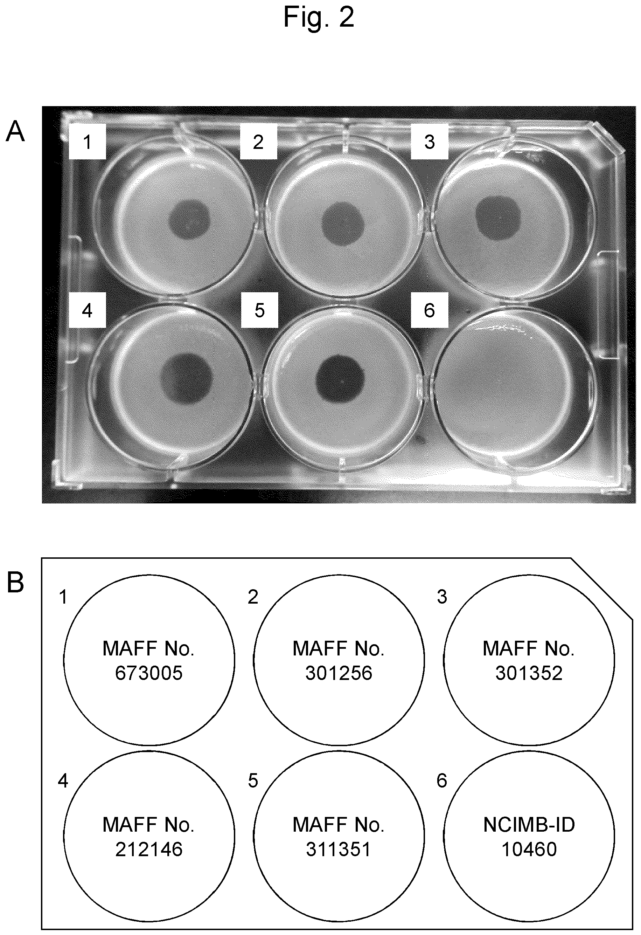

[Figure 2] Figure 2 shows the bacteriolytic ability of the 2nd bacteriophage obtained in Example 2. Figure 2A shows a static culture with agar plates on which the bacteria of the *Xanthomonas* genus and *Pseudomonas fluorescens* as a control were spread and cultured, and onto the central portion of which a refined solution of the 2nd phage was then dropped. Figure 2B is a diagram of the plates corresponding to Figure 2A and shows the bacteria listed in Table 4, which were spread on the respective plates.

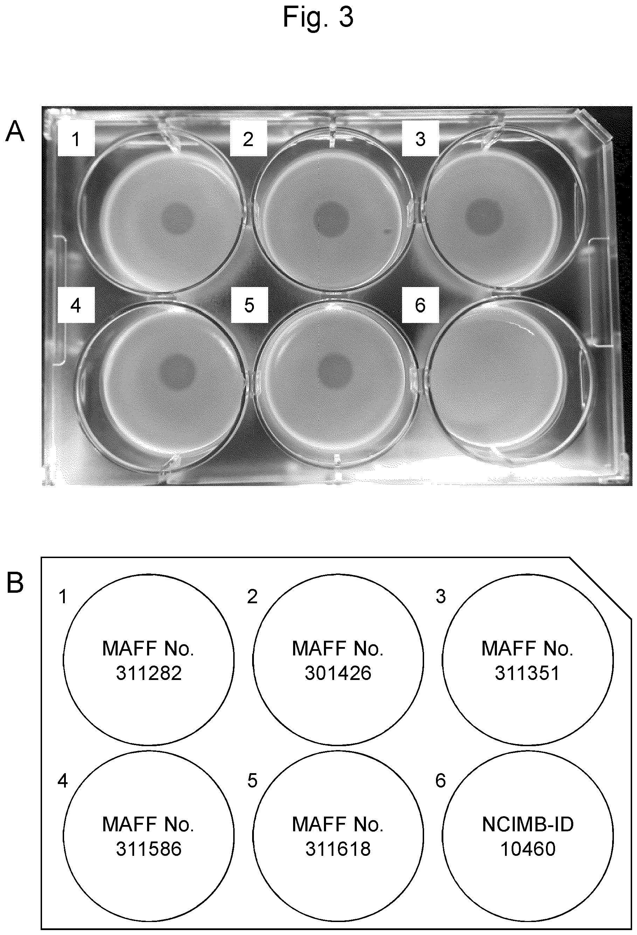

[Figure 3] Figure 3 shows the bacteriolytic ability of the 3rd bacteriophage obtained in Example 3. Figure 3A shows a static culture with agar plates on which the bacteria of the *Xanthomonas* genus and *Pseudomonas fluorescens* as a control, which are shown in Table 5, were spread and cultured, and onto which a refined solution of the 3rd phage was then dropped. Figure 3B is a diagram of the plates corresponding to Figure 3A and shows the positions of the refined solutions of phage dropped onto the respective plates. In Figures 3A and 3B, 1 indicates a plate on which the bacteria with the ID MAFF No. 311282 was spread, 2 indicates a plate on which the bacteria with the ID MAFF No. 301426 was spread, 3 indicates a plate on which the bacteria with the ID MAFF No. 311351 was spread, 4 indicates a plate on which the bacteria with the ID MAFF No. 311586 was spread, 5 indicates a plate on which the bacteria with the ID MAFF No. 311618 was spread, and 6 indicates a plate on which *Pseudomonas fluorescens* with the ID NCIMB-ID 10460 as a control was spread.

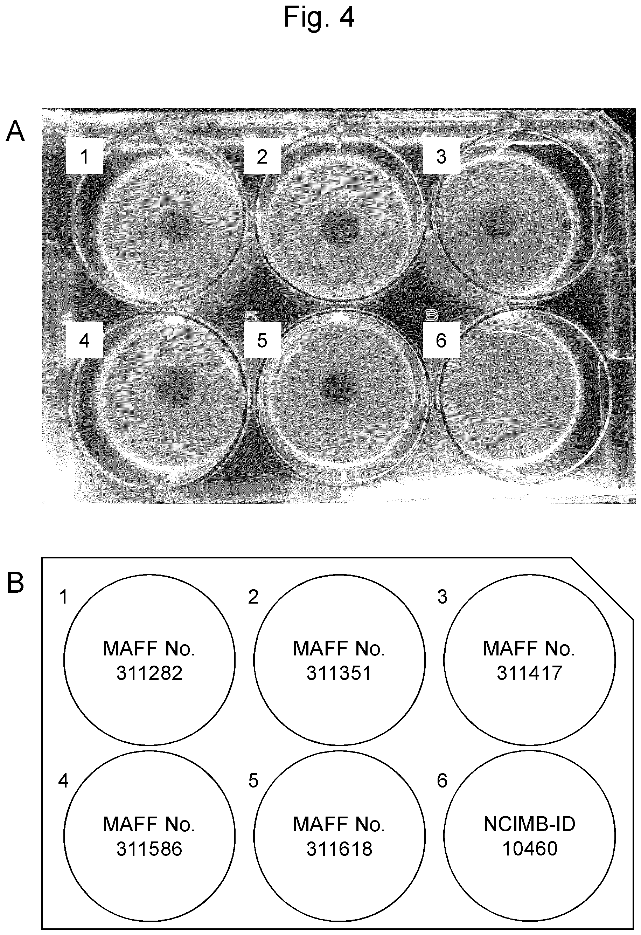

[Figure 4] Figure 4 shows the bacteriolytic ability of the 4th bacteriophage obtained in Example 4. Figure 4A shows a static culture with agar plates on which the bacteria of the *Xanthomonas* genus and *Pseudomonas fluorescens* as a control were spread and cultured, and onto the central portion of which a refined solution of the 4th phage was then dropped. Figure 4B is a diagram of the plates corresponding to Figure 4A and shows the bacteria listed in Table 6, which were spread on the respective plates.

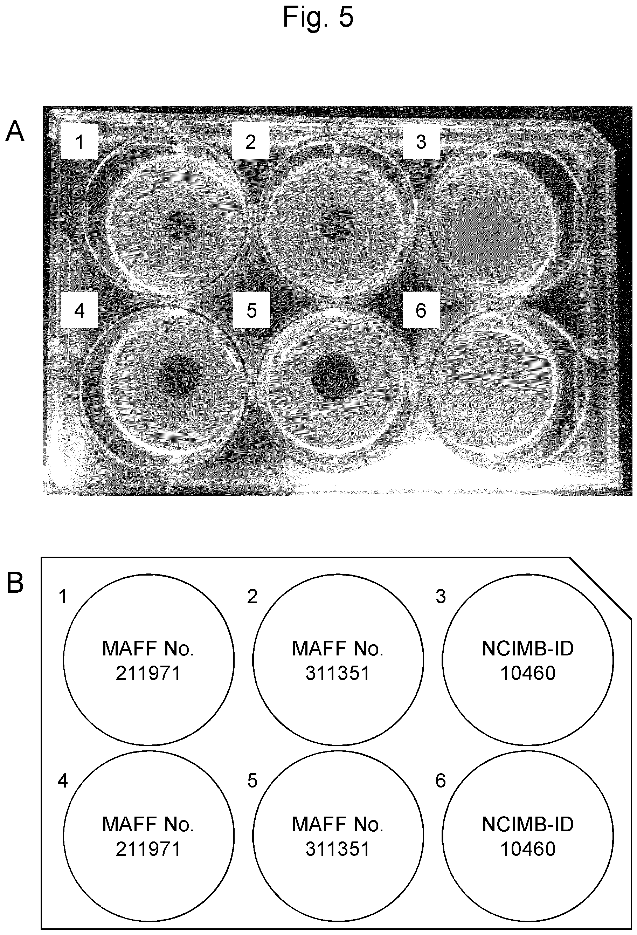

[Figure 5] Figure 5 shows the bacteriolytic ability of the 5th bacteriophage obtained in Example 5. The upper part (1 to 3) of Figure 5 shows the bacteriolytic ability of the bacteriophage of SEQ ID NO: 18, and the lower part (4 to 6) shows the bacteriolytic ability of the bacteriophage of SEQ ID NO: 19. Figure 5A shows a static culture with agar plates on which the bacteria of the *Xanthomonas* genus and *Pseudomonas fluorescens* as a control, which are shown in Table 7, were spread and cultured, and onto which a refined solution of the 5th phage was then dropped. Figure 5B is a diagram of the plates corresponding to Figure 5A, and shows the positions of the refined solutions of phage dropped onto the respective plates. In Figures 5A and 5B, 1 and 4 each indicate a plate on which the bacteria with the ID MAFF No. 211971 was spread, 2 and 5 each indicate a plate on which the bacteria with the ID MAFF No. 311351 was spread, and 3 and 6 each indicate a plate on which *Pseudomonas fluorescens* with the ID NCIMB-ID 10460 as a control was spread.

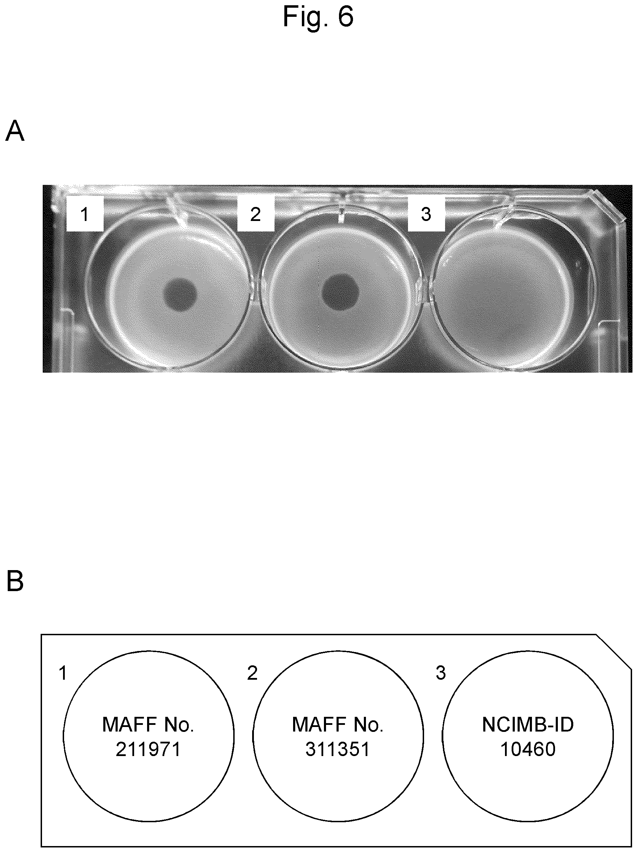

[Figure 6] Figure 6 shows the bacteriolytic ability of the 6th bacteriophage obtained in Example 6. Figure 6A shows a static culture with agar plates on which the bacteria of the *Xanthomonas* genus and *Pseudomonas fluorescens* as a control were spread and cultured, and onto the central portion of which a refined solution of the 6th phage was then dropped. Figure 6B is a diagram of the plates corresponding to Figure 6A and shows the bacteria listed in Table 9, which were spread on the respective plates.

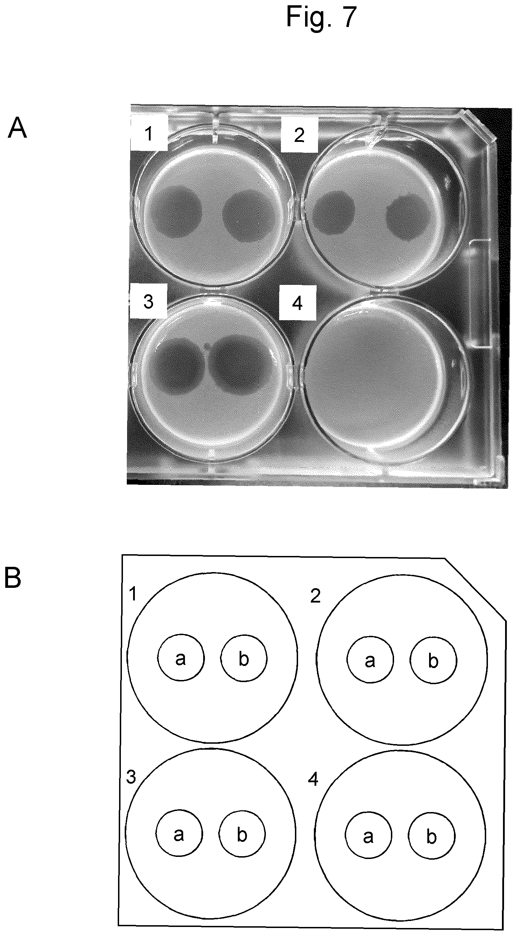

[Figure 7] Figure 7 shows the bacteriolytic ability of the 7th bacteriophage obtained in Example 7. Figure 7A shows a static culture with agar plates on which the bacteria of the *Xanthomonas* genus and *Pseudomonas fluorescens* as a control, which are shown in Table 10, were spread and cultured, and onto which a refined solution of the 7th phage was then dropped. Figure 7B is a diagram of the plates corresponding to Figure 7A and shows the positions of the refined solutions of phage dropped onto the respective plates. In Figures 7A and 7B, 1 indicates a plate on which the bacteria with the ID MAFF No. 311351 was spread, 2 indicates a plate on which the bacteria with the ID MAFF No. 311622 was spread, 3 indicates a plate on which the bacteria with the ID MAFF No. 106642 was spread, and 4 indicates a plate on which *Pseudomonas fluorescens* with the ID NCIMB-ID 10460 as a control was spread. In Figure 7B, a indicates the position of a refined solution of a phage having the genomic DNA sequence of SEQ ID NO: 28, and b indicates the position of a refined solution of a phage having the genomic DNA sequence of SEQ ID NO: 29.

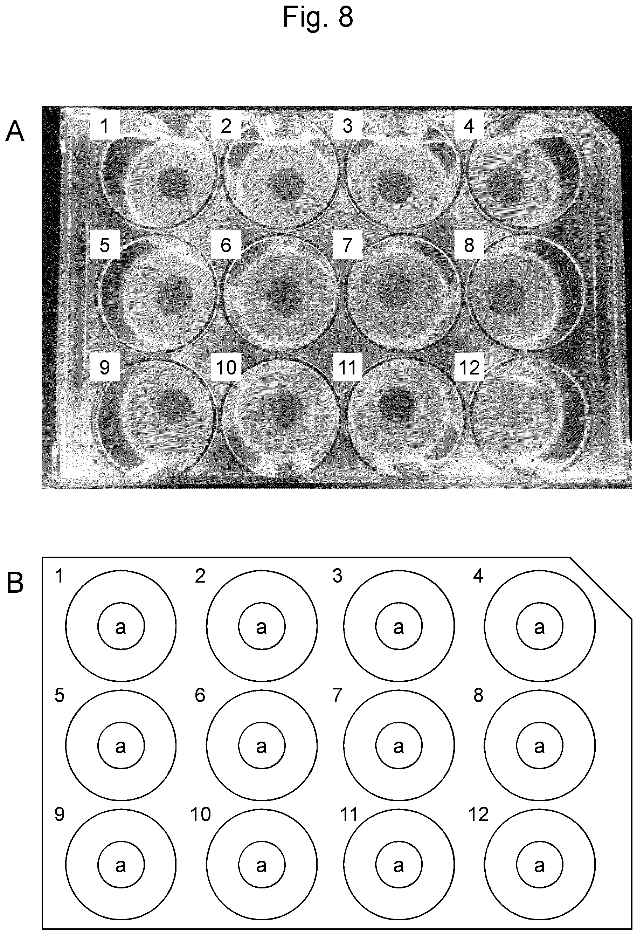

[Figure 8] Figure 8 shows the bacteriolytic ability of the 8th bacteriophage obtained in Example 8. Figure 8A shows a static culture with agar plates on which the bacteria of the *Xanthomonas* genus and *Pseudomonas fluorescens* as a control, which are shown in Table 11, were spread and cultured, and onto which a refined solution of the 8th phage was then dropped. Figure 8B is a diagram of the plates corresponding to Figure 8A and shows the positions of the refined solutions of phage dropped onto the respective plates. In Figures 8A and 8B, 1 indicates a plate on which the bacteria with the ID MAFF No. 211971 was spread, 2 indicates a plate on which the bacteria with the ID MAFF No. 311282 was spread, 3 indicates a plate on which the bacteria with the ID MAFF No. 301420 was spread, 4 indicates a plate on which the bacteria with the ID MAFF No. 301426 was spread, 5 indicates a plate on which the bacteria with the ID MAFF No. 311351 was spread, 6 indicates a plate on which the bacteria with the ID MAFF No. 311414 was spread, 7 indicates a plate on which the bacteria with the ID MAFF No. 311417 was spread, 8 indicates a plate on which the bacteria with the ID MAFF No. 311562 was spread, 9 indicates a plate on which the bacteria with the ID MAFF No. 311571 was spread, 10 indicates a plate on which the bacteria with the ID MAFF No. 311618 was spread, 11 indicates a plate on which the bacteria with the ID MAFF No. 311622 was spread, and 12 indicates a plate on which *Pseudomonas fluorescens* with the ID NCIMB-ID 10460 as a control was spread. In Figure 8B, a indicates the position of a refined solution of a phage having the genomic DNA sequence of SEQ ID NO: 32.

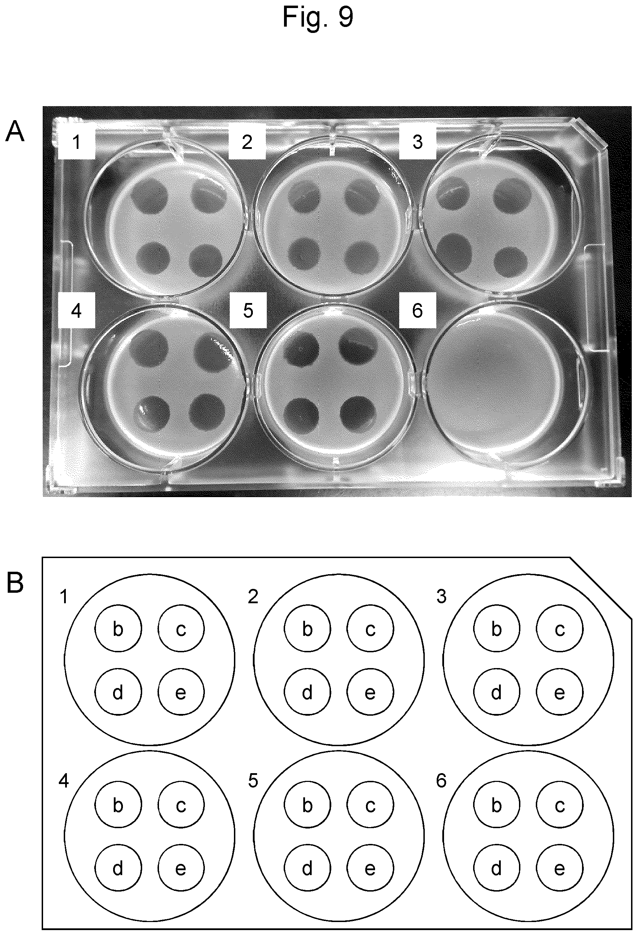

[Figure 9] Figure 9 shows the bacteriolytic ability of the 8th bacteriophage obtained in Example 8. Figure 9A shows a static culture with agar plates on which the bacteria of the *Xanthomonas* genus and *Pseudomonas fluorescens* as a control, which are shown in Table 11, were spread and cultured, and onto which the refined solution of the 8th phage was then dropped. Figure 9B is a diagram of the plates corresponding to Figure 9A and shows the positions of the refined solutions of phage dropped onto the respective plates. In Figures 9A and 9B, 1 indicates a plate on which the bacteria with the ID MAFF No. 211971 was spread, 2 indicates a plate on which the bacteria with the ID MAFF No. 311282 was spread, 3 indicates a plate on which the bacteria with the ID MAFF No. 301420 was spread, 4 indicates a plate on which the bacteria with the ID MAFF No. 301426 was spread, 5 indicates a plate on which the bacteria with the ID MAFF No. 311351 was spread, and 6 indicates a plate on which *Pseudomonas fluorescens* with the ID NCIMB-ID 10460 as a control was spread. In Figure 9B, b indicates the position of a refined solution of a phage having the genomic DNA sequence of SEQ ID NO: 33, c indicates the position of a refined solution of a phage having the genomic DNA sequence of SEQ ID NO: 34, d indicates the position of a refined solution of a phage having the genomic DNA sequence of SEQ ID NO: 35, and e indicates the position of a refined solution of a phage having the genomic DNA sequence of SEQ ID NO: 36.

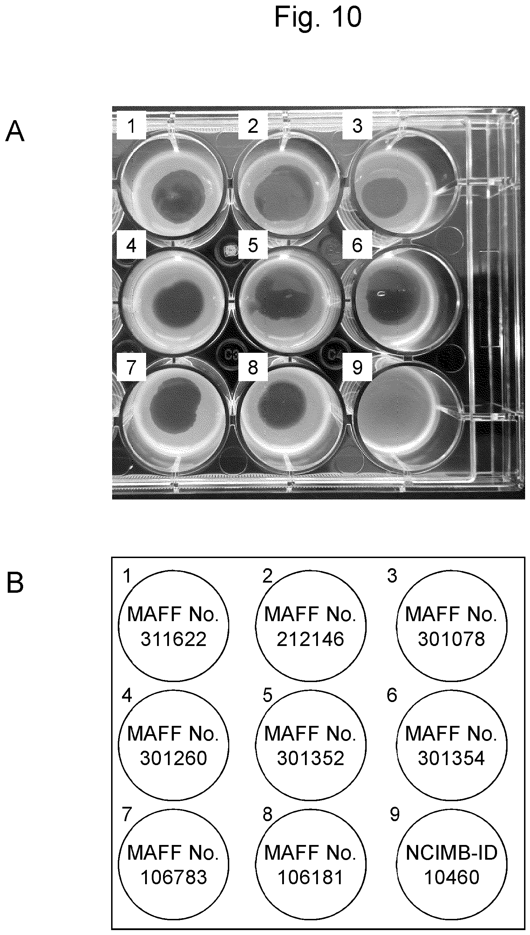

[Figure 10] Figure 10 shows the bacteriolytic ability of a bacteriophage obtained in Example 9. Figure 10A shows a static culture with agar plates on which the bacteria of the *Xanthomonas* genus and *Pseudomonas fluorescens* as a control, which are shown in Table 13, were spread and cultured, and onto which a refined solution of phage was then dropped. Figure 10B is a diagram of the plates corresponding to Figure 10A and shows the positions of the refined solutions of phage dropped onto the respective plates.

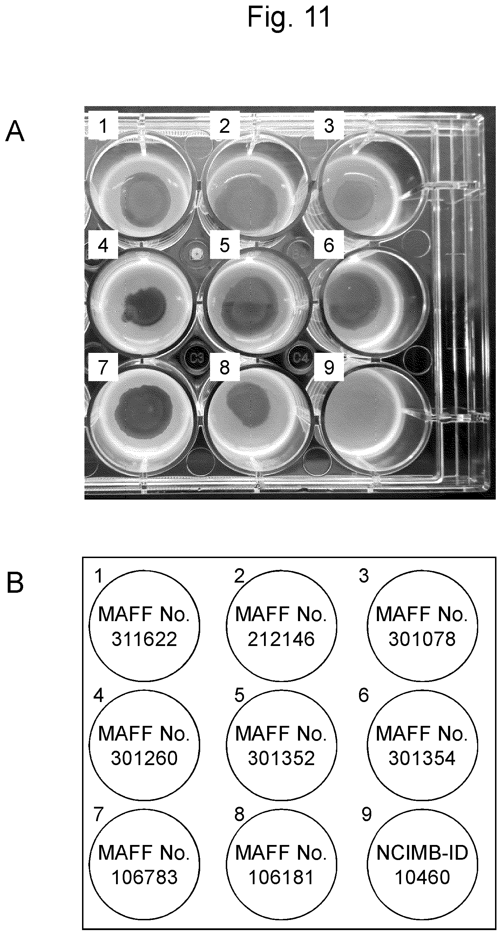

[Figure 11] Figure 11 shows the bacteriolytic ability of a bacteriophage obtained in Example 10. Figure 11A shows a static culture with agar plates on which the bacteria of the *Xanthomonas* genus and *Pseudomonas fluorescens* as a control, which are shown in Table 13, were spread and cultured, and onto which a refined solution of phage was then dropped. Figure 11B is a diagram of the plates corresponding to Figure 11A and shows the positions of the refined solutions of phage dropped onto the respective plates.

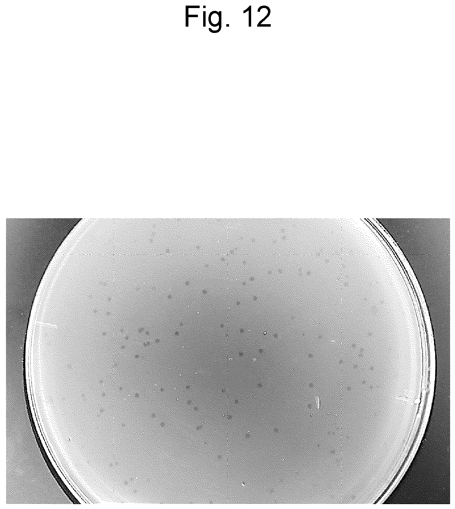

[Figure 12] Figure 12 shows one example of a plate in a plaque assay of the 1st bacteriophage obtained in Example 1.

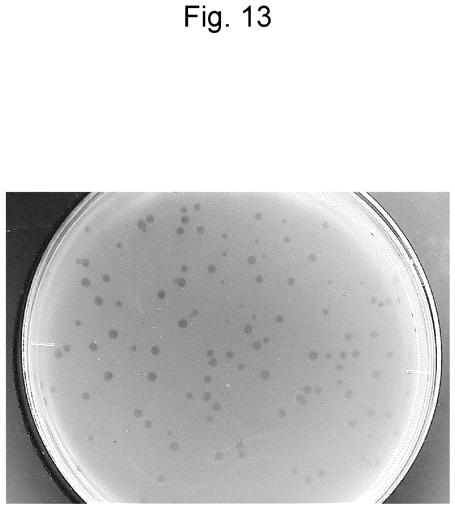

[Figure 13] Figure 13 shows one example of a plate in a plaque assay of the 2nd bacteriophage obtained in Example 2.

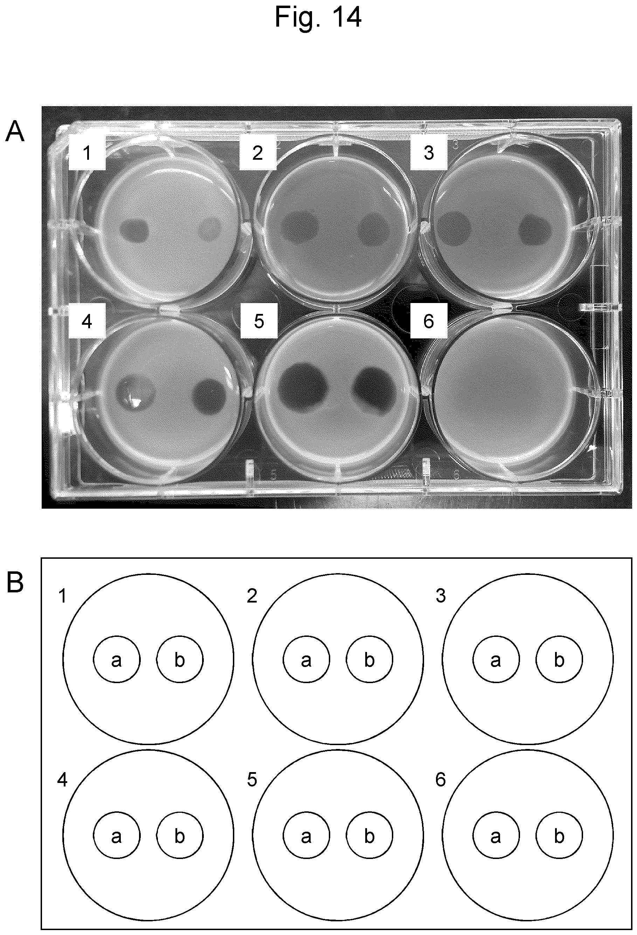

[Figure 14] Figure 14 shows the bacteriolytic ability of the 2nd bacteriophage obtained in Example 11. Figure 14A shows a static culture with agar plates on which the bacteria of the *Xanthomonas* genus and *Pseudomonas fluorescens* as a control, which are shown in Table 4, were spread and cultured, and onto which the refined solution of 2nd phage was then dropped. Figure 14B is a diagram of the plates corresponding to Figure 14A and shows the positions of the refined solutions of phage dropped onto the respective plates. In Figures 14A and 14B, 1 indicates a plate on which the bacteria with the ID MAFF No. 673005 was spread, 2 indicates a plate on which the bacteria with the ID MAFF No. 301256 was spread, 3 indicates a plate on which the bacteria with the ID MAFF No. 301352 was spread, 4 indicates a plate on which the bacteria with the ID MAFF No. 212146 was spread, 5 indicates a plate on which the bacteria with the ID MAFF No. 311351 was spread, and 6 indicates a plate on which *Pseudomonas fluorescens* with the ID NCIMB-ID 10460 as a control was spread. In Figure 14B, a indicates the position of a refined solution of a phage having the genomic DNA sequence of SEQ ID NO: 47, and b indicates the position of a refined solution of a phage having the genomic DNA sequence of SEQ ID NO: 48.

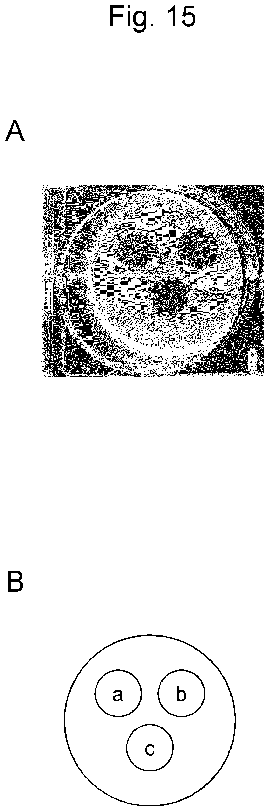

[Figure 15] Figure 15 shows the bacteriolytic ability of the 7th phage obtained in Example 2 and two kinds of 2nd bacteriophages obtained in Example 11. Figure 15A shows a static culture with an agar plate on which the bacteria of the *Xanthomonas* genus, which are shown in Table 14, were spread and cultured, and onto which the refined solution of 2nd phage was then dropped. Figure 15B is a diagram of the plates corresponding to Figure 15A and shows the positions of the refined solutions of phage dropped onto the respective plates. In Figure 15B, a indicates the position of a refined solution of a phage having the genomic DNA sequence of SEQ ID NO: 12, b indicates the position of a refined solution of a phage having the genomic DNA sequence of SEQ ID NO: 47, and c indicates the position of a refined solution of a phage having the genomic DNA sequence of SEQ ID NO: 48.

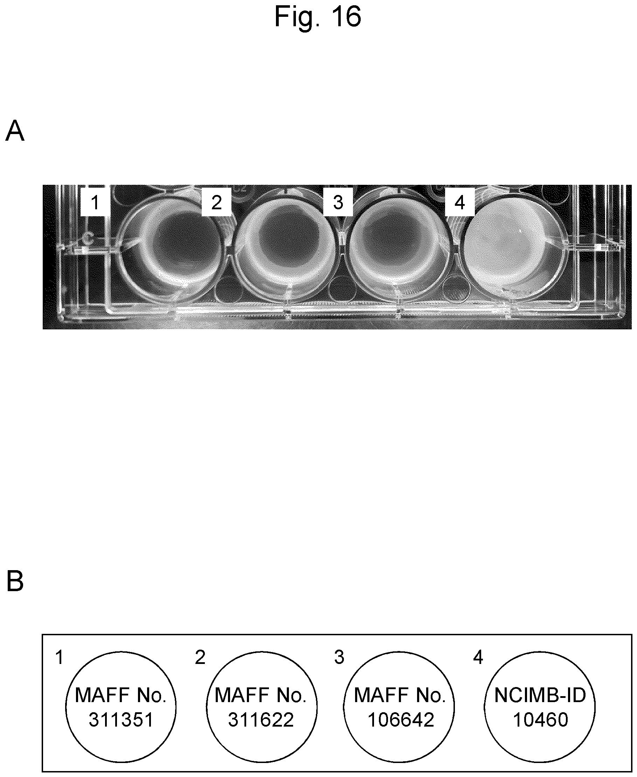

[Figure 16] Figure 16 shows the bacteriolytic ability of the 7th bacteriophage obtained in Example 12. Figure 16A shows a static culture with agar plates on which the bacteria of the *Xanthomonas* genus and *Pseudomonas fluorescens* as a control, which are shown in Table 10, were spread and cultured, and onto which a refined solution of phage was then dropped. Figure 16B is a diagram of the plates corresponding to Figure 16A and shows the IDs of the bacteria cultured on the respective plates.

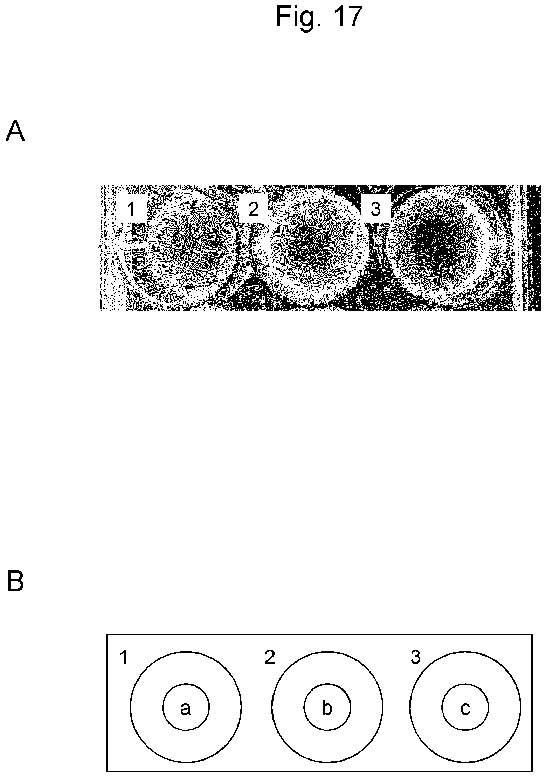

[Figure 17] Figure 17 shows the bacteriolytic ability of two kinds of 7th phages obtained in Example 7 and a phage obtained in Example 12. Figure 17A shows a static culture with an agar plate on which the bacteria of the *Xanthomonas* genus with the ID MAFF No. 301260 were spread and cultured, and onto which a refined solution of phage was then dropped. Figure 17B is a diagram of the plates corresponding to Figure 17A and shows the positions of the refined solutions of phage dropped onto the respective plates. In Figure 17B, a indicates the position of a refined solution of a phage having the genomic DNA sequence of SEQ ID NO: 28, b indicates the position of a refined solution of a phage having the genomic DNA sequence of SEQ ID NO: 29, and c indicates the position of a refined solution of a phage obtained in Example 12 and having the genomic DNA sequence of SEQ ID NO: 53.

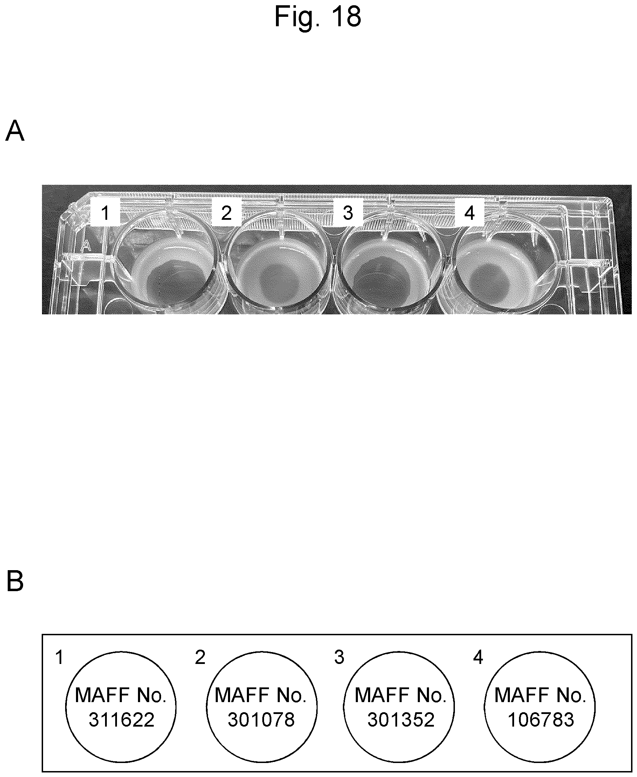

[Figure 18] Figure 18 shows the bacteriolytic ability of a 9th bacteriophage obtained in Example 13. Figure 18A shows a static culture with an agar plate on which the bacteria of the *Xanthomonas* genus, which are shown in Table 13, were spread and onto which a refined solution of phage was then dropped. Figure 18B is a diagram of the plates corresponding to Figure 18A and shows the IDs of the bacteria cultured on the respective plates.

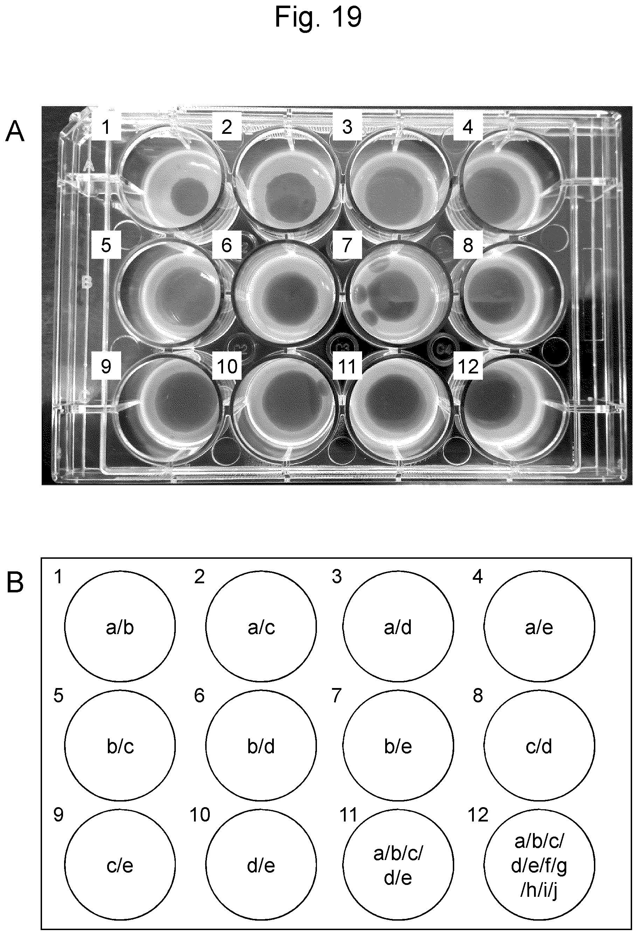

[Figure 19] Figure 19 shows the bacteriolytic ability of the combinations of bacteriophages tested in Example 14. Figure 19A shows a static culture on an agar plate on which the bacteria of the *Xanthomonas* genus with the ID MAFF No. 311351 were spread and cultured, and onto which a refined solution of phage was then dropped. Figure 19B is a diagram of the plates corresponding to Figure 19A and shows the kinds of phages comprised in the refined solutions of phages dropped onto the respective plates. In Figure 19B, a indicates the 1st phage having the genomic DNA sequence of SEQ ID NO: 8, b indicates the 2nd phage having the genomic DNA sequence of SEQ ID NO: 13, c indicates the 7th phage having the genomic DNA sequence of SEQ ID NO: 28, d indicates the 9th phage having the genomic DNA sequence of SEQ ID NO: 41, e indicates the 10th phage having the genomic DNA sequence of SEQ ID NO: 44, f indicates the 3rd phage having the genomic DNA sequence of SEQ ID NO: 14, g indicates the 4th phage having the genomic DNA sequence of SEQ ID NO: 17, h indicates the 5th phage having the genomic DNA sequence of SEQ ID NO: 18, i indicates the 6th phage having the genomic DNA sequence of SEQ ID NO: 23, and j indicates the 8th phage having the genomic DNA sequence of SEQ ID NO: 32, respectively. In the diagram, the mark "/" indicates that the phages before and after the mark are used in combination. For example, "a/b" indicates that the 1st phage and the 2nd phage are used in combination.

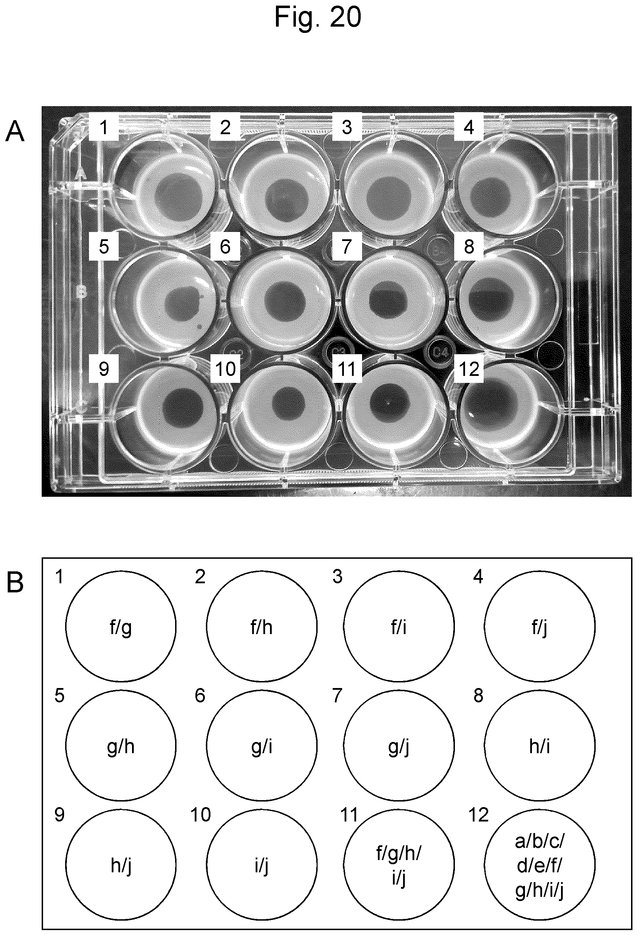

[Figure 20] Figure 20 shows the bacteriolytic ability of the combinations of bacteriophages tested in Example 14. Figure 20A shows a static culture on an agar plate on which the bacteria of the *Xanthomonas* genus with the ID MAFF No. 311351 were spread and cultured, and onto which a refined solution of phage was then dropped. Figure 20B is a diagram of the plates corresponding to Figure 20A and shows the kinds of phages comprised in the refined solutions of phages dropped onto the respective plates. In Figure 20B, a indicates the 1st phage having the genomic DNA sequence of SEQ ID NO: 8, b indicates the 2nd phage having the genomic DNA sequence of SEQ ID NO: 13, c indicates the 7th phage having the genomic DNA sequence of SEQ ID NO: 28, d indicates the 9th phage having the genomic DNA sequence of SEQ ID NO: 41, e indicates the 10th phage having the genomic DNA sequence of SEQ ID NO: 44, f indicates the 3rd phage having the genomic DNA sequence of SEQ ID NO: 14, g indicates the 4th phage having the genomic DNA sequence of SEQ ID NO: 17, h indicates the 5th phage having the genomic DNA sequence of SEQ ID NO: 18, i indicates the 6th phage having the genomic DNA sequence of SEQ ID NO: 23, and j indicates the 8th phage having the genomic DNA sequence of SEQ ID NO: 32, respectively. In the diagram, the mark "/" indicates that the phages before and after the mark are used in combination. For example, "f/g" indicates that the 3rd phage and the 4th phage are used in combination.

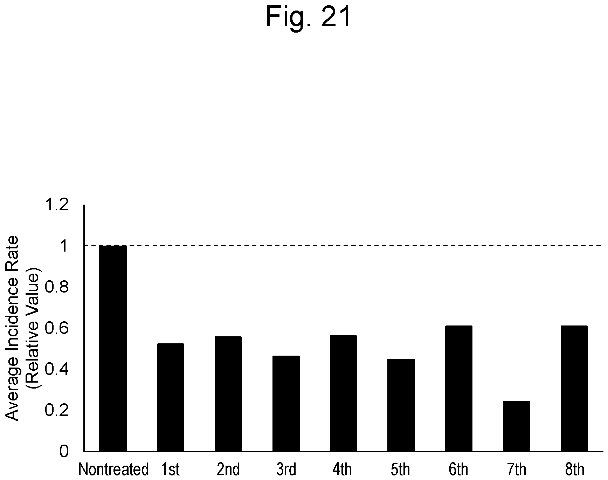

[Figure 21] Figure 21 graphs the result of a disease control effect test on a shot hole disease of *Prunus spp.* tested in Example 15. The average incidence rate is expressed as a relative value with respect to a nontreated group. The number below each bar indicates the kind of phage used. In each experimental group, each of the 1st to 8th phages was used alone.

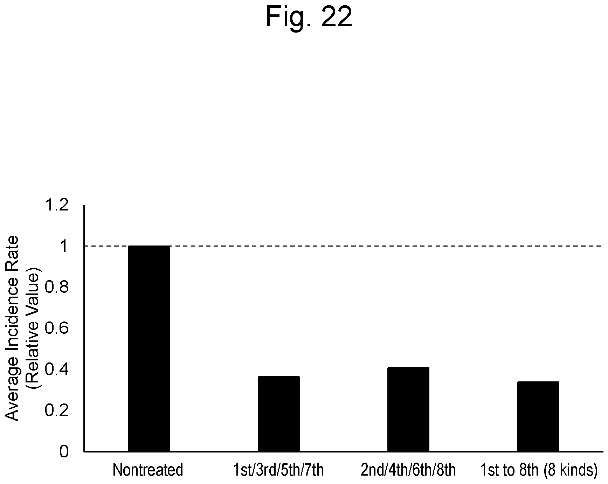

[Figure 22] Figure 22 graphs the result of a disease control effect test on a shot hole disease of *Prunus spp.* tested in Example 15. The average incidence rate is expressed as a relative value with respect to a nontreated group. The numbers below each bar indicate the kinds of phages used in combination. In each experimental group, a combination of four kinds of phages consisting of the 1st phage, the 3rd phage, the 5th phage, and the 7th phage; a combination of four kinds of phages consisting of the 2nd phage, the 4th phage, the 6th phage, and the 8th phage; and a combination of eight kinds of phages consisting of the 1st phage to the 8th phage were used, respectively.

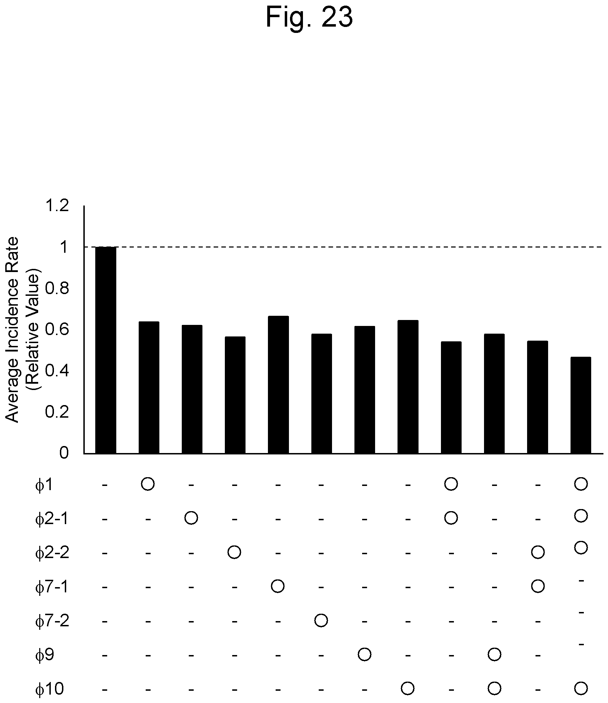

[Figure 23] Figure 23 graphs the result of a disease control effect test on a black rot disease of broccoli tested in Example 16. The average incidence rate is expressed as a relative value with respect to a nontreated group. The number below each bar indicates the kind of phage used. In the graph, a circle indicates that the phage was used, and "-" indicates that the phage was not used. Φ1 indicates the 1st phage having the genomic DNA sequence of SEQ ID NO: 10, Φ2-1 indicates the 2nd phage having the genomic DNA sequence of SEQ ID NO: 13, Φ2-2 indicates the 2nd phage having the genomic DNA sequence of SEQ ID NO: 47, Φ7-1 indicates the 7th phage having the genomic DNA sequence of SEQ ID NO: 28, Φ7-2 indicates the 7th phage having the genomic DNA sequence of SEQ ID NO: 53, Φ9 indicates the 9th phage having the genomic DNA sequence of SEQ ID NO: 41, and Φ10 indicates the 10th phage having the genomic DNA sequence of SEQ ID NO: 44, respectively.

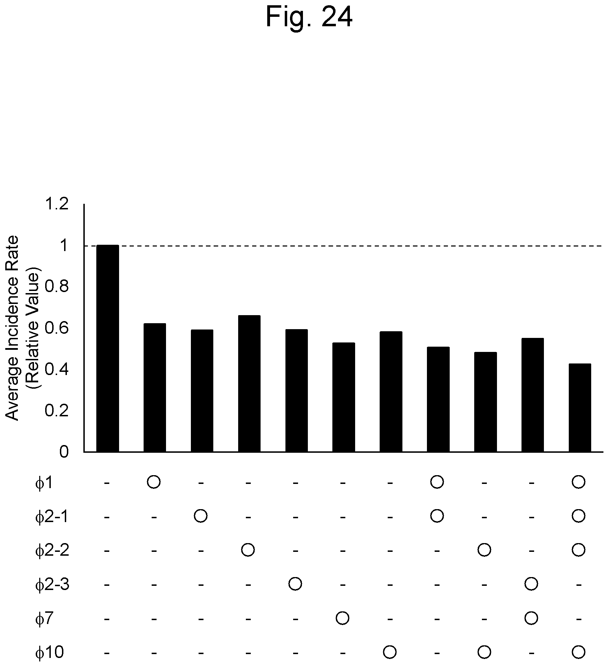

[Figure 24] Figure 24 graphs the result of a disease control effect test on a bacterial spot disease of tomato tested in Example 17. The average incidence rate is expressed as a relative value with respect to a nontreated group. The number below each bar indicates the kind of phage used. In the graph, a circle indicates that the phage was used, and "-" indicates that the phage was not used. Φ1 indicates the 1st phage having the genomic DNA sequence of SEQ ID NO: 10, Φ2-1 indicates the 2nd phage having the genomic DNA sequence of SEQ ID NO: 13, Φ2-2 indicates the 2nd phage having the genomic DNA sequence of SEQ ID NO: 47, Φ2-3 indicates the 2nd phage having the genomic DNA sequence of SEQ ID NO: 48, Φ7 indicates the 7th phage having the genomic DNA sequence of SEQ ID NO: 28, and Φ10 indicates the 10th phage having the genomic DNA sequence of SEQ ID NO: 44, respectively.

### Description of Embodiments

### 1. Bacteriolytic Agent

### 1-1. Overview

A first aspect of the present invention is a bacteriolytic agent. A bacteriolytic agent of the present invention consists of a bacteriophage having a genomic sequence comprising a specific nucleotide sequence.

A bacteriolytic agent of the present invention exhibits bacteriolytic ability against target bacteria that can be pathogenic bacteria for a plant disease.

### 1-2. Definition

Terms used herein are defined below.

A "bacteriolytic agent" as used herein refers to a drug consisting of a bacteriophage having bacteriolytic ability against target bacteria.

"Bacteria", besides archaea and eukaryotes, is one of the three major biological lineages into which the whole biological world is classified. A bacterium is composed of a cell without a nucleus (acaryote) and can self-reproduce itself with a nutrient source. Bacteria are named with a genus and a species under a family in accordance with the International Code of Nomenclature of Bacteria.

"Target bacteria" as used herein refers to host bacteria that may be a target for a phage constituting a bacteriolytic agent of the present invention or a phage comprised in a composition for plant disease control and a composition according to the present invention. A specific example is a bacterium having a membrane surface receptor to be recognized on the outer membrane (epicyte) by the phage. Another example is a bacterium that has on the outer membrane a membrane surface receptor to be recognized by a tail fiber protein consisting of a specific amino acid sequence. Yet another example is a bacterium that has on the outer membrane a membrane surface receptor to be recognized by a tail tubular protein consisting of a specific amino acid sequence. A "membrane surface receptor" is a site to which, for example, a tail, a tail fiber, and the like of a phage are bound, and is composed of a protein, a lipopolysaccharide, pili, or the like that is present in the outer layer of the bacterial outer membrane. A specific example of the target bacteria herein is bacteria of the *Xanthomonas* genus.

"Bacteria of the *Xanthomonas* genus" refers to a bacteria belonging to *Xanthomonas* genus. Bacteria of the *Xanthomonas* genus commonly produce a yellow pigment called Xanthomonasin, and many of the bacteria are known to be plant pathogenic bacteria. In this connection, there are subtypes and pathovars as subclasses of species, and they are indicated by *subsp.* or *pv.* following the name of the bacteria, respectively. Additionally, the smallest unit of classification is a strain, which refers to a cell population considered to be genetically uniform. Table 1 below shows typical bacteria of the *Xanthomonas* genus, host plants thereof, and plant diseases caused thereby.

**[Table 1]**

| **Species** | **Pathovar** | **Host** | **Disease** |
|---|---|---|---|
| ***X. arboricola*** | *pruni* | Peach | Bacterial spot |
| ***X. arboricola*** | *juglandis* | Walnut | Bacterial blight |
| ***X. arboricola*** | *corylina* | Turkish Hazel | Leaf spot |
| ***X. axonopodis*** | *citrumelo* | Citrus | Bacterial spot |
| ***X. axonopodis*** | *glycines* | Soybean | Bacterial pustule |
| ***X. axonopodis*** | *manihotis* | Cassava | Bacterial blight |
| ***X. axonopodis*** | *malvacearum* | Cotton | Bacterial blight |
| ***X. axonopodis*** | *punicae* | Pomegranate | Leaf blight |
| ***X. albilineans*** | | Sugarcane | Leaf scald |
| ***X. campestris*** | *campestris* | Cabbage | Black rot |
| ***X. campestris*** | *musacearum* | Banana | Bacterial wilt |
| ***X. campestris*** | *vasculorum* | Sugarcane | Gumming disease |
| ***X. campestris*** | *vesicatoria* | Tomato/Pepper | Bacterial spot |
| ***X. campestris*** | *vitians* | Lettuce | Bacterial spot |
| ***X. campestris*** | *raphani* | Cabbage | Leaf spot |
| ***X. citri*** | *citri* | Orange | Bacterial canker |
| ***X. citri*** | *malvacearum* | Cotton | Angular leaf spot |
| ***X. citri*** | *mangiferaeindicae* | Mango | Black spot |
| ***X. cucurbitae*** | | Pumpkin | Bacterial Spot |
| ***X. fragariae*** | | Strawberry | Angular leaf spot |
| ***X. fuscans*** | *fuscans* | Bean | Bacterial blight |
| ***X. hortorum*** | *carotae* | Carrot | Bacterial blight |
| **X. *oryzae*** | *oryzae* | Rice | Leaf blight |

Among the bacteria of the *Xanthomonas* genus, *Xanthomonas arboricola, Xanthomonas campestris, Xanthomonas citri, Xanthomonas oryzae,* and *Xanthomonas cucurbitae* are preferable as target bacteria in the present invention, without limitation. Specific examples of *Xanthomonas arboricola* include: *Xanthomonas arboricola pv. pruni,* the pathovar of which is *pruni*; and *Xanthomonas arboricola pv. juglandis,* the pathovar of which is *juglandis.* Specific examples of *Xanthomonas campestris* include: *Xanthomonas campestris pv. vesicatoria,* the pathovar of which is *vesicatoria*; *Xanthomonas campestris pv. campestris,* the pathovar of which is *campestris*; *Xanthomonas campestris pv. vitians,* the pathovar of which is *vitians*; and *Xanthomonas campestris pv. raphani,* the pathovar of which is *raphani.* Specific examples of *Xanthomonas citri* include *Xanthomonas citri subsp. citri.* Specific examples of *Xanthomonas oryzae* include *Xanthomonas oryzae pv. oryzae,* the pathovar of which is *oryzae.*

A "bacteriophage" (herein often abbreviated simply as a "phage," as mentioned above) is a generic term for viruses that infect bacteria. A common phage is composed of three parts: a head (head part), a tail (tail part), and a tail fiber (tail fiber part). The head, constituted by a capsomere that is a coat protein, is composed of a capsid (viral shell) having an icosahedral structure, in the inner space of which the genomic DNA of a phage is encapsulated. The tail has a tubular structure composed of a tail tubular protein and a sheath protein covering the same. One end and the other of the tail are linked to the head and the tail fiber, respectively. The tail functions as an introduction tube through which the genomic DNA in the head is injected into the cell of the host bacteria. The tail fiber has a structure of several fibers composed of a tail fiber protein. The tail and the tail fiber serve a host-recognizing function and an attachment function, i.e., to recognize a receptor on the surface of the outer membrane of host bacteria and be attached to the cell surface. A phage has an extremely high host specificity, and this characteristic is based on the functions of the tail and the tail fiber.

A phage does not infect a eukaryote, and thus, a drug comprising a phage is harmless to humans, animals, and plants. In this connection, phages are roughly classified into a "bacteriolytic cycle", a "lysogenic cycle", and a "bacteriolytic/lysogenic cycle" according to the mode of infection. In the lysogenic cycle, a phage incorporates (introduces) its own DNA into a chromosome of target bacteria without lysing the bacteria and proliferates as the bacteria proliferate. On the other hand, in the bacteriolytic cycle, a phage self-proliferates in a cell of host bacteria and then lyses the host bacteria to release a large number of daughter phages. As a phage in the present invention, a virulent phage that undergoes the bacteriolytic cycle is intended.

A "tail fiber protein" is a protein constituting the tail fiber of a phage, as described above. It is known that the tail fiber protein plays an important role in the specificity of the host recognition and attachment capability of the tail and the tail fiber (Nobrega F.L. et al., Nat. Rev. Microbiol., 2018, 16: 760-773). Accordingly, even if the host bacteria for the novel phage characterized by the tail fiber protein are the same as for a known phage, the novel phage is different in the recognition site of the host and hence provides a very high utility value, for example, a possibility of exhibiting bacteriolytic ability even to bacteria having, for example, infection resistance to a known phage.

A "tail fiber gene" refers to a gene encoding the tail fiber protein comprised in the genomic DNA of a phage.

A "tail tubular protein" is a protein constituting the tubular structure of the tail of a phage, as described above. It is known that the tail tubular protein interacts with the tail fiber and, together with the tail fiber, plays an important role in the specificity of the host recognition and attachment capability (Maozhi Hu, *et ai.,* 2020, 9:1, 855-867). As tail tubular proteins, a tail tubular fiber protein A and a tail tubular protein B are known. The "tail tubular protein A" is a protein that forms a ring at the lower part of the tubular structure of the tail and interacts with the tail fiber. The "tail tubular protein B" is a protein that forms the end of the lower part of the tubular structure of the tail and is bound to a receptor on the surface of the outer membrane of host bacteria.

A "tail tubular gene" refers to a gene encoding the tail tubular protein comprised in the genomic DNA of a phage. The "tail tubular protein A gene" and the "tail tubular protein B gene" refer to a gene encoding the tail tubular protein A and a gene encoding the tail tubular protein B, respectively.

"Bacteriolysis" refers to a phenomenon that destroys the cell membrane of bacteria. As described above, the phenomenon is mainly observed in the mode of infection of a virulent phage. Bacteriolysis kills bacteria. Bacteriolysis starts when a phage is specifically attached to a target bacterium and injects its own DNA into a cell of the target bacterium via the tail. Then, the phage utilizes the translational mechanism of the bacteria for self-reproduction to produce a large number of daughter phages and then lyses the bacteria to release the daughter phages to the outer space.

A "plant disease" as used herein is a generic term for diseases caused in a plant. A known plant disease includes a disease caused by an infectious pathogen such as a virus, bacteria, filamentous fungus, actinomycete, viroid, *Phytoplasma,* nematode, mite, or insect, or a disease caused by a noninfectious pathogen such as the lack or excess of a nutrient or water, drug poisoning, or the like. A plant disease herein refers to a disease whose pathogens are bacteria, i.e., a plant pathogenic bacterium, unless otherwise specified. A plant pathogenic bacterium herein corresponds to the above-described target bacteria, for example, bacteria of the *Xanthomonas* genus, unless otherwise specified.

"Control" as used herein refers to prevention or treatment (extermination) (from the home page of the Japan Crop Protection Association). Accordingly, "plant disease control" as used herein refers to the prevention of a plant disease, particularly against a target bacterium, or to the treatment of a plant disease caused by a target bacterium.

A "target plant" as used herein refers to a plant to which the below-described composition for plant disease control according to the present invention is to be applied. This plant is a plant that has a specific plant disease caused by the infection of the target bacteria, or a plant that has the possibility of being infected by the target bacteria.

### 1-3. Constitution

A bacteriolytic agent of the present invention consists of a bacteriophage.

### <1st Phage>

The 1st phage is characterized by comprising, in the genomic DNA, a gene encoding a tail fiber protein consisting of a specific amino acid sequence and exhibits bacteriolytic ability specifically to target bacteria.

### (1-1) Tail Fiber Protein

The tail fiber protein consists of the amino acid sequence of SEQ ID NO: 1 composed of 1371 amino acid residues, the amino acid sequence having addition, deletion, and/or substitution of one or a plurality of amino acids in the amino acid sequence of SEQ ID NO: 1, or an amino acid sequence having an amino acid sequence identity of 90% or more, 95% or more, 96% or more, 97% or more, 98% or more, or 99% or more to the amino acid sequence of SEQ ID NO: 1. Each of the tail fiber proteins is characterized by having target-bacteria-specific recognizing ability.

In this regard, "a plurality" as used herein refers to 2 to 10, for example, 2 to 7, 2 to 5, 2 to 4, or 2 to 3. Additionally, a "substitution (of an amino acid)" refers to a substitution within a group of conservative amino acids, in which the amino acids are similar in properties such as the electric charge, side chain, polarity, and aromaticity among the 20 kinds of amino acids constituting natural proteins. The substitution is, for example, within the uncharged polarity amino acid group having a low-polarity side chain (Gly, Asn, Gln, Ser, Thr, Cys, and Tyr), the branched-chain amino acid group (Leu, Val, and Ile), the neutral amino acid group (Gly, Ile, Val, Leu, Ala, Met, and Pro), the neutral amino acid group having a hydrophilic side chain (Asn, Gln, Thr, Ser, Tyr, and Cys), the acidic amino acid group (Asp and Glu), the basic amino acid group (Arg, Lys, and His), and the aromatic amino acid group (Phe, Tyr, and Trp). An amino acid substitution in such a group is known to be less likely to change the properties of a polypeptide, and thus, is preferable.

Furthermore, an "amino acid sequence identity" as used herein is a value that indicates the ratio of the sites at which the kinds of amino acid residues are the same in a range to be compared between two amino acid sequences. Even when the two amino acid sequences are different in length, the amino acid sequence identity can be calculated by aligning the amino acids so that the degree of coincidence of the amino acids in a range to be compared becomes the highest. Without limitation, a typical algorithm for such an analysis is BLAST. BLAST may be utilized with various software or web services. An amino acid sequence identity may be calculated easily, utilizing, for example, genetic information processing software GENETYX (https://www.genetyx.cojp/) or the NCBI-provided BLAST server (https://blast.ncbi.nlm.nih.gov/Blast.cgi). Besides BLAST, an algorithm called FASTA, or the like, may be utilized, as long as it is capable of calculating an appropriate identity.

The present inventors have discovered three kinds of phages having bacteriolytic ability against bacteria of the *Xanthomonas* genus, and identified a tail fiber gene from each of the genomic DNA sequences (SEQ ID NOs: 8 to 10, respectively) of these phages. The amino acid sequences (SEQ ID NOs: 2 to 4) encoded by these tail fiber genes were analyzed using the genetic information processing software GENETYX and were consequently found to have a high sequence identity of 98% or more to each other. SEQ ID NO: 1 is an amino acid sequence common among the above-described amino acid sequences of SEQ ID NOs: 2 to 4.

Accordingly, the amino acid sequence of SEQ ID NO: 1 may be the amino acid sequence of any of SEQ ID NOs: 2 to 4.

### (1-2) Tail Fiber Gene

The 1st bacteriophage comprises, in the genomic DNA of the phage, a tail fiber gene consisting of a nucleotide sequence encoding the tail fiber protein.

Specific examples of the nucleotide sequence of a tail fiber gene include: the nucleotide sequence of any of SEQ ID NOs: 5 to 7; the nucleotide sequence having addition, deletion, and/or substitution of one or a plurality of nucleotides in the nucleotide sequence of any of SEQ ID NOs: 5 to 7; furthermore, a nucleotide sequence having a sequence identity of 90% or more, 95% or more, 96% or more, 97% or more, 98% or more, or 99% or more to the nucleotide sequence of any of SEQ ID NOs: 5 to 7; and a nucleotide sequence that hybridizes with the nucleotide sequence complementary to the nucleotide sequence of any of SEQ ID NOs: 5 to 7 under highly stringent conditions.

As with the above-described amino acid sequence identity, a "nucleotide sequence identity" as used herein is a value that indicates the ratio of the sites at which the kinds of nucleotides are the same in a range to be compared between two nucleotide sequences. Even when the two nucleotide sequences are different in length, the nucleotide sequence identity can be calculated by aligning the nucleotides so that the degree of coincidence of the nucleotides in a range to be compared becomes the highest. Without limitation, a nucleotide sequence identity may be analyzed using an analysis algorithm such as MUMmer, besides the above-described BLAST and FASTA.

The "highly stringent conditions" as used herein refer to environmental conditions that are hardly prone to nonspecific hybridization. Under highly stringent conditions, a hybrid can be formed with a nucleic acid having a target nucleotide sequence, but a hybrid cannot substantially be formed with a nucleic acid having a nonspecific nucleotide sequence. Generally, highly stringent conditions refer to low-salt-concentration and high-temperature conditions. The low-salt concentration is, for example, 15 to 750 mM, preferably 15 to 500 mM, 15 to 300 mM, or 15 to 200 mM. Additionally, the high temperature is, for example, 50 to 68°C or 55 to 70°C. Specific examples of highly stringent conditions include conditions where hybridization is followed by washing with 0.1 × SSC and 0.1% SDS at 65°C.

Each of the proteins encoded by the tail fiber genes has bacteriolytic ability against target bacteria.

### (1-3) Genomic DNA

The 1st bacteriophage comprises the tail fiber gene. A gene and a nucleotide sequence other than the tail fiber gene are not limited. The phage genomic DNA is, for example, a genomic DNA consisting of the nucleotide sequence of any of SEQ ID NOs: 8 to 10 (201015 bp, 200185 bp, and 200277 bp respectively); the nucleotide sequence having addition, deletion, and/or substitution of one or a plurality of nucleotides in the nucleotide sequence of the region other than the tail fiber gene within the nucleotide sequence of any of SEQ ID NOs: 8 to 10; furthermore, a nucleotide sequence having a sequence identity of 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more to the nucleotide sequence of the region other than the tail fiber gene within the nucleotide sequence of any of SEQ ID NOs: 8 to 10; the nucleotide sequence having addition, deletion, and/or substitution of one or a plurality of nucleotides in the nucleotide sequence of any of SEQ ID NOs: 8 to 10; or furthermore, a nucleotide sequence having a sequence identity of 90.0% or more, 90.5% or more, 91.0% or more, 91.5% or more, 92.0% or more, 92.5% or more, 93.0% or more, 93.5% or more, 94.0% or more, 94.5% or more, 95.0% or more, 95.5% or more, 96.0% or more, 96.5% or more, 97.0% or more, 97.5% or more, 98.0% or more, 98.5% or more, 99.0% or more, 99.1% or more, 99.2% or more, 99.3% or more, 99.4% or more, 99.5% or more, 99.6% or more, 99.7% or more, 99.8% or more, or 99.9% or more when aligned with respect to the nucleotide sequence of any of SEQ ID NOs: 8 to 10.

In this regard, the software and analysis server used herein may indicate a sequence identity according to the index, such as Average Nucleotide Identity (ANI), and these may also be used. In this connection, for a very long phage genomic DNA, it is not easy to set the whole range as the range to be compared by alignment. Accordingly, the above-described sequence identity may be applicable for the range to be aligned automatically provided by the above-mentioned software or Web service. For example, in an analysis using the NCBI-provided BLAST server, the ratio of the range to be compared out of the whole range of phage genomic DNA is calculated as a value called Query Cover after the automatic alignment in the maximum alignable range. When a sequence identity is thus calculated with the partial range being aligned, the result may be used as a basis to estimate the sequence identity of the nucleotide sequence in the whole range of phage genomic DNA (the sequence identity of the whole range). For example, a value obtained by multiplying a Query Cover value by the value of a sequence identity in the range to be compared after alignment (the sequence identity in the range to be aligned) may be used as the estimated value of the sequence identity of the whole range. In this case, to increase the accuracy of the estimated value, a further correction may be added. For example, a sequence identity expected for a range other than the range to be aligned may be reckoned in.

For example, when a BLAST analysis is made using GENETYX-NGS packaged in GENETYX, the sequence identity (Average Nucleotide Identity: ANI) of SEQ ID NO: 8 to SEQ ID NO: 9 is 98% or more over the range of 96% or more of the full length, and the sequence identity to SEQ ID NO: 10 is 98% or more over the range of 95% or more of the full length. Accordingly, the sequence identity of the genomic DNA sequence of SEQ ID NO: 8 to the full length of the genomic DNA sequence of SEQ ID NO: 9 may be estimated to be 94% or more. Additionally, the sequence identity of the genomic DNA sequence of SEQ ID NO: 8 to the full length of the genomic DNA sequence of SEQ ID NO: 10 may be estimated to be 93% or more.

In this regard, the genomic DNA of a phage herein is packaged linearly or circularly. Additionally, in a next-generation genome sequencer analysis, the genomic DNA is fragmented, the nucleotide sequence of each of the fragments is read, and an analysis for connecting the sequences is performed to determine a sequence. In the case of a phage, the sequences are often connected without a reference genomic DNA sequence (de novo assembly). Therefore, it is difficult to unambiguously determine the start and end of a genome to be analyzed (Merrill, B.D., et al. BMC Genomics, 2016 17, 679). The starts and ends of genomic sequences in comparison may differ and are automatically taken into consideration in an analysis using software or an analysis server.

### (1-4) Effects

A bacteriolytic agent of the present invention comprising the 1st phage can exhibit bacteriolytic ability against a wide range of various bacterial species of *Xanthomonas* genus and can be used for various plant diseases.

Generally, a phage with too high specificity cannot cover the diversity of target bacteria and has a limited effect. Additionally, it is more preferable, from an industrial viewpoint, that a phage can be used for a plurality of plant diseases. Accordingly, a phage that exhibits bacteriolytic ability against a wide range of various bacterial species as a bacteriolytic agent of the present invention is extremely useful.

### <2nd Phage>

The 2nd phage is characterized by comprising, in the genomic DNA, a gene encoding a tail fiber protein consisting of a specific amino acid sequence and exhibits bacteriolytic ability specifically to target bacteria.

### (2-1) Tail Fiber Protein

The tail fiber protein consists of: the amino acid sequence of SEQ ID NO: 11 composed of 487 amino acid residues, or the amino acid sequence having substitution of one amino acid other than at positions 278 and 350 in the amino acid sequence of SEQ ID NO: 11. The tail fiber protein consisting of the amino acid sequence of SEQ ID NO: 11, or a variant thereof, can allow extremely useful host specificity, i.e., being specific to bacteria in a specific genus and exhibiting bacteriolytic ability against a wide range of various bacterial species in the specific genus.

As described above, in the case of the amino acid sequence having substitution of one amino acid in the amino acid sequence of SEQ ID NO: 11, the position of substitution is not particularly limited, as long as it is other than positions 278 and 350. For example, one arbitrary amino acid may be substituted in the range from position 1 to position 250 in the amino acid sequence of SEQ ID NO: 11. Specifically, for example, one amino acid may be substituted at position 50 or after, 55 or after, 60 or after, 65 or after, 66 or after, 67 or after, 68 or after, 70 or after, 80 or after, 90 or after, 100 or after, 110 or after, 120 or after, 130 or after, 140 or after, 145 or after, 150 or after, 151 or after, 152 or after, 153 or after, or 154 or after in the amino acid sequence of SEQ ID NO: 11. Additionally, for example, one amino acid may be substituted at position 200 or before, 190 or before, 180 or before, 170 or before, 160 or before, 159 or before, 158 or before, 157 or before, 156 or before, 155 or before, or 154 or before in the amino acid sequence of SEQ ID NO: 11. For example, one amino acid may be substituted at a position between 50 and 200, between 67 and 156, between 80 and 156, between 100 and 156, between 110 and 156, between 120 and 156, between 130 and 156, between 140 and 156, between 150 and 156, between 153 and 156, between 154 and 156, or at position 154. The amino acid region from position 67 to position 156 in the amino acid sequence of SEQ ID NO: 11 conceivably forms one domain. Hence, when one amino acid is substituted and the amino acid is in this amino acid region, the resulting phage can have the characteristics of the 2nd phage, regardless of the position of the amino acid.

The amino acid substitution is not particularly limited to any type. For example, it may be a conservative substitution or a substitution within the amino acid groups having a low-polarity side chain (Gly, Asn, Gln, Ser, Thr, Cys, Tyr, Leu, Val, Ile, Val, Ala, Met, and Pro). For example, the substitution may be between the uncharged polar amino acid group (Gly, Asn, Gln, Ser, Thr, Cys, or Tyr) or the neutral amino acid group having a hydrophilic side chain (Asn, Gln, Thr, Ser, Tyr, or Cys) and the neutral amino acid group (Gly, Ile, Val, Leu, Ala, Met, or Pro). More specifically, the substitution may be, for example, between threonine (Thr) and alanine (Ala). One example of such a substituted amino acid sequence is the amino acid sequence of SEQ ID NO: 45. This sequence is the amino acid sequence having a substitution of the amino acid at position 154, threonine (Thr), with alanine (Ala) in the amino acid sequence of SEQ ID NO: 11.

### (2-2) Tail Fiber Gene

The 2nd bacteriophage comprises, in the genomic DNA of the phage, a tail fiber gene consisting of a nucleotide sequence encoding the tail fiber protein.

A specific nucleotide sequence of the tail fiber gene is not particularly limited, as long as it encodes the amino acid sequence of SEQ ID NO: 11 or the amino acid sequence having substitution of one amino acid other than at positions 278 and 350 in the amino acid sequence of SEQ ID NO: 11. An example is a gene consisting of the nucleotide sequence of SEQ ID NO: 12 encoding the amino acid sequence of SEQ ID NO: 11. Another example is a gene consisting of the nucleotide sequence of SEQ ID NO: 46 encoding the amino acid sequence of SEQ ID NO: 45.

A protein encoded by the tail fiber gene can allow extremely useful host specificity, i.e., being specific to bacteria in a specific genus and exhibiting bacteriolytic ability against a wide range of various bacterial species in the specific genus.

### (2-3) Genomic DNA

The 2nd bacteriophage comprises the tail fiber gene. A gene and a nucleotide sequence other than the tail fiber gene are not limited. The phage genomic DNA is, for example, a genomic DNA consisting of the 63753 bp nucleotide sequence of SEQ ID NO: 13; the 63743 bp nucleotide sequence of SEQ ID NO: 47 or 48; the nucleotide sequence having addition, deletion, and/or substitution of one or a plurality of nucleotides in the nucleotide sequence in the region other than the tail fiber gene within the nucleotide sequence of SEQ ID NO: 13, 47, or 48; furthermore, a nucleotide sequence having a sequence identity of 98.5% or more, 98.6% or more, 98.7% or more, 98.8% or more, or 98.9% or more, 99.0% or more, 99.1% or more, 99.2% or more, 99.3% or more, 99.4% or more, 99.5% or more, 99.6% or more, 99.7% or more, 99.8% or more, or 99.9% or more to the nucleotide sequence in the region other than the tail fiber gene within the nucleotide sequence of SEQ ID NO: 13, 47, or 48; the nucleotide sequence having addition, deletion, and/or substitution of one or a plurality of nucleotides in the nucleotide sequence of SEQ ID NO: 13, 47 or 48; furthermore, a nucleotide sequence having a sequence identity of 99.0% or more, 99.1% or more, 99.2% or more, 99.3% or more, 99.4% or more, 99.5% or more, 99.6% or more, 99.7% or more, 99.8% or more, or 99.9% or more when aligned with respect to the nucleotide sequence of SEQ ID NO: 13, 47 or 48; or a nucleotide sequence that hybridizes, under highly stringent conditions, with the nucleotide sequence of the region other than the tail fiber gene in the nucleotide sequence of SEQ ID NO: 13, 47, or 48.

### (2-4) Effects

A bacteriolytic agent of the present invention comprising the 2nd phage can exhibit bacteriolytic ability against a wide range of various bacterial species of *Xanthomonas* genus and can be used for various plant diseases.

Generally, a phage with too high specificity cannot cover the diversity of target bacteria and has a limited effect. Additionally, it is more preferable, from an industrial viewpoint, that a phage may be used for a plurality of plant diseases. Accordingly, a phage that exhibits bacteriolytic ability against a wide range of various bacterial species as a bacteriolytic agent comprising the 2nd phage according to the present invention is extremely useful.

### <3rd Phage>

The 3rd phage is characterized by having a genomic DNA sequence comprising a specific nucleotide sequence and exhibits specific bacteriolytic ability against target bacteria.

### (3-1) Genomic DNA

The genomic DNA sequence comprises or consists of: the 61291 bp nucleotide sequence of SEQ ID NO: 14; the nucleotide sequence having addition, deletion, and/or substitution of one or a plurality of nucleotides in the nucleotide sequence of SEQ ID NO: 14; or a nucleotide sequence having a sequence identity of 80% or more, 83% or more, 85% or more, 88% or more, 90% or more, 95% or more, 96% or more, 97% or more, 98% or more, or 99% or more to the nucleotide sequence of SEQ ID NO: 14. A bacteriophage comprising any of the genomic DNA sequences is characterized in that the bacteriophage can be specifically attached to target bacteria, and inject the genomic DNA into a cell of the target bacteria.

### (3-2) Effects

A bacteriolytic agent of the present invention comprising the 3rd phage can exhibit bacteriolytic ability against bacteria of the *Xanthomonas* genus and can be used for a plant disease.

### <4th Phage>

The 4th phage is characterized by comprising, in the genomic DNA, a gene encoding a tail fiber protein consisting of a specific amino acid sequence and exhibits bacteriolytic ability specifically to target bacteria.

### (4-1) Tail Fiber Protein

The tail fiber protein consists of the amino acid sequence of SEQ ID NO: 15 composed of 604 amino acid residues, the amino acid sequence having addition, deletion, and/or substitution of one or a plurality of amino acids in the amino acid sequence of SEQ ID NO: 15, or an amino acid sequence having an amino acid sequence identity of 90% or more, 95% or more, 96% or more, 97% or more, 98% or more, or 99% or more to the amino acid sequence of SEQ ID NO: 15. Each of the tail fiber proteins is characterized by having target-bacteria-specific recognizing ability.

### (4-2) Tail Fiber Gene

The 4th bacteriophage comprises, in the genomic DNA of the phage, a tail fiber gene consisting of a nucleotide sequence encoding the tail fiber protein.

A specific nucleotide sequence of the tail fiber gene is, for example, the nucleotide sequence of SEQ ID NO: 16 encoding the amino acid sequence of SEQ ID NO: 15; the nucleotide sequence having addition, deletion, and/or substitution of one or a plurality of nucleotides in the nucleotide sequence of SEQ ID NO: 16; furthermore, a nucleotide sequence having a nucleotide sequence identity of 90% or more, 95% or more, 96% or more, 97% or more, 98% or more, or 99% or more to the nucleotide sequence of SEQ ID NO: 16; or a nucleotide sequence that hybridizes with the nucleotide sequence complementary to the nucleotide sequence of SEQ ID NO: 16 under highly stringent conditions.

Each of the proteins encoded by the tail fiber genes has bacteriolytic ability against target bacteria.

### (4-3) Genomic DNA

The 4th bacteriophage comprises the tail fiber gene. A gene and a nucleotide sequence other than the tail fiber gene are not limited. For example, the phage genomic DNA is a genomic DNA consisting of: the 46393 bp nucleotide sequence of SEQ ID NO: 17; the nucleotide sequence having addition, deletion, and/or substitution of one or a plurality of nucleotides in the nucleotide sequence of the region other than the tail fiber gene within the nucleotide sequence of SEQ ID NO: 17; furthermore, a nucleotide sequence having a sequence identity of 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more to the nucleotide sequence of the region other than the tail fiber gene within the nucleotide sequence of SEQ ID NO: 17; the nucleotide sequence having addition, deletion, and/or substitution of one or a plurality of nucleotides in the nucleotide sequence of SEQ ID NO: 17; or furthermore, a nucleotide sequence having a sequence identity of 90.0% or more, 90.5% or more, 91.0% or more, 91.5% or more, 92.0% or more, 92.5% or more, 93.0% or more, 93.5% or more, 94.0% or more, 94.5% or more, 95.0% or more, 95.5% or more, 96.0% or more, 96.5% or more, 97.0% or more, 97.5% or more, 98.0% or more, 98.5% or more, 99.0% or more, 99.1% or more, 99.2% or more, 99.3% or more, 99.4% or more, 99.5% or more, 99.6% or more, 99.7% or more, 99.8% or more, or 99.9% or more when aligned with respect to the nucleotide sequence of SEQ ID NO: 17.

### (4-4) Effects

A bacteriolytic agent of the present invention comprising the 4th phage can exhibit bacteriolytic ability against bacteria of the *Xanthomonas* genus and can be used for a plant disease.

### <5th Phage>

The 5th phage is characterized by having a genomic DNA sequence comprising a specific nucleotide sequence, and a bacteriolytic agent of the present invention exhibits specific bacteriolytic ability against target bacteria.

### (5-1) Genomic DNA

The genomic DNA sequence of the 5th bacteriophage has: the 43177 bp nucleotide sequence of SEQ ID NO: 18 or the 43741 bp nucleotide sequence of SEQ ID NO: 19; the nucleotide sequence having addition, deletion, and/or substitution of one or a plurality of nucleotides in the nucleotide sequence of SEQ ID NO: 18 or 19; a nucleotide sequence having a sequence identity of 90% or more, 95% or more, 96% or more, 97% or more, 98% or more, or 99% or more to the nucleotide sequence of SEQ ID NO: 18 or 19; a nucleotide sequence having a sequence identity of 95.0% or more, 95.5% or more, 96.0% or more, 96.5% or more, 97.0% or more, 97.5% or more, 98.0% or more, 98.5% or more, 99.0% or more, 99.1% or more, 99.2% or more, 99.3% or more, 99.4% or more, 99.5% or more, 99.6% or more, 99.7% or more, 99.8% or more, or 99.9% or more when aligned with respect to the nucleotide sequence of SEQ ID NO: 18 or 19; or furthermore a nucleotide sequence that hybridizes with the nucleotide sequence complementary to the nucleotide sequence of SEQ ID NO: 18 or 19 under highly stringent conditions.

### (5-2) Effects

A bacteriolytic agent of the present invention comprising the 5th phage can exhibit bacteriolytic ability against bacteria of the *Xanthomonas* genus and can be used for a plant disease.

### <6th Phage>

The 6th phage is characterized by comprising, in the genomic DNA, a gene encoding a protein newly discovered in the present invention (herein often referred to simply as a "protein of the present invention") and exhibits specific bacteriolytic ability against target bacteria.

### (6-1) the Protein of the Present Invention

The protein of the present invention consists of the amino acid sequence of SEQ ID NO: 21 composed of 84 amino acid residues, the amino acid sequence having addition, deletion, and/or substitution of one or a plurality of amino acids in the amino acid sequence of SEQ ID NO: 21, or an amino acid sequence having an amino acid sequence identity of 90% or more, 95% or more, 96% or more, 97% or more, 98% or more, or 99% or more to the amino acid sequence of SEQ ID NO: 21. Each of the proteins of the present invention is characterized by having target-bacteria-specific recognizing ability.

A protein of the present invention is a novel protein that plays an important role in determining a host range.

### (6-2) Gene Encoding the Protein of the Present Invention

The 6th bacteriophage comprises, in the genomic DNA of the phage, a gene encoding the protein of the present invention (herein often referred to simply as a "gene of the present invention").

The gene of the present invention is not particularly limited, as long as it encodes the amino acid sequence of SEQ ID NO: 21. A specific example of the nucleotide sequence is, for example, the nucleotide sequence of SEQ ID NO: 22 encoding the amino acid sequence of SEQ ID NO: 21; the nucleotide sequence having addition, deletion, and/or substitution of one or a plurality of nucleotides in the nucleotide sequence of SEQ ID NO: 22; furthermore, a nucleotide sequence having a nucleotide sequence identity of 90% or more, 95% or more, 96% or more, 97% or more, 98% or more, or 99% or more to the nucleotide sequence of SEQ ID NO: 22; or a nucleotide sequence that hybridizes with the nucleotide sequence complementary to the nucleotide sequence of SEQ ID NO: 22 under highly stringent conditions.

Each of the proteins encoded by the gene of the present invention has bacteriolytic ability against target bacteria.

### (6-3) Genomic DNA

The 6th bacteriophage comprises the gene of the present invention. A gene and a nucleotide sequence other than the gene of the present invention are not limited. For example, the phage genomic DNA is a genomic DNA consisting of: the 42708 bp nucleotide sequence of SEQ ID NO: 23; the nucleotide sequence having addition, deletion, and/or substitution of one or a plurality of nucleotides in the nucleotide sequence of the region other than the gene of the present invention within the nucleotide sequence of SEQ ID NO: 23; furthermore, a nucleotide sequence having a sequence identity of 95% or more, 96% or more, 97% or more, 98% or more, or 99% or more to the nucleotide sequence of the region other than the gene of the present invention within the nucleotide sequence of SEQ ID NO: 23; the nucleotide sequence having addition, deletion, and/or substitution of one or a plurality of nucleotides in the nucleotide sequence of SEQ ID NO: 23; or furthermore, a nucleotide sequence having a sequence identity of 95.0% or more, 95.5% or more, 96.0% or more, 96.5% or more, 97.0% or more, 97.5% or more, 98.0% or more, 98.5% or more, 99.0% or more, 99.1% or more, 99.2% or more, 99.3% or more, 99.4% or more, 99.5% or more, 99.6% or more, 99.7% or more, 99.8% or more, or 99.9% or more when aligned with respect to the nucleotide sequence of SEQ ID NO: 23.

### (6-4) Effects

A bacteriolytic agent of the present invention comprising the 6th phage can exhibit bacteriolytic ability against bacteria of the *Xanthomonas* genus, and can be used for a plant disease.

### <7th Phage>

The 7th phage is characterized by comprising, in the genomic DNA, a gene encoding a tail tubular protein consisting of a specific amino acid sequence and exhibits bacteriolytic ability specifically to target bacteria.

### (7-1) Tail Tubular Protein

The tail tubular protein encompasses a tail tubular protein A and a tail tubular protein B. The tail tubular protein A consists of: the amino acid sequence of SEQ ID NO: 24 or 49 composed of 205 amino acid residues, the amino acid sequence having addition, deletion, and/or substitution of one or a plurality of amino acids in the amino acid sequence of SEQ ID NO: 24 or 49, or an amino acid sequence having an amino acid sequence identity of 97% or more, 97.5% or more, 98% or more, 98.5% or more, 99% or more, or 99.5% or more to the amino acid sequence of SEQ ID NO: 24 or 49.

The amino acid substitution here may comprise an amino acid substitution(s) at one or more positions selected from the group consisting of positions 28, 108, 110, 153, 157, 189, and 201 in the amino acid sequence of SEQ ID NO: 24 or 49. The substitutions include substitutions, for example, at two or more, three or more, four or more, five or more, six or more, or all positions among these positions.

The amino acid substitution is not particularly limited to any specific type. For example, the substitution may be a conservative substitution, or may comprise one or a plurality of nonconservative substitutions. Specifically, the amino acid sequence of the tail tubular protein A of the 7th phage may be, for example, the amino acid sequence of SEQ ID NO: 60. This amino acid sequence is the same amino acid sequence as SEQ ID NO: 24 or 49, except for the amino acids at the following positions: glutamic acid (Glu) or aspartic acid (Asp) at position 28; serine (Ser) or asparagine (Asn) at position 108; glutamine (Gln) or histidine (His) at position 110; glutamine (Gln) or lysine (Lys) at position 153; aspartic acid (Asp) or asparagine (Asn) at position 157; tyrosine (Tyr) or phenyl alanine (Phe) at position 189; and valine (Val) or tyrosine (Tyr) at position 201.

The tail tubular protein B consists of the amino acid sequence of SEQ ID NO: 25 (correctly SEQ ID NO: 57) or 50, composed of 834 amino acid residues, the amino acid sequence having addition, deletion, and/or substitution of one or a plurality of amino acids in the amino acid sequence of SEQ ID NO: 25 (correctly SEQ ID NO: 57) or 50, or an amino acid sequence having an amino acid sequence identity of 97% or more, 97.5% or more, 97.6% or more, 98% or more, 98.5% or more, 99% or more, or 99.5% or more to the amino acid sequence of SEQ ID NO: 25 (correctly SEQ ID NO: 57) or 50.

The amino acid substitution here may comprise an amino acid substitution at one or more positions selected from the group consisting of positions 25, 53, 216, 221, 272, 389, 395, 410, 425, 472, 607, 623, 662, 670, 699, 707, 757, 763, 764, and 765 in the amino acid sequence of SEQ ID NO: 57 or 50. The substitutions may include substitutions, for example, at 2 or more, 3 or more, 4 or more, 5 or more, 10 or more, 15 or more, or all positions among these positions.

The amino acid substitution is not particularly limited to any specific type. For example, the substitution may be a conservative substitution, or may comprise one or a plurality of nonconservative substitutions. Specifically, the amino acid sequence of the tail tubular protein B of the 7th phage may be, for example, the amino acid sequence of SEQ ID NO: 61. This amino acid sequence is the same amino acid sequence as SEQ ID NO: 57 or 50, except for the amino acids at the following positions: alanine (Ala) or proline (Pro) at position 25; alanine (Ala) or serine (Ser) at position 53; alanine (Ala) or proline (Pro) at position 216; histidine (His) or tyrosine (Tyr) at position 221; valine (Val) or (Ile) at position 272; alanine (Ala) or glutamic acid (Glu) at position 389; serine (Ser) or alanine (Ala) at position 395; valine (Val) or isoleucine (Ile) at position 410; isoleucine (Ile) or valine (Val) at position 425; glycine (Gly) or alanine (Ala) at position 472; alanine (Ala) or serine (Ser) at position 607; isoleucine (Ile) or valine (Val) at position 623; arginine (Arg) or serine (Ser) at position 662; leucine (Leu) or glutamine (Gln) at position 670; threonine (Thr) or asparagine (Asn) at position 699; aspartic acid (Asp) or glycine (Gly) at position 707; serine (Ser) or alanine (Ala) at position 757; glycine (Gly) or serine (Ser) at position 763; serine (Ser) or asparagine (Asn) at position 764; methionine (Met) or valine (Val) at position 765.

Each of the tail tubular proteins is characterized by having an effect on target-bacteria-specific recognizing ability.

### (7-2) Tail Tubular Gene

The 7th bacteriophage comprises, in the genomic DNA of the phage, a tail tubular gene consisting of a nucleotide sequence encoding the tail tubular protein.

The tail tubular gene is not particularly limited, as long as it encodes a tail tubular protein. The tail tubular gene encompasses a tail tubular protein A gene and a tail tubular protein B gene.

A specific nucleotide sequence of the tail tubular protein A gene is, for example, the nucleotide sequence of SEQ ID NO: 26 or 51 encoding the amino acid sequence of SEQ ID NO: 24 or 49; the nucleotide sequence having addition, deletion, and/or substitution of one or a plurality of nucleotides in the nucleotide sequence of SEQ ID NO: 26 or 51; furthermore, a nucleotide sequence having a nucleotide sequence identity of 97% or more, 97.5% or more, 98% or more, 98.5% or more, 99% or more, or 99.5% or more to the nucleotide sequence of SEQ ID NO: 26 or 51; or a nucleotide sequence that hybridizes with the nucleotide sequence complementary to the nucleotide sequence of SEQ ID NO: 26 or 51 under highly stringent conditions. The nucleotide sequence of the tail tubular protein A gene may be the nucleotide sequence encoding the amino acid sequence of SEQ ID NO: 60.

A specific nucleotide sequence of the tail tubular protein B gene is, for example, the nucleotide sequence of SEQ ID NO: 27 (correctly SEQ ID NO: 58) or 52 encoding the amino acid sequence of SEQ ID NO: 25 (correctly SEQ ID NO: 57) or 50; the nucleotide sequence having addition, deletion, and/or substitution of one or a plurality of nucleotides in the nucleotide sequence of SEQ ID NO: 27 (correctly SEQ ID NO: 58) or 52; furthermore, a nucleotide sequence having a nucleotide sequence identity of 97% or more, 97.5% or more, 98% or more, 98.5% or more, 99% or more, or 99.5% or more to the nucleotide sequence of SEQ ID NO: 27 (correctly SEQ ID NO: 58) or 52; or a nucleotide sequence that hybridizes with the nucleotide sequence complementary to the nucleotide sequence of SEQ ID NO: 27 (correctly SEQ ID NO: 58) or 52 under highly stringent conditions. The nucleotide sequence of the tail tubular protein B gene may be the nucleotide sequence encoding the amino acid sequence of SEQ ID NO: 61.

Each of the proteins encoded by the tail tubular genes has bacteriolytic ability against target bacteria.

### (7-3) Genomic DNA

The 7th bacteriophage comprises the tail tubular gene. A gene and a nucleotide sequence other than the tail tubular gene are not limited. For example, the phage genomic DNA is a genomic DNA consisting of: the 44716 bp nucleotide sequence of SEQ ID NO: 28, the 44829 bp nucleotide sequence of SEQ ID NO: 29, or the 44585 bp nucleotide sequence of SEQ ID NO: 53: the nucleotide sequence having addition, deletion, and/or substitution of one or a plurality of nucleotides in the nucleotide sequence of the region other than the tail tubular gene within the nucleotide sequence of SEQ ID NO: 28, 29, or 53; furthermore, a nucleotide sequence having a sequence identity of 95% or more, 96% or more, 97% or more, 98% or more, or 99% or more to the nucleotide sequence of the region other than the tail tubular gene within the nucleotide sequence of SEQ ID NO: 28, 29, or 53; the nucleotide sequence having addition, deletion, and/or substitution of one or a plurality of nucleotides in the nucleotide sequence of SEQ ID NO: 28, 29, or 53; or furthermore, a nucleotide sequence having a sequence identity of 95.0% or more, 95.5% or more, 96.0% or more, 96.5% or more, 97.0% or more, 97.5% or more, 98.0% or more, 98.5% or more, 99.0% or more, 99.1% or more, 99.2% or more, 99.3% or more, 99.4% or more, 99.5% or more, 99.6% or more, 99.7% or more, 99.8% or more, or 99.9% or more when aligned with respect to the nucleotide sequence of SEQ ID NO: 28, 29, or 53.

### (7-4) Effects

A bacteriolytic agent of the present invention comprising the 7th phage can exhibit bacteriolytic ability against a wide range of various bacterial species of *Xanthomonas* genus, and can be used for various plant diseases.

Generally, a phage with too high specificity cannot cover the diversity of target bacteria and has a limited effect. Additionally, it is more preferable, from an industrial viewpoint, that a phage may be used for a plurality of plant diseases. Accordingly, a phage that exhibits bacteriolytic ability against two or more bacterial species as a bacteriolytic agent comprising the 7th phage according to the present invention is extremely useful.

### <8th Phage>

The 8th phage is characterized by having a genomic DNA sequence comprising a specific nucleotide sequence and exhibits specific bacteriolytic ability against target bacteria.

### (8-1) Genomic DNA

The genomic DNA sequence may be a genomic DNA sequence comprising or consisting of the nucleotide sequence of any of SEQ ID NOs: 32 to 36 (44388 bp, 44377 bp, 45279 bp, 44378 bp, and 44408 bp respectively); the nucleotide sequence having addition, deletion, and/or substitution of one or a plurality of nucleotides in the nucleotide sequence of any of SEQ ID NOs: 32 to 36; or a nucleotide sequence having a sequence identity of 80% or more, 81% or more, 82% or more, 83% or more, 84% or more, 85% or more, 86% or more, 87% or more, 88% or more, 89% or more, 90% or more, 90.5% or more, 91.0% or more, 91.5% or more, 92.0% or more, 92.5% or more, 93.0% or more, 93.5% or more, 94.0% or more, 94.5% or more, 95.0% or more, 95.5% or more, 96.0% or more, 96.5% or more, 97.0% or more, 97.5% or more, 98.0% or more, 98.5% or more, 99.0% or more, 99.1% or more, 99.2% or more, 99.3% or more, 99.4% or more, 99.5% or more, 99.6% or more, 99.7% or more, 99.8% or more, or 99.9% or more when aligned with respect to the nucleotide sequence of any of SEQ ID NOs: 32 to 36.

### (8-2) Effects

A bacteriolytic agent of the present invention comprising the 8th phage can exhibit bacteriolytic ability against bacteria of the *Xanthomonas* genus and can be used for a plant disease.

### <9th Phage>

The 9th phage is characterized by comprising, in the genomic DNA, a gene encoding a tail tubular protein composed of a specific amino acid sequence and exhibits bacteriolytic ability specifically to target bacteria.

### (9-1) Tail Tubular Protein

The tail tubular protein encompasses a tail tubular protein A and a tail tubular protein B. The tail tubular protein A consists of the amino acid sequence of SEQ ID NO: 37 composed of 206 amino acid residues, the amino acid sequence having addition, deletion, and/or substitution of one or a plurality of amino acids in the amino acid sequence of SEQ ID NO: 37, or an amino acid sequence having an amino acid sequence identity of 92% or more, 93% or more, 94% or more, 95% or more, 96% or more, 97% or more, 97.5% or more, 98% or more, 98.5% or more, 99% or more, or 99.5% or more to the amino acid sequence of SEQ ID NO: 37.

The tail tubular protein B consists of the amino acid sequence of SEQ ID NO: 38 or 54 composed of 847 amino acid residues, the amino acid sequence having addition, deletion, and/or substitution of one or a plurality of amino acids in the amino acid sequence of SEQ ID NO: 38 or 54, or an amino acid sequence having an amino acid sequence identity of 92% or more, 93% or more, 94% or more, 95% or more, 96% or more, 97% or more, 97.5% or more, 98% or more, 98.5% or more, 99% or more, 99.1% or more, 99.2% or more, 99.3% or more, 99.4% or more, or 99.5% or more to the amino acid sequence of SEQ ID NO: 38 or 54.

The amino acid substitution here may comprise an amino acid substitution at one or more positions selected from the group consisting of positions 61, 108, 404, 679, 682, and 727 in the amino acid sequence of SEQ ID NO: 38 or 54. The substitutions may include substitutions, for example, at two or more, three or more, four or more, five or more, or all positions among these positions.

The amino acid substitution is not particularly limited to any specific type. For example, the substitution may be a conservative substitution or may comprise one or a plurality of nonconservative substitutions. For example, the substitution may be in the amino acid group having a low-polarity side chain (Gly, Asn, Gln, Ser, Thr, Cys, Tyr, Leu, Val, Ile, Val, Ala, Met, and Pro). For example, the substitution may be between the uncharged polar amino acid group (Gly, Asn, Gln, Ser, Thr, Cys, or Tyr) or the neutral amino acid group having a hydrophilic side chain (Asn, Gln, Thr, Ser, Tyr, or Cys) and the neutral amino acid group (Gly, Ile, Val, Leu, Ala, Met, or Pro). More specifically, the substitution may be, for example, between threonine (Thr) and alanine (Ala).

A specific example of the amino acid sequence may be the amino acid sequence of SEQ ID NO: 59. This amino acid sequence is the same amino acid sequence as SEQ ID NO: 38, except that the amino acids and the positions are as follows: the amino acid at position 61 is threonine (Thr) or alanine (Ala); the amino acid at position 108 is glutamine (Gln) or lysine (Lys); the amino acid at position 404 is proline (Pro) or glutamine (Gln); the amino acid at position 679 is proline (Pro) or serine (Ser); the amino acid at position 682 is threonine (Thr) or alanine (Ala); and the amino acid at position 727 is isoleucine (Ile) or valine (Val).

A phage in which the amino acid sequence of the tail tubular protein B consists of the amino acid sequence having addition, deletion, and/or substitution of one or a plurality of amino acids in the amino acid sequence of SEQ ID NO: 59, or the amino acid sequence having an amino acid sequence identity of 92% or more, 93% or more, 94% or more, 95% or more, 96% or more, 97% or more, 97.5% or more, 98% or more, 98.5% or more, 99% or more, 99.1% or more, 99.2% or more, 99.3% or more, 99.4% or more, or 99.5% or more to the amino acid sequence of SEQ ID NO: 59 is also encompassed in the 9th phage of the present invention.

Each of the tail tubular proteins is characterized by having an effect on target-bacteria-specific recognizing ability.

### (9-2) Tail Tubular Gene

The 9th bacteriophage comprises, in the genomic DNA of the phage, a tail tubular gene consisting of a nucleotide sequence encoding the tail tubular protein.

The tail tubular gene is not particularly limited, as long as it encodes a tail tubular protein. The tail tubular gene encompasses a tail tubular protein A gene and a tail tubular protein B gene.

A specific nucleotide sequence of the tail tubular protein A gene is, for example, the nucleotide sequence of SEQ ID NO: 39 encoding the amino acid sequence of SEQ ID NO: 37; the nucleotide sequence having addition, deletion, and/or substitution of one or a plurality of nucleotides in the nucleotide sequence of SEQ ID NO: 39; furthermore, a nucleotide sequence having a nucleotide sequence identity of 90% or more, 95% or more, 96% or more, 97% or more, 97.5% or more, 98% or more, 98.5% or more, 99% or more, or 99.5% or more to the nucleotide sequence of SEQ ID NO: 39; or a nucleotide sequence that hybridizes with the nucleotide sequence complementary to the nucleotide sequence of SEQ ID NO: 39 under highly stringent conditions. The nucleotide sequence of the tail tubular protein A gene may be the nucleotide sequence encoding the amino acid sequence of SEQ ID NO: 59.

A specific nucleotide sequence of the tail tubular protein B gene is, for example, the nucleotide sequence of SEQ ID NO: 40 or 55 encoding the amino acid sequence of SEQ ID NO: 38 or 54; the nucleotide sequence having addition, deletion, and/or substitution of one or a plurality of nucleotides in the nucleotide sequence of SEQ ID NO: 40 or 55; furthermore, a nucleotide sequence having a nucleotide sequence identity of 90% or more, 95% or more, 96% or more, 97% or more, 97.5% or more, 98% or more, 98.5% or more, 99% or more, or 99.5% or more to the nucleotide sequence of SEQ ID NO: 40 or 55; or a nucleotide sequence that hybridizes with the nucleotide sequence complementary to the nucleotide sequence of SEQ ID NO: 40 or 55 under highly stringent conditions. A specific nucleotide sequence of the tail tubular protein B gene is, for example, the nucleotide sequence encoding the amino acid sequence of SEQ ID NO: 59.

Each of the proteins encoded by the tail tubular genes has bacteriolytic ability against target bacteria.

### (9-3) Genomic DNA

The 9th bacteriophage comprises the tail tubular gene. A gene and a nucleotide sequence other than the tail tubular gene are not limited. For example, the phage genomic DNA is a genomic DNA consisting of: the 43667 bp nucleotide sequence of SEQ ID NO: 41 or the 43336 bp nucleotide sequence of SEQ ID NO: 56: the nucleotide sequence having addition, deletion, and/or substitution of one or a plurality of nucleotides in the nucleotide sequence of the region other than the tail tubular gene within the nucleotide sequence of SEQ ID NO: 41 or 56; furthermore, a nucleotide sequence having a sequence identity of 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more to the nucleotide sequence of the region other than the tail tubular gene within the nucleotide sequence of SEQ ID NO: 41 or 56; the nucleotide sequence having addition, deletion, and/or substitution of one or a plurality of nucleotides in the nucleotide sequence of SEQ ID NO: 41 or 56; or furthermore, a nucleotide sequence having a sequence identity of 90% or more, 91% or more, 92% or more, 93% or more, 94% or more, 95.0% or more, 95.5% or more, 96.0% or more, 96.5% or more, 97.0% or more, 97.5% or more, 98.0% or more, 98.5% or more, 99.0% or more, 99.1% or more, 99.2% or more, 99.3% or more, 99.4% or more, 99.5% or more, 99.6% or more, 99.7% or more, 99.8% or more, or 99.9% or more when aligned with respect to the nucleotide sequence of SEQ ID NO: 41 or 56.

### (9-4) Effects

A bacteriolytic agent of the present invention can exhibit bacteriolytic ability against a wide range of various bacterial species of *Xanthomonas* genus and can be used for various plant diseases.

Generally, a phage with too high specificity cannot cover the diversity of target bacteria and has a limited effect. Additionally, it is more preferable, from an industrial viewpoint, that a phage may be used for a plurality of plant diseases. Accordingly, a phage that exhibits bacteriolytic ability against two or more bacterial species as a bacteriolytic agent of the present invention is extremely useful.

### <10th Phage>

The 10th phage is characterized by comprising, in the genomic DNA, a gene encoding a tail fiber protein consisting of a specific amino acid sequence and exhibits bacteriolytic ability specifically to target bacteria.

### (10-1) Tail Fiber Protein

The tail fiber protein consists of the amino acid sequence of SEQ ID NO: 42 composed of 568 amino acid residues, the amino acid sequence having addition, deletion, and/or substitution of one or a plurality of amino acids in the amino acid sequence of SEQ ID NO: 42, or an amino acid sequence having an amino acid sequence identity of 90% or more, 95% or more, 96% or more, 97% or more, 98% or more, or 99% or more to the amino acid sequence of SEQ ID NO: 42. Each of the tail fiber proteins is characterized by having target-bacteria-specific recognizing ability.

### (10-2) Tail Fiber Gene

The 10th bacteriophage comprises, in the genomic DNA of the phage, a tail fiber gene consisting of a nucleotide sequence encoding the tail fiber protein.

A specific nucleotide sequence of the tail fiber gene is, for example, the nucleotide sequence of SEQ ID NO: 43 encoding the amino acid sequence of SEQ ID NO: 42; the nucleotide sequence having addition, deletion, and/or substitution of one or a plurality of nucleotides in the nucleotide sequence of SEQ ID NO: 43; furthermore, a nucleotide sequence having a nucleotide sequence identity of 90% or more, 95% or more, 96% or more, 97% or more, 98% or more, or 99% or more to the nucleotide sequence of SEQ ID NO: 43; or a nucleotide sequence that hybridizes with the nucleotide sequence complementary to the nucleotide sequence of SEQ ID NO: 43 under highly stringent conditions.

Each of the proteins encoded by the tail fiber genes has bacteriolytic ability against target bacteria.

### (10-3) Genomic DNA

The 10th bacteriophage comprises the tail fiber gene. A gene other than the tail fiber gene and a nucleotide sequence are not limited. For example, the phage genomic DNA is a genomic DNA consisting of: the 76340 bp nucleotide sequence of SEQ ID NO: 44; the nucleotide sequence having addition, deletion, and/or substitution of one or a plurality of nucleotides in the nucleotide sequence of the region other than the tail fiber gene within the nucleotide sequence of SEQ ID NO: 44; furthermore, a nucleotide sequence having a sequence identity of 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more to the nucleotide sequence of the region other than the tail fiber gene within the nucleotide sequence of SEQ ID NO: 44; the nucleotide sequence having addition, deletion, and/or substitution of one or a plurality of nucleotides in the nucleotide sequence of SEQ ID NO: 44; or furthermore, a nucleotide sequence having a sequence identity of 90.0% or more, 90.5% or more, 91.0% or more, 91.5% or more, 92.0% or more, 92.5% or more, 93.0% or more, 93.5% or more, 94.0% or more, 94.5% or more, 95.0% or more, 95.5% or more, 96.0% or more, 96.5% or more, 97.0% or more, 97.5% or more, 98.0% or more, 98.5% or more, 99.0% or more, 99.1% or more, 99.2% or more, 99.3% or more, 99.4% or more, 99.5% or more, 99.6% or more, 99.7% or more, 99.8% or more, or 99.9% or more when aligned with respect to the nucleotide sequence of SEQ ID NO: 44.

### (10-4) Effects

A bacteriolytic agent of the present invention can exhibit bacteriolytic ability against a wide range of various bacterial species of *Xanthomonas* genus and can be used for various plant diseases.

### 2. Composition for Plant Disease Control

### 2-1. Overview

A second aspect of the present invention is a composition, particularly a composition usable for plant disease control. A composition of the present invention is characterized by comprising the bacteriolytic agent according to the first aspect as an active component.

Using the composition of the present invention for plant disease control can provide sustainable plant protectives (agricultural chemicals) against a bacterial plant disease, wherein the protectives are safe for the human body, free from drug poisoning to the environment, and capable of preventing or treating a specific plant disease of interest.

In this regard, the expression "composition for plant disease control" as used herein refers to the composition of the present invention used for plant disease control.

### 2-2. Constitution

### 2-2-1. Component

A composition of the present invention comprises, as an essential component, a bacteriophage that is a bacteriolytic agent described in the first aspect as an active component. Additionally, the composition may comprise an agriculturally acceptable carrier and/or medium to the extent that the bacteriolytic ability of the phage to target bacteria is not impaired or inhibited. Furthermore, the composition may comprise other active components, if desired. Each component is specifically described below.

### (1) Active Component (Bacteriolytic Agent)

A composition of the present invention comprises the bacteriolytic agent according to the first aspect as an essential active component. Because this active component lyses the target bacteria of the present invention, the composition for plant disease control can prevent or treat a plant disease caused by the target bacteria.

The specific constitution of the bacteriolytic agent has been described in detail in the first aspect and is thus omitted here.

When the composition is used for plant disease control, the amount of the active component comprised per unit amount in the composition depends on various conditions, such as the dosage form, the kind of plant pathogenic bacteria, the kind of a target plant, the site of application, and the method of application. The amount to be comprised is preferably sufficient for the phage, as an active component, to contact and infect plant pathogenic bacteria that have infected a target plant. Accordingly, it can be determined within the scope of common technical knowledge in the art, considering the conditions such that the bacteriolytic agent comprised in a composition for plant disease control according to the present invention is in an effective amount for target bacteria after application.

A composition for plant disease control according to the present invention may comprise, as an active component(s) in combination, one or more other phages that specifically recognize bacteria of the *Xanthomonas* genus and have bacteriolytic ability, as specifically exemplified below. For example, even if target bacteria are the same between phages, the phages, if capable of recognizing different cell surface receptors, can be expected to allow a synergistic effect or supportive effect of the bacteriolytic ability, depending on the combination.

When a composition of the present invention comprises a plurality of kinds of phages, the specific kind of each phage is not particularly limited. For example, the composition may comprise, as an active component, only the phage described herein, or may additionally comprise other arbitrary phages as a further active component. Additionally, the composition may comprise phages having a similar host range to each other and/or phages having not similar host range from each other. Examples of a combination of phages having a similar host range include: a combination that exhibits bacteriolytic ability against the same bacteria in the same genus (for example, *Xanthomonas* genus) in common; a combination of phages that exhibits bacteriolytic ability against one or more same species of bacteria in the same genus (for example, *Xanthomonas arboricola*) in common; and a combination of phages that exhibit bacteriolytic ability against one or more same pathovars of bacteria in the same genus (for example, *Xanthomonas arboricola* pv. *pruni*) in common. Additionally, examples of a combination of phages having not similar host range include: a combination of a phage that exhibits, and a phage that does not exhibit, bacteriolytic ability against bacteria belonging to a specific genus (for example, *Xanthomonas* genus); a combination of a phage that exhibits, and a phage that does not exhibit, bacteriolytic ability against bacteria of a specific pathovar in a specific genus (for example, *Xanthomonas arboricola* pv. *pruni*); a combination of a phage that exhibits, and a phage that does not exhibit, bacteriolytic ability against bacteria of a specific species in a specific genus (for example, *Xanthomonas arboricola*); and a combination of a phage that specifically exhibits, and a phage that nonspecifically exhibits, bacteriolytic ability against bacteria of a specific genus (for example, *Xanthomonas* genus), species (for example, *Xanthomonas arboricola*), or pathovar (for example, *Xanthomonas arboricola* pv. *pruni*).

The composition of the present invention may comprise, for example, a combination of two or more kinds of phages from the 1st to 10th phage described herein. Specifically, the composition may comprise, for example, a combination of two or more phages selected from the group consisting of the 1st phage, 2nd phage, 7th phage, 9th phage, and 10th phage. Alternatively, the composition may comprise, for example, a combination of two or more phages selected from the group consisting of the 3rd phage, 4th phage, 5th phage, 6th phage, and 8th phage. Furthermore, the composition may comprise, for example, a combination of the following: one or more phages selected from the group consisting of the 1st phage, 2nd phage, 7th phage, 9th phage, and 10th phage; and one or more phages selected from the group consisting of the 3rd phage, 4th phage, 5th phage, 6th phage, and 8th phage.

The number of phages comprised in the composition of the present invention, from the 1st to the 10th phage described herein, is not particularly limited, as long as it is one or more. The composition may comprise, for example, 2 or more kinds, 3 or more kinds, 4 or more kinds, 5 or more kinds, 6 or more kinds, 7 or more kinds, 8 or more kinds, 9 or more kinds, or 10 or more kinds of phages. Additionally, the composition of the present invention may comprise a plurality of kinds of phages that have genomic DNA sequences different from each other but are encompassed in the scope of each phage (for example, the 2nd phage or the like). Specifically, the composition may comprise two or more kinds of 2nd phages and/or two or more kinds of 7th phages.

For example, the phages encompass a phage comprising, in the genomic DNA, a gene encoding the amino acid sequence of SEQ ID NO: 1 as an amino acid sequence common to the tail fiber proteins, specifically a phage comprising, in the genomic DNA, a gene encoding the amino acid sequence of any of SEQ ID NOs: 2 to 4, and a phage comprising, in the genomic DNA, a gene encoding the amino acid sequence having addition, deletion, and/or substitution of one or a plurality of amino acids in the amino acid sequence of any of SEQ ID NOs: 1 to 4, or an amino acid sequence having a sequence identity of 90% or more to any of SEQ ID NOs: 1 to 4. Specific examples of such a gene encoding an amino acid sequence include a gene consisting of: the nucleotide sequence of any of SEQ ID NOs: 5 to 7; the nucleotide sequence having addition, deletion, and/or substitution of one or a plurality of nucleotides in the nucleotide sequence of any of SEQ ID NOs: 5 to 7; or a nucleotide sequence having a sequence identity of 90% or more to any of SEQ ID NOs: 5 to 7. Examples of the genomic DNA of a phage comprising such a gene include: a genomic DNA consisting of the nucleotide sequence of any of SEQ ID NOs: 8 to 10; and a genomic DNA consisting of: the nucleotide sequence having addition, deletion, and/or substitution of one or a plurality of nucleotides in the nucleotide sequence of any of SEQ ID NOs: 8 to 10; a nucleotide sequence having a sequence identity of 90% or more to any of SEQ ID NOs: 8 to 10; the nucleotide sequence having addition, deletion, and/or substitution of one or a plurality of nucleotides in the nucleotide sequence other than the nucleotide sequence encoding the amino acid sequence of any of SEQ ID NOs: 2 to 4 within the nucleotide sequence of any of SEQ ID NOs: 8 to 10; or a nucleotide sequence having a sequence identity of 80% or more to the nucleotide sequence other than the nucleotide sequence encoding the amino acid sequence of any of SEQ ID NOs: 2 to 4 within the nucleotide sequence of any of SEQ ID NOs: 8 to 10.

Additional examples include: a phage comprising, in the genomic DNA, a gene encoding the amino acid sequence of SEQ ID NO: 11 or 45 as the amino acid sequence of the tail fiber protein; and a phage comprising, in the genomic DNA, a gene encoding the amino acid sequence having addition, deletion, and/or substitution of one or a plurality of amino acids other than at positions 278 and 350 in the amino acid sequence of SEQ ID NO: 11, or an amino acid sequence having a sequence identity of 90% or more to SEQ ID NO: 11. Specific examples of such a gene encoding an amino acid sequence include a gene consisting of: the nucleotide sequence of SEQ ID NO: 12 or 46; the nucleotide sequence having addition, deletion, and/or substitution of one or a plurality of nucleotides in the nucleotide sequence of SEQ ID NO: 12 or 46; or a nucleotide sequence having a sequence identity of 90% or more to SEQ ID NO: 12 or 46. Furthermore, specific examples of the genomic DNA of a phage comprising such a gene include: a genomic DNA consisting of the nucleotide sequence of SEQ ID NO: 13, 47, or 48; and a genomic DNA consisting of: the nucleotide sequence having addition, deletion, and/or substitution of one or a plurality of nucleotides in the nucleotide sequence of SEQ ID NO: 13, 47, or 48; a nucleotide sequence having a sequence identity of 90% or more to SEQ ID NO: 13, 47, or 48; the nucleotide sequence having addition, deletion, and/or substitution of one or a plurality of nucleotides in the nucleotide sequence other than the nucleotide sequence encoding the amino acid sequence of SEQ ID NO: 11 or 45 within the nucleotide sequence of SEQ ID NO: 13, 47, or 48; or a nucleotide sequence having a sequence identity of 80% or more to the nucleotide sequence other than the nucleotide sequence encoding the amino acid sequence of SEQ ID NO: 11 or 45 within the nucleotide sequence of SEQ ID NO: 13, 47, or 48.

Furthermore, examples of the genomic DNA of the phage include: a genomic DNA comprising the nucleotide sequence of any of SEQ ID NOs: 14, 18, 19, and 32 to 36; a genomic DNA comprising the nucleotide sequence having addition, deletion, and/or substitution of one or a plurality of nucleotides in the nucleotide sequence of any of SEQ ID NOs: 14, 18, 19, and 32 to 36; and a genomic DNA comprising a nucleotide sequence having a sequence identity of 90% or more to any of SEQ ID NOs: 14, 18, 19, and 32 to 36.

Additional examples include: a phage comprising, in the genomic DNA, a gene encoding the amino acid sequence of SEQ ID NO: 15 or 42 as the amino acid sequence of the tail fiber protein; and a phage comprising, in the genomic DNA, a gene encoding the amino acid sequence having addition, deletion, and/or substitution of one or a plurality of amino acids in the amino acid sequence of SEQ ID NO: 15 or 42, or an amino acid sequence having a sequence identity of 90% or more to SEQ ID NO: 15 or 42. Specific examples of such a gene encoding an amino acid sequence include a gene consisting of: the nucleotide sequence of SEQ ID NO: 16 or 43; the nucleotide sequence having addition, deletion, and/or substitution of one or a plurality of nucleotides in the nucleotide sequence of SEQ ID NO: 16 or 43; or a nucleotide sequence having a sequence identity of 90% or more to SEQ ID NO: 16 or 43. Furthermore, specific examples of the genomic DNA of a phage comprising such a gene include: a genomic DNA consisting of the nucleotide sequence of SEQ ID NO: 17 or 44; and a genomic DNA consisting of: the nucleotide sequence having addition, deletion, and/or substitution of one or a plurality of nucleotides in the nucleotide sequence of SEQ ID NO: 17 or 44; a nucleotide sequence having a sequence identity of 90% or more to SEQ ID NO: 17 or 44; the nucleotide sequence having addition, deletion, and/or substitution of one or a plurality of nucleotides in the nucleotide sequence other than the nucleotide sequence encoding the amino acid sequence of SEQ ID NO: 15 or 42 within the nucleotide sequence of SEQ ID NO: 17 or 44; or a nucleotide sequence having a sequence identity of 80% or more to the nucleotide sequence other than the nucleotide sequence encoding the amino acid sequence of SEQ ID NO: 15 or 42 within the nucleotide sequence of SEQ ID NO: 17 or 44.

Additional examples include: a phage comprising, in the genomic DNA, a gene encoding the amino acid sequence of SEQ ID NO: 21; and a phage comprising, in the genomic DNA, a gene encoding the amino acid sequence having addition, deletion, and/or substitution of one or a plurality of amino acids in the amino acid sequence of SEQ ID NO: 21, or an amino acid sequence having a sequence identity of 90% or more to SEQ ID NO: 21. Specific examples of such a gene encoding an amino acid sequence include a gene consisting of: the nucleotide sequence of SEQ ID NO: 22; the nucleotide sequence having addition, deletion, and/or substitution of one or a plurality of nucleotides in the nucleotide sequence of SEQ ID NO: 22; or a nucleotide sequence having a sequence identity of 90% or more to SEQ ID NO: 22. Furthermore, specific examples of the genomic DNA of a phage comprising such a gene include: a genomic DNA consisting of the nucleotide sequence of SEQ ID NO: 23; and a genomic DNA consisting of: the nucleotide sequence having addition, deletion, and/or substitution of one or a plurality of nucleotides in the nucleotide sequence of SEQ ID NO: 23; a nucleotide sequence having a sequence identity of 90% or more to SEQ ID NO: 23; the nucleotide sequence having addition, deletion, and/or substitution of one or a plurality of nucleotides in the nucleotide sequence other than the nucleotide sequence encoding the amino acid sequence of SEQ ID NO: 21 within the nucleotide sequence of SEQ ID NO: 23; or a nucleotide sequence having a sequence identity of 80% or more to the nucleotide sequence other than the nucleotide sequence encoding the amino acid sequence of SEQ ID NO: 21 within the nucleotide sequence of SEQ ID NO: 23.

Further examples include: a phage comprising, in the genomic DNA, a TTPA (Tail-Tubular protein A) gene consisting of the amino acid sequence of SEQ ID NO: 24, 49, 60, or 37 and a TTPB (Tail-Tubular protein B) gene consisting of the amino acid sequence of SEQ ID NO: 25 (correctly SEQ ID NO: 57), 50, 61, 38, 54, or 59; and a phage comprising, in the genomic DNA, a TTPA gene encoding the amino acid sequence having addition, deletion, and/or substitution of one or a plurality of amino acids in the amino acid sequence of SEQ ID NO: 24, 49, 37, 25 (correctly SEQ ID NO: 57), 50, 38, 54, or 59, or an amino acid sequence having a sequence identity of 90% or more to SEQ ID NO: 24, 49, 37, 25 (correctly SEQ ID NO: 57), 50, 38, 54, or 59. Specific examples of such a gene encoding an amino acid sequence include a gene consisting of: the nucleotide sequence of SEQ ID NO: 26, 39, 27 (correctly SEQ ID NO: 58), 40, or 55; the nucleotide sequence having addition, deletion, and/or substitution of one or a plurality of nucleotides in the nucleotide sequence of 26, 39, 27 (correctly SEQ ID NO: 58), 40, or 55; or a nucleotide sequence having a sequence identity of 90% or more to SEQ ID NO: 26, 39, 27 (correctly SEQ ID NO: 58), 40, or 55. Furthermore, specific examples of the genomic DNA of a phage comprising such a gene include: a genomic DNA consisting of the nucleotide sequence of SEQ ID NO: 28, 29, 41, or 56; and a genomic DNA consisting of: the nucleotide sequence having addition, deletion, and/or substitution of one or a plurality of nucleotides in the nucleotide sequence of SEQ ID NO: 28, 29, 41, or 56; a nucleotide sequence having a sequence identity of 90% or more to SEQ ID NO: 28, 29, 41, or 56; the nucleotide sequence having addition, deletion, and/or substitution of one or a plurality of nucleotides in the nucleotide sequence other than the nucleotide sequence encoding the amino acid sequence of SEQ ID NO: 24, 37, 25 (correctly SEQ ID NO: 57), 50, 38, 54, or 59 within the nucleotide sequence of SEQ ID NO: 28, 29, 41, or 45; or a the nucleotide sequence having a sequence identity of 80% or more to the nucleotide sequence other than the nucleotide sequence encoding the amino acid sequence of SEQ ID NO: 24, 37, 25 (correctly SEQ ID NO: 57), 50, 38, 54, or 59 within the nucleotide sequence of SEQ ID NO: 28, 29, 41, or 56.

The sequence identity herein is not particularly limited. Specifically, the sequence identity may be, for example, 80% or more, 81% or more, 82% or more, 83% or more, 84% or more, 85% or more, 86% or more, 87% or more, 88% or more, 89% or more, 90.0% or more, 90.5% or more, 91.0% or more, 91.5% or more, 92.0% or more, 92.5% or more, 93.0% or more, 93.5% or more, 94.0% or more, 94.5% or more, 95.0% or more, 95.5% or more, 96.0% or more, 96.5% or more, 97.0% or more, 97.5% or more, 98.0% or more, 98.5% or more, 99.0% or more, 99.1% or more, 99.2% or more, 99.3% or more, 99.4% or more, 99.5% or more, 99.6% or more, 99.7% or more, 99.8% or more, or 99.9% or more to a sequence as a reference.

### (2) Agriculturally Acceptable Carrier and Medium

An "agriculturally acceptable carrier and/or medium" refers to a substance that facilitates the application of a composition, can maintain the survivability and infectiousness of a phage as an active component, and/or can control the rate of action, and that, when applied outdoor, has no or an extremely small harmful influence on an environment such as the soil and the water quality, and further has no or an extremely low harmfulness to an animal, particularly a human.

### (2-1) Carrier

Specific examples of agriculturally acceptable carriers include surfactants, protective agents, and excipients. Additionally, if desired, a wetting agent, emulsifying agent, pH buffering agent, and the like may be utilized in small amounts. The carrier may be blended in advance, or may be blended immediately before application.

The surfactant has an effect on enhancing the physicochemical properties of the composition with respect to parts of a plant, the properties including hygroexpansivity (wetting capability), emulsifying capability, dispersibility, permeating capability, adhesiveness, defoaming capability, and spreading capability. The surfactant may be utilized as a main component of an agricultural adjuvant called a spreading agent. Examples of spreading agents include nonionic surfactants, combinations of a nonionic surfactant and an anionic surfactant, paraffins, and polyoxyethylene resin acid esters. More specific examples include polyoxyethylenealkyl ether compounds, polyoxyethylene fatty acid ester compounds, lignin sulfonic acid salt compounds, naphthylmethanesulfonic acid salt compounds, alkylsulfosuccinic acid salt compounds, and tetraalkylammonium salt compounds.

A protective agent for a phage is expected to have an effect, such as decreasing damage due to ultraviolet rays. Examples include skim milks, caseins, and gelatins.

Examples of excipients include glucoses, lactoses, sucroses, gelatins, starches, malts, and flours.

### (2-2) Solvent

Specific examples of agriculturally acceptable solvents include water (including an aqueous solution), buffers, and liquid culture media. The solvent is, preferably, an aseptic liquid.

### (3) Another Active Component

A composition of the present invention may comprise, in addition to a bacteriolytic agent according to the first aspect, one or more other active components having the same and/or different pharmacologic actions to the extent that the component(s) does/do not affect the bacteriolytic ability of the phage constituting the bacteriolytic agent.

The (an)other active component(s) is/are not limited to any kind. For example, the phage may have bacteriolytic ability against the same and/or different bacteria. Examples of the phage having bacteriolytic ability against the same bacteria include another phage that specifically recognizes and is attached to bacteria of the *Xanthomonas* genus in the same manner as a phage constituting a bacteriolytic agent according to the first aspect.

Other examples include, as another active component, insecticides, herbicides, fertilizers (for example, urea, ammonium nitrate, and perphosphate), and, if desired, known chemical pesticides, antibiotics, and biological agricultural chemicals.

### 2-2-2. Dosage Form

The dosage form of a composition of the present invention, when the composition is used for plant disease control, may be any dosage form, as long as it is possible that a site of infection of target bacteria on a target plant, the fixability to a target plant, and/or the easiness of infection of a phage as an active component against target bacteria is maintained. For example, the composition for plant disease control may be suspended in a suitable solution to be formed into a liquid agent or a dispersible (water-dispersible) agent, or may be mixed with a carrier and solidified to be formed into a solid-state powder, granule, or gel. For example, when the site of infection of target bacteria on a target plant is a leaf, flower, fruit, stem, branch, or trunk in the aerial part, a liquid, dispersible agent, or gel, which widely spreads in such a site of infection, and has high fixability, is suitable, without limitation. On the other hand, when the site of infection of target bacteria is a root or an underground stem in the underground part, a powder or granule, that is released in a sustained manner in soil, and can sustainably achieve an effect at the site of infection, is suitable, without limitation.

### 2-3. Method for Application

When a composition of the present invention is used as a composition for plant disease control, a method for applying the composition may be a method known in the art, and is not particularly limited, as long as it is possible to apply the composition for plant disease control to a target plant. A phage as an active component of a composition for plant disease control can intrude through any part of the whole surface of the target plant, such as the stem-and-leaf part and the root part, and thus, may be applied by a suitable method in accordance with the purpose. For example, when the site of application is in an aerial part such as a stem-and-leaf part, the composition for plant disease control may be applied so as to come into direct contact with the site of application. The direct contact involves, for example, painting, spraying, scattering, or immersing the composition for plant disease control for the site of application. The application is particularly preferably performed to the site, where it is infected or potentially infected by target bacteria, of a target plant. Additionally, the application may be indirect through soil when the site of application is in an underground part, such as a root, or by addition to a culture medium when the site is in the medium. "Soil" as used herein is not particularly limited, as long as it is possible to grow a target plant. Usually, a planting soil comprising a suitable nutrient, and having a suitable pH value is utilized. Where the soil is from is not limited. Additionally, a "culture medium" refers to a planting medium artificially prepared for a target plant. The medium may be a solid medium, such as an agar medium, or may be a liquid medium. The culture medium is, for example, an isolating bed, a root zone restriction pot, or a nursery. The composition of the culture medium may be a medium composition known in the art. The composition may be suitably selected according to the kind of plant, or the like.

### 2-4. Target Plant

A target plant for a composition for plant disease control according to the present invention is not particularly limited to any kind, as long as it is possible to develop a plant disease caused by the target bacteria of the present invention. The target plant may be either an angiosperm or a gymnosperm. Furthermore, the plant may be an herbaceous plant or a woody plant. Suitable specific examples of the target plant include agriculturally important plants, for example: crop plants such as cereal crops, vegetables, and fruits; and flowers and ornamental plants. Specific examples include monocotyledons, such as Poaceae plants (for example, rice, wheat, barley, corn, sugarcane, *Sorghum,* kaoliangs, and turfgrasses), Musaceae plants (for example, bananas), Amaryllidaceae plants (for example, *Allium,* onions, garlic, and garlic chives), and Liliaceae plants (for example, lilies and tulips). Additional examples include dicotyledons, such as Brassicaceae plants (for example, cabbages, radishes, Chinese cabbages, and rapeseeds), Asteraceae plants (for example, lettuces, burdocks, and *Chrysanthemum*)*,* Fabaceae plants (for example, soybeans, peanuts, garden peas, phaseoli, lentils, chickpeas, fava beans, and licorice), Solanaceae plants (for example, tomatoes, eggplants, potatoes, tobaccos, bell peppers, red peppers, and petunia), Rosaceae plants (for example, strawberries, apples, pears, peaches, *Eriobotrya,* almonds, plums, roses, Japanese apricots, and cherries), Cucurbitaceae plants (for example, cucumbers, gourd, pumpkins, melons, and watermelons), Anacardiaceae plants (for example, mangoes, pistachios, and cashew nuts), Lauraceae plants (for example, avocadoes) Rutaceae plants (for example, mandarin oranges, grapefruits, lemons, and *Citrus junos*), Convolvulaceae plants (for example, sweet potatoes), Theaceae plants (for example, *Camellia sinensis*)*,* and Vitaceae plants (for example, grapes).

### 2-5. Target Plant Disease

A target plant disease to which a composition for plant disease control according to the present invention is to be applied is any plant disease that is caused by the target bacteria of the present invention. The disease is, preferably, a plant disease caused by bacteria of the *Xanthomonas* genus. Examples include: bacterial spot observed in peaches and the like; bacterial blight observed in walnuts and the like; bacterial pustule observed in soybeans and the like; angular leaf spot observed in strawberries and the like; bacterial blight observed in tomatoes, bell peppers, lettuces, and the like; black rot observed in cabbages, Chinese cabbages, broccoli, and the like; bacterial canker observed in oranges, grapefruit, and the like; angular leaf spot observed in cotton and the like; and leaf blight observed in rice and the like.

### 3. Method for Controlling Plant Disease

### 3-1. Overview

A third aspect of the present invention is a method for controlling plant disease. A method for controlling plant disease according to the present invention is characterized by applying a composition for plant disease control according to the second aspect to a target plant to control a plant disease of the target plant.

A method for controlling plant disease according to the present invention can control a plant disease caused by bacteria, particularly bacteria of the *Xanthomonas* genus, in a target plant.

### 3-2. Method

A method for controlling plant disease according to the present invention comprises a contacting step as an essential step.

The "contacting step" is a step of contacting a composition for plant disease control according to the second aspect to the target plant. This step is basically in accordance with "2-3. Method for Application" in the second aspect regarding the composition for plant disease control.

In the present aspect, "contact" refers to contact between a composition for plant disease control and a target plant. More specifically, the contact is between the following: a bacteriolytic agent, i.e., a phage, according to the first aspect as an active component of a composition for plant disease control; and part of a target plant, preferably a site that is infected or to be potentially infected by target bacteria. This step is intended to infect a phage as an active component with target bacteria, whereby the target bacteria are lysed. As a result, an effect on controlling the plant disease caused by target bacteria can be achieved.

The contact may be any of direct contact and indirect contact. The direct contact in the present aspect means that the composition for plant disease control is brought directly into contact with a predetermined site of the target plant. Specifically, for example, this means that a composition for plant disease control, in liquid form or gel form, is painted, sprayed, scattered, or immersed for part of a target plant. In this case, the part of a plant as a target of contact is mainly a site such as a leaf, flower, fruit, stem, branch, and/or trunk. On the other hand, the indirect contact in the present aspect means that the composition for plant disease control is brought into contact with a predetermined site of the target plant via an intermedium. For example, this means that a composition for plant disease control, in particulate form, is applied into the soil around the root of a target plant. The phage, as an active component, is transported via water or the like in soil, and absorbed by the root.

### 3-3. Effects

A phage as an active component of a composition obtained by the present invention can kill target bacteria efficiently, and hence, is useful to prevent and inhibit a disease caused by target bacteria. Additionally, the specific bacteriolytic ability serves as a basis for detecting and identifying target bacteria, thus enabling a disease to be diagnosed. An agent that has been used customarily for bacteria of the *Xanthomonas* genus includes a copper agent or an antibiotic, but such an agent may cause drug poisoning or a disruption of the balance of a bacterial lawn. For example, some strains of *Pseudomonas fluorescens* have proved to have a plant-growth-promoting effect (Haas D., Defago G., Nature Reviews in Microbiology, 2005, 3 (4), 307-19), and a copper agent and an antibiotic have a high possibility of also killing such bacteria having a symbiotic relationship with a plant. On the other hand, since a phage is an organism-derived substance, there is no report of the manifestation of drug poisoning, and since a phage has high specificity, its influence on the balance of a bacterial lawn is extremely limited.

### 4. Method for Identifying Bacteria of the Xanthomonas Genus

### 4-1. Overview

A fourth aspect of the present invention is a method for identifying bacteria of the *Xanthomonas* genus. A method for identification according to the present invention is characterized by utilizing the host specificity of a phage constituting the bacteriolytic agent according to the first aspect to identify bacteria of the *Xanthomonas* genus.

The present invention can determine and identify whether unidentified plant pathogenic bacteria causative of a plant disease are bacteria of the *Xanthomonas* genus.

### 4-2. Method

A method for identification according to the present invention comprises a culturing step, a mixing step, a mixture culturing step, and a determining step as essential steps, and additionally, an isolating step as an optional step. Each step is described below.

### (1) Isolating Step

The "isolating step" is a step of isolating subject bacteria from plant tissue affected by a plant disease. This step is an optional step, and may be performed if desired.

The "subject bacteria" refers to plant pathogenic bacteria, the species of which has not been revealed, and that are used for a method for identifying bacteria of the *Xanthomonas* genus in the fourth aspect or a method for detecting bacteria of the *Xanthomonas* genus in the below-described fifth aspect of the present invention.

The plant tissue may be any site of a plant that has developed a plant disease, and is preferably a site at which the symptom of the plant disease is recognized remarkably. For example, from a peach that has developed a shot hole disease of *Prunus spp.,* a leaf with a disease observed therein may be used.

To isolate subject bacteria from plant tissue taken, a specimen with a disease observed therein may be immersed in a solvent such as water to extract the subject bacteria, and the specimen may be fragmented and crushed upon the extraction. Subsequently, the extraction liquid may be streaked on an agar culture medium, and a single colony may be picked.

### (2) Culturing Step

The "culturing step" is a step of culturing the subject bacteria isolated, and obtaining a culture preparation. Subject bacteria may be cultured by a method known in the art.

The "culture preparation" is obtained by culturing subject bacteria, and may be either liquid or solid.

In this step, subject bacteria are unidentified, and thus, the culture medium to be used in this step is desirably a medium that can culture a wide range of plant pathogenic bacteria. At least a culture medium that can culture bacteria of the *Xanthomonas* genus as target bacteria to be identified in the present invention is used. Such a culture medium may be, for example, a medium comprising one or more components selected from: protein enzymatic decomposition products such as peptone and tryptone; organism-derived extracts such as potato dextrose and yeast extracts; amino acids or salts thereof, such as glutamic acid; saccharides such as glucoses and sucroses; and inorganic salts such as sodium chloride, magnesium chloride, and potassium dihydrogen phosphate. Specific examples of the culture medium and the composition include LB media (tryptone, yeast extract, and sodium chloride), YPG media (yeast extract, peptone, and glucose), PD media (potato dextrose), peptone-supplemented Suwa's media (sucrose, glutamic acid, and peptone), and the like.

The subject bacteria isolated are seeded in the culture medium, and cultured under suitable culture conditions. A culture with stirring under culture conditions, for example, at 20 to 40°C, 20 to 30°C, 22 to 28°C, or 24 to 26°C, yields a culture preparation. The culture time is not limited, and may be any period of time, for example, the culture may be performed until the turbidity at 600 nm reaches approximately 1.0. The present step yields a culture solution of subject bacteria. Additionally, the culture may be a multi-step culture that is double-step or more. For example, a culture solution obtained by a culture in a solution medium can be supplemented with a soft agar containing liquid medium, poured into a solid medium such as an agar medium, solidified, and subjected to further culture.

### (3) Mixing Step

The "mixing step" is a step of mixing the culture preparation obtained in the culturing step and the bacteriolytic agent according to the first aspect to obtain a mixture.

The "mixture" is a mixture of a culture preparation and a bacteriolytic agent, and may be either liquid or solid.

The mixing method is not particularly limited, as long as it is capable of mixing the culture preparation and the bacteriolytic agent. The bacteriolytic agent according to the first aspect may be in a solid state, or may be suspended in water or a liquid culture medium, and added in a liquid state.

When the culture preparation and the bacteriolytic agent are both liquid, the capacity ratio of the culture preparation to the bacteriolytic agent may be 1:9, 2:8, 3:7, 4:6, 5:5, 6:4, 7:3, 8:2, or 9: 1. After the addition, the culture preparation and the bacteriolytic agent may be mixed sufficiently by stirring or the like. On the other hand, when a soft agar containing liquid medium is superposed as described above, the culture preparation is solid. In this case, the bacteriolytic agent may be dropped on a solid culture preparation, such as on the surface of a gel, whereby both are mixed on the solid medium to obtain a mixture.

### (4) Mixture Culturing Step

The "mixture culturing step" is a step of culturing the mixture under predetermined conditions.

Here, in the culture of the mixture, the mixture may also be supplemented with a soft agar containing liquid medium, poured into a solid medium such as an agar medium, solidified, and subjected to further culture.

The basic procedure in this step are in accordance with the culturing step above. In this step, the culture is preferably based on, but not limited to, what is called a plaque assay method so that the bacteriolysis of the subject bacteria by a phage constituting the bacteriolytic agent can be more easily verified in the below-described determining step. For example, after part of the mixture solution is mixed with a soft agar medium having the same composition, and before the soft agar medium is solidified, the resulting mixture may be poured onto an agar medium having the same composition, and spread on the whole culture medium. Then, the mixture may be cultured under the same conditions as in the culturing step.

### (5) Determining Step

The "determining step" is a step of determining that the subject bacteria is bacteria of the *Xanthomonas* genus when the subject bacteria is lysed after the culturing step.

Without limitation, for example, when a plaque assay method is used, whether bacteriolysis occurs may be determined according to whether a plaque has been formed. A plaque exists on the soft agar medium, that is spread and solidified on the agar culture medium, after the above-described mixture culturing step, this result indicates that the subject bacteria has been lysed by an infection of a phage constituting a bacteriolytic agent of the present invention. Accordingly, the subject bacteria in this case can be determined to be bacteria of the *Xanthomonas* genus. On the other hand, when the subject bacteria proliferates on the whole agar culture medium, but no plaque at all exists, the subject bacteria can be determined not to be bacteria of the *Xanthomonas* genus.

To render the determination more accurate, a negative control and/or a positive control may be prepared simultaneously and used to verify that no plaque is generated in the negative control, and that a plaque is observed in the positive control, wherein the negative control is mixed with a culture medium comprising no bacteriolytic agent in the mixture culturing step, and wherein, for the positive control, bacteria of the *Xanthomonas* genus identified are used from the culturing step rather than subject bacteria.

### 4-3. Effects

A method for identifying bacteria of the *Xanthomonas* genus according to the present invention can identify whether a plant disease is caused by bacteria of the *Xanthomonas* genus.

Additionally, a method for identifying bacteria of the *Xanthomonas* genus according to the present invention can detect whether bacteria of the *Xanthomonas* genus exist in a lesion site of a plant that has developed a plant disease expected to be caused by the bacteria of the *Xanthomonas* genus.

### Examples

Examples herein correspond to Examples in Japanese Patent Application No. 2022-156882, as follows: Example 1 to Example 1, Example 2 to Example 2, Example 3 to Example 3, Example 4 to Example 4, Example 5 to Example 5, Example 6 to Example 6, Example 7 to Example 7, Example 8 to Example 8, Example 9 to Example 9, and Example 10 to Example 10, respectively. Additionally, Tables 1 to 13 herein are completely the same as Tables 1 to 13 in Japanese Patent Application No. 2022-156882. Additionally, Figures 1 to 11 herein are completely the same as Figures 1 to 11 in Japanese Patent Application No. 2022-156882. Additionally, SEQ ID NOs: 1 to 44 herein are completely the same as SEQ ID NOs: 1 to 44 in Japanese Patent Application No. 2022-156882.

### <Example 1: Isolation of Novel Bacteriophage, and Bacteriolytic Ability Thereof (1)>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In this Example, plant pathogenic bacteria as targets were all obtained from the National Agriculture and Food Research Organization (NARO). Each of the bacteria used in this Example are listed in Table 2, together with the deposition numbering by the National Agriculture and Food Research Organization.

**[Table 2]**

| ID | Species | Source of Isolation | Place of Harvest |
|---|---|---|---|
| MAFF No. 311019 | *Xanthomonas oryzae pv. oryzae* | Rice | Gifu |
| MAFF No. 211965 | *Xanthomonas oryzae pv. oryzae* | Rice | Okinawa |
| MAFF No. 211964 | *Xanthomonas oryzae pv. oryzae* | Rice | Miyazaki |
| MAFF No. 211191 | *Xanthomonas oryzae pv. oryzae* | Rice | Miyagi |
| MAFF No. 211971 | *Xanthomonas arboricola pv. pruni* | Peach | Yamanashi |
| MAFF No. 311351 | *Xanthomonas arboricola pv. pruni* | Peach | Fukushima |
| MAFF No. 301256 | *Xanthomonas campestris pv. vesicatoria* | Tomato | Tokushima |
| MAFF No. 301294 | *Xanthomonas campestris pv. vesicatoria* | Pepper | Tokushima |
| MAFF No. 301352 | *Xanthomonas campestris pv. vitians* | Lettuce | Wakayama |
| MAFF No. 106765 | *Xanthomonas campestris pv. campestris* | Broccoli | Ibaraki |
| MAFF No. 301078 | *Xanthomonas citri subsp. citri* | Orange | Wakayama |
| MAFF No. 673005 | *Xanthomonas cucurbitae* | Pumpkin | Hokkaido |
| NCIMB-ID:10460 | *Pseudomonas fluorescens* | Egg | U.S.A. |

As a control, a *Pseudomonas fluorescens* bacterial strain (NCIMB-ID: 10460) that is reported to have a plant growth promoting action and the like, and is beneficial to the environment was obtained from NCIMB, a laboratory in The United Kingdom National Culture Collection, UKNCC.

For culturing the *Xanthomonas oryzae pv. oryzae,* the liquid medium (SW + P Broth) prepared by dissolving 0.5 g of monosodium L-glutamate, 1.0 g of MgCl₂.6H₂O, 0.1 g of KH₂PO₄, 5 g of sucrose, and 5 g of peptone in 1 L of H₂O and autoclaving, was used. For culturing the other bacteria of the *Xanthomonas* genus and bacteria of *Pseudomonas fluorescens,* the liquid medium (YPG Broth) prepared by dissolving 1 g of peptone, 1 g of a yeast extract, and 2 g of glucose in 1 L of H₂O and autoclaving, was used. Additionally, as an agar culture medium, agar culture medium (referred to as "SW+P Agar" when SW+P Broth was used, or as "YPG Agar" when YPG Broth was used) prepared by adding agar at 15 g per L to the above-described Broth (SW+P Broth or YPG Broth) and autoclaving, was used. Furthermore, to prepare a soft agar medium (Top Agar) to be superposed on the upper layer of the agar culture medium, agarose was added at 5 g per L to the above-described Broth, and autoclaved. The resulting Top Agar was stored at approximately 50°C, and utilized, when necessary.

Each of the above-described bacterial strains sent in a dry powder state was suspended in 0.1 mL of Broth, and then subjected to a streak culture on Agar (SW+P Agar or YPG Agar) at 25°C, and a single colony was isolated. The isolated colony was inoculated to Broth at 25°C, and subjected to a shaking culture to produce a preculture solution. In main culture, the preculture solution was inoculated to Broth, and cultured at 25°C for 10 to 30 hours until the turbidity (Optical Density at 600 nm) reached approximately 1.0. The culture solution after the culture was directly used as bacteria suspension as it is.

### (2) Isolation and Purification of 1st Phage

A novel phage was isolated from natural dirty water or soil obtained in Japan. The method for isolating a phage was based on a conventional plaque assay method. First, dirty water from a pond, lake, or the like, or dirty water in which soil is suspended in water was filtrated through a 0.45 µm filter to prepare a phage-containing solution. Subsequently, the bacteria suspension and the phage-containing solution were mixed in equal amounts, and left at room temperature for approximately 10 minutes. Next, 0.2 mL of the bacteria/phage mixture solution was added to 3 mL of Top Agar, quickly mixed with a vortex mixer, and then poured on Agar. After the Top Agar was solidified, static culture was performed at 25°C for approximately 12 hours. On the bacterial lawn formed by the culture, a bacteriolytic plaque was formed. Then, a chip with its end cut was used to suck gel from the plaque portion, and a phage having bacteriolytic ability against bacteria of the *Xanthomonas* genus was isolated. Then, the above-described procedure was repeated to purify the phage, using a phage-containing solution comprising a high concentration of the phage isolated was used instead of dirty water.

A total of three kinds of new phages were isolated from natural dirty water and soil, using a method for detecting a bacteriolytic plaque formed on a soft agar medium on which any of the above-described bacterial strains was amplified. The three kinds of novel phages isolated in Example 1 are referred to as "1st phage".

The isolated phage was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to further purify the phage. The composition of the SM Buffer is shown in Table 3.

**[Table 3]**

| SM Buffer | Final | Add to 1L |
|---|---|---|
| NaCl | 0.1 M | 5.8 g |
| Mg₂SO₄·7H₂O | 10 mM | 1 g |
| 1M Tris-HCl pH 8.0 | 50 mM | 50 mL |
| Gelatin | 0.1% | 0.1 g |

### (3) Amplification and Refinement of 1st Phage

The 1st phage isolated and purified was amplified and refined by a plate lysate (PL) method that is an amplifying method using a plaque assay method. In order for many plaques to be formed on Agar, a bacteria/phage mixture solution was prepared, mixed with Top Agar, then spread on YPG Agar, and cultured. Then, 3 mL of SM Buffer was added to the Top Agar having plaques formed thereon, and shaken at 25°C for approximately 30 minutes, and the supernatant was passed through a 0.2 µm filter to collect a collected solution containing a phage.

To refine the 1st phage, 1 g of PEG 6000 (at a final concentration of 10%) and 0.4 g of NaCl (at a final concentration of 4%) were added to and dissolved in 10 mL of the collected solution, and the resulting solution was rotated using a rotator at 4°C overnight. Then, the supernatant was removed by centrifugation under 15,000 × g at 4°C for 60 minutes. The pellets collected were suspended in 0.5 mL of SM Buffer again. Subsequently, 0.5 mL of chloroform was added, and the resulting mixture was stirred vigorously, and left on ice for 6 hours. After centrifugation under 8,000 × g at 4°C for 10 minutes, the upper layer was collected carefully to obtain a refined solution of phage. The concentration of the refined solution of phage is commonly expressed as a titer [PFU/mL] based on the number of plaques (Plaque Forming Unit, PFU) in accordance with a plaque assay method, and serves as one index indicative of bacteriolytic ability. The titer of the refined solution of the 1st phage prepared was determined by a plaque assay method using a solution suitably diluted, and was confirmed to be 10⁸ PFU/mL or more.

### (4) Evaluation of Host Range of 1st Phage

The host range of the 1st phage was evaluated by a spot test method. Only a bacteria suspension in an amount of 0.1 mL was added to and mixed with 3 mL of Top Agar, then poured onto Agar, spread on the whole plate, and solidified. Bacteria suspensions were the bacteria suspensions prepared in (1) above from the bacteria of the *Xanthomonas* genus shown in Table 1 as well as a bacteria suspension prepared as a control from *Pseudomonas fluorescens.* Then, approximately 5 µL of the refined solution of phage was dropped, and subjected to a static culture at 25°C for approximately 12 hours. When the solution became clear in circular shape (approximately 1 cm in diameter) at the site of dropping on the plate having the bacterial lawn formed thereon, it was determined that the phage dropped had bacteriolytic ability against the bacterial strain.

One example of the results is shown in Figure 1. When the 1st phage exhibited bacteriolytic ability, only the site where the refined solution of phage was dropped became clear in the bacterial lawn formed on the plate. The three kinds of 1st phages obtained in the present invention exhibited bacteriolytic ability against the bacterial strains belonging to all five species of bacteria: *Xanthomonas arboricola, Xanthomonas campestris, Xanthomonas citri, Xanthomonas oryzae,* and *Xanthomonas cucurbitae,* which are shown in Table 2. On the other hand, it has also been verified that the phages do not exhibit bacteriolytic ability against *Pseudomonas fluorescens.* There is an example of a report that a phage exhibits the ability against two or more species of bacteria of the *Xanthomonas* genus, but such a pattern as described above has not been reported (Nakayinga R. et al., BMC Microbiology, 2021, 21: 291).

Figure 12 shows one example of a plate in a plaque assay performed on the 1st phage having the nucleotide sequence of SEQ ID NO: 8, using *Xanthomonas oryzae pv. oryzae* with the ID of MAFF No.311019 as bacteria species. As a result of bacteriolysis, many plaques were formed, thus revealing that the 1st phage exhibited bacteriolytic ability against this bacteria isolated from a rice plant. These results suggest the possibility that the 1st phage can be applied to various plant diseases. For example, this suggests the usefulness for control of shot hole disease of *Prunus spp.,* rice leaf blight, orange bacterial canker, black rot disease of broccoli, and the like, which are caused by bacteria of the *Xanthomonas* genus, and is expected to be very valuable in industrial uses.

### (5) Genomic Analysis of 1st Phage

The genomic DNA sequence of the 1st phage was determined and analyzed.

### (i) Preparation and Sequencing of Genomic DNA of 1st Phage

The genome of the 1st phage was extracted using a TURBO DNA-free^{™} kit (Thermo Fisher Scientific Inc.). Host bacteria-derived genomic DNAs, which become contaminants, were removed by a treatment according to the manual attached to the kit. Then, using NucleoSpin^{(R)} Virus (Machery-Nagel & Co. KG), the outer-shell molecules of the phage were decomposed by a Proteinase K treatment according to the attached manual. Via refining the genomic DNA using a silica spin column, a genomic DNA solution of the 1st phage was prepared. Then, the concentration of the genomic DNA was measured using a Qubit dsDNA HS Assay kit (Thermo Fisher Scientific Inc.), and 50 µL of the genomic DNA solution was prepared at a final concentration of 0.2 ng/µL. Subsequently, by a treatment with Nextera XT DNA Library Prep (Illumina, Inc.) and according to the attached manual, the genome of the 1st phage was fragmented, and an adapter sequence was added by PCR. Next, Agilent High Sensitivity DNA Kit (Agilent Technologies, Inc.) was used for electrophoresis with Bioanalyzer (Agilent Technologies, Inc.) to measure the average bp size of the sample, and determine the concentration of the DNA fragments. Lastly, using a Miseq Reagent kit (Illumina, Inc.), a sample for measurement was prepared by a treatment according to the attached manual, and a measurement was made using a next-generation sequencer Miseq (Illumina, Inc.). Utilizing a CLC genomics workbench (Qiagen N.V.), the data obtained was pretreated (for example, trimmed), and then subjected to a de novo assembly to obtain the contig sequence corresponding to the genomic sequence of the phage.

### (ii) Bioinformatics Analysis based on Genomic Sequence Information

The genomic nucleotide sequences (SEQ ID NOs: 8 to 10) of the 1st phages were analyzed by BLAST with GENETYX-NGS packaged in GENETYX, consequently revealing that the nucleotide sequences of SEQ ID NOs: 8 to 10 had a high sequence identity to each other. For example, the sequence identity (the average nucleotide identity (ANI)) of the nucleotide sequence of SEQ ID NO: 8 to the nucleotide sequence of SEQ ID NO: 9 was 98% or more over the range of 96% or more of the full length, and the sequence identity of the nucleotide sequence of SEQ ID NO: 8 to the nucleotide sequence of SEQ ID NO: 10 was 98% or more over the range of 95% or more of the full length.

Furthermore, the genomic sequence information on the above-described three kinds of phages was searched for tail fiber genes. The RAST server (https://rast.nmpdr.org/) and PHASTER (https://phaster.ca/) were searched for tail fiber genes, resulting in identifying, from the genomic sequences of the respective phages, the nucleotide sequences of SEQ ID NOs: 5 to 7 were estimated to be tail fiber genes. The nucleotide sequences of SEQ ID NOs: 5 to 7 were analyzed with GENETYX, consequently revealing that the nucleotide sequences of SEQ ID NOs: 5 to 7 had a high sequence identity to each other, for example, the sequence identity of the nucleotide sequence of SEQ ID NO: 5 to the nucleotide sequence of each of SEQ ID NOs: 6 and 7 was 96% or more, and a sequence identity between the nucleotide sequences of SEQ ID NOs: 6 and 7 was 98% or more. Additionally, the amino acid sequences (SEQ ID NOs: 2 to 4) encoded by the nucleotide sequences of SEQ ID NOs: 5 to 7 were analyzed with GENETYX, consequently revealing that the amino acid sequences of SEQ ID NOs: 2 to 4 had a high sequence identity to each other, and a sequence identity between the amino acid sequences of SEQ ID NOs: 2 to 4 was 98% or more.

On the basis of the genomic nucleotide sequences (SEQ ID NOs: 8 to 10) of the above-described phages, and using the NCBI-provided BLAST server (https://blast.ncbi.nlm.nih.gov/Blast.cgi), similar DNA sequences were searched for, and the sequence identities were verified. As a result of the search, the genomic sequence of a phage Mija described in the U.S. Patent Application Publication No. 2016/0309723 (SEQ ID NO: 29 in the publication) had the highest analogous score, for example, a sequence identity thereof to the genomic nucleotide sequence of SEQ ID NO: 8 was 86.5% over the range of 93% of the full length.

The bacteriolytic ability of the phage Mija was verified to *Xylella fastidiosa* and the like, and the host range of the phage Mija is different from the host range of the phage of the present invention. Generally, it is considered that the characteristics related to the host specificity and host range of a phage depend largely on the role of a tail fiber protein (Nobrega F.L. et al., Nat. Rev. Microbiol., 2018, 16: 760-773), and the present inventors consider that the above-described difference in the host range is caused by a difference in the tail fiber gene.

Accordingly, the nucleotide sequences (SEQ ID NOs: 5 to 7) of the tail fiber genes identified were used as a query to search the genomic sequence of the phage Mija for a similar DNA sequence. Consequently, only region found in the genomic sequence of the phage Mija was a region having a sequence identity of 83% to the nucleotide sequence of SEQ ID NO: 5 over the range as small as 16% of the full length, and a DNA sequence homologous to the nucleotide sequences of SEQ ID NOs: 5 to 7 over the full length was not found. This result has revealed that the difference in the host range between the phage Mija and the three kinds of phages obtained in Examples herein is due to the difference in the tail fiber gene.

To examine whether the tail fiber gene of a phage of the present invention is novel, the amino acid sequences of SEQ ID NOs: 2 to 4 were used as a query to search the NCBI-provided BLAST server for a similar amino acid sequence. Consequently, the tail fiber protein (GenBank Accession No.: AMQ65898.1) of *Stenotrophomonas* phage vB_SmaS-DLP_6 had the highest analogous score. However, a sequence identity of the amino acid sequence to each of the amino acid sequences of SEQ ID NOs: 2 to 4 was low, and for example, a sequence identity was only 58.47% to the sequence of SEQ ID NO: 2 over the range as small as 35% of the full length. In this connection, *Stenotrophomonas* phage vB_SmaS-DLP_6 targets the *Stenotrophomonas* genus, i.e., targets bacteria different in the genus from the phage of the present invention.

The above-described results have revealed that a phage in the present invention has a conventionally unreported novel host range in the *Xanthomonas* genus, and that the characteristics of the phage rely on the novel tail fiber gene in particular.

### <Example 2: Isolation of Novel Bacteriophage, and Bacteriolytic Ability Thereof (2)>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In this Example, the plant pathogenic bacteria as target bacteria were bacteria of the *Xanthomonas* genus, and all the bacterial strains were obtained from the National Agriculture and Food Research Organization (NARO). Each of the bacteria used in Example 2 is listed in Table 4, together with the deposition numbering (MAFF No.) by the National Agriculture and Food Research Organization.

**[Table 4]**

| ID | Species | Source of Isolation | Place of Harvest |
|---|---|---|---|
| MAFF No. 311019 | *Xanthomonas oryzae* pv. *oryzae* | Rice | Gifu |
| MAFF No. 211965 | *Xanthomonas oryzae* pv. *oryzae* | Rice | Okinawa |
| MAFF No. 211964 | *Xanthomonas oryzae* pv. *oryzae* | Rice | Miyazaki |
| MAFF No. 211191 | *Xanthomonas oryzae* pv. *oryzae* | Rice | Miyagi |
| MAFF No. 211971 | *Xanthomonas arboricola* pv. *pruni* | Peach | Yamanashi |
| MAFF No. 311351 | *Xanthomonas arboricola* pv. *pruni* | Peach | Fukushima |
| MAFF No. 212146 | *Xanthomonas arboricola* pv. *juglandis* | Walnut | Nagano |
| MAFF No. 301256 | *Xanthomonas campestris* pv. *vesicatoria* | Tomato | Tokushima |
| MAFF No. 301294 | *Xanthomonas campestris* pv. *vesicatoria* | Pepper | Tokushima |
| MAFF No. 301352 | *Xanthomonas campestris* pv. *vitians* | Lettuce | Wakayama |
| MAFF No. 301078 | *Xanthomonas citri* subsp. *citri* | Orange | Wakayama |
| MAFF No. 673005 | *Xanthomonas cucurbitae* | Pumpkin | Hokkaido |
| NCIMB-ID:10460 | *Pseudomonas fluorescens* | Egg | U.S.A. |

Additionally, as a control, a *Pseudomonas fluorescens* bacterial strain (NCIMB-ID: 10460), which is of *Pseudomonas* genus, was obtained from NCIMB, a laboratory in The United Kingdom National Culture Collection, UKNCC.

Bacteria of the *Xanthomonas* genus and *Pseudomonas fluorescens* were cultured in accordance with the method described in "(1) Obtainment and Culture of Plant Pathogenic Bacteria" in Example 1.

### (2) Isolation and Purification of 2nd Phage

A novel phage was isolated from natural dirty water or soil obtained in Japan. A method for isolation and a method for purification were in accordance with the methods described in "(2) Isolation and Purification of 1st Phage" in Example 1. Consequently, one kind of new phage was isolated from natural dirty water and soil. This novel phage isolated in Example 2 is referred to as a "2nd phage".

The isolated phage was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to purify the phage.

### (3) Amplification and Refinement of 2nd Phage

The 2nd phage, isolated and purified, was amplified and refined by the plate lysate (PL) method. Specific method was in accordance with the method described in "(3) Amplification and Refinement of 1 st Phage" in Example 1. The titer of the refined solution of the 2nd phage prepared was determined by a plaque assay method using a solution suitably diluted, and was confirmed to be 10⁸ PFU/mL or more.

### (4) Evaluation of Host Range of 2nd Phage

The host range of the 2nd phage was evaluated by the spot test method. The basic operation was in accordance with the method described in "(4) Evaluation of Host Range of 1st Phage" in Example 1.

One example of the results is shown in Figure 2. When the 2nd phage exhibited bacteriolytic ability, only the site where the refined solution of phage was dropped became clear in the bacterial lawn formed on the plate. The 2nd phage obtained in the present invention exhibited bacteriolytic ability against all the bacteria of the *Xanthomonas* genus shown in Table 4. On the other hand, the phage did not exhibit bacteriolytic ability against *Pseudomonas fluorescens.* As with Example 1, a phage that exhibits bacteriolytic ability against each of the diverse bacterial species, such as shown in Table 4, has not been known hitherto.

Figure 13 shows one example of a plate in a plaque assay performed on the 2nd phage, using *Xanthomonas oryzae pv. oryzae* with the ID of MAFF No.311019 as a bacterial species. As a result of bacteriolysis, many plaques were formed, thus revealing that the 2nd phage exhibited bacteriolytic ability against this bacteria isolated from a rice plant. These results suggest that the 2nd phage isolated exhibited bacteriolytic ability against a wide range of bacteria of the *Xanthomonas* genus.

Additionally, these results suggest that the 2nd phage is useful for control of shot hole disease of *Prunus spp.,* rice leaf blight, orange bacterial canker, black rot disease of broccoli, and the like, which are caused by bacteria of the *Xanthomonas* genus.

### (5) Genomic Analysis of 2nd Phage

The genomic DNA sequence of the 2nd phage was determined and analyzed.

### (i) Preparation and Sequencing of Genomic DNA of 2nd Phage

The genome of the 2nd phage was extracted using a TURBO DNA-free^{™} kit (Thermo Fisher Scientific Inc.). The basic operation was in accordance with the method described in "(5) Genomic Analysis of 1st Phage, (i) Preparation and Sequencing of Genomic DNA of 1st Phage" in Example 1.

### (ii) Bioinformatics Analysis based on Genomic Sequence Information

On the basis of the genomic nucleotide sequence (SEQ ID NO: 13) of the phage obtained in (i) above, and using the NCBI-provided BLAST server (the same as the above), similar DNA sequences were searched for, and the sequence identities were verified. As a result of the search, the genomic sequence of a phage (the access code: MT664984.1) that targets bacteria of the *Xanthomonas* genus, the bacteria called Xp12, had the highest analogous score, with a sequence identity of 98.42%. However, the only known bacterial species against which the phage Xp12 exhibits bacteriolytic ability is *Xanthomonas oryzae* (Nakayinga R. et al., BMC Microbiology, 2021, 21: 291).

To clarify the cause of the difference in host specificity between Xp12 and the 2nd phage isolated in this Example, the sequences of the tail fiber proteins of both phages were compared.

First, a tail fiber gene was identified from the genomic sequence of the 2nd phage in this Example. The gene was identified utilizing a RAST server (https://rast.nmpdr.org/) and a PHASTER server (https://phaster.ca/). Consequently, the nucleotide sequence of SEQ ID NO: 12 was identified as the tail fiber gene.

Furthermore, the amino acid sequence (SEQ ID NO: 11) encoded by the gene consisting of the nucleotide sequence of SEQ ID NO: 12 was compared with the amino acid sequence (the access code: QNN97189.1) of the analogous protein of Xp12, revealing that the kind of amino acid was different at position 278 and position 350. Specifically, valine at position 278 in Xp12 was changed to alanine, and serine at position 350 was changed to tryptophan. These are not conservative substitutions, and serine in particular often contributes to the protein-protein interaction, suggesting a high possibility that these substitutions cause a difference in the function.

From the above-described results, it has been estimated that the 2nd phage has a conventionally unreported novel host range in the *Xanthomonas* genus, and that the characteristics of the phage rely on the tail fiber gene.

### <Example 3: Isolation of Novel Bacteriophage, and Bacteriolytic Ability Thereof (3)>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In this Example, the plant pathogenic bacteria were bacteria of the *Xanthomonas* genus, and all the bacterial strains were obtained from the National Agriculture and Food Research Organization (NARO). Each of the bacteria used in this Example is listed in Table 5, together with the deposition numbering (MAFF No.) by the National Agriculture and Food Research Organization.

**[Table 5]**

| ID | Species | Source of Isolation | Place of Harvest |
|---|---|---|---|
| MAFF No. 311282 | *Xanthomonas arboricola* pv. *pruni* | Peach | Fukushima |
| MAFF No. 301426 | *Xanthomonas arboricola* pv. *pruni* | Peach | Fukushima |
| MAFF No. 311351 | *Xanthomonas arboricola* pv. *pruni* | Peach | Fukushima |
| MAFF No. 311586 | *Xanthomonas arboricola* pv. *pruni* | Peach | Nagano |
| MAFF No. 311618 | *Xanthomonas arboricola* pv. *pruni* | Peach | Wakayama |
| NCIMB-ID: 10460 | *Pseudomonas fluorescens* | Egg | U.S.A. |

As a control, a *Pseudomonas fluorescens* bacterial strain (NCIMB-ID: 10460), which is a of *Pseudomonas* genus, was obtained from NCIMB, a laboratory in The United Kingdom National Culture Collection, UKNCC.

Bacteria of the *Xanthomonas* genus and *Pseudomonas fluorescens* were cultured in accordance with the method described in "(1) Obtainment and Culture of Plant Pathogenic Bacteria" in Example 1.

### (2) Isolation and Purification of 3rd Phage

A novel phage was isolated from natural dirty water or soil obtained in Japan. A method for isolation and a method for purification were in accordance with the methods described in "(2) Isolation and Purification of 1st Phage" in Example 1. Consequently, one kind of new phage was isolated from natural dirty water and soil. This novel phage isolated in Example 3 is referred to as a "3rd phage".

The isolated phage was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to purify the phage.

### (3) Amplification and Refinement of 3rd Phage

The 3rd phage, isolated and purified, was amplified and refined by the plate lysate (PL) method. Specific method was in accordance with the method described in "(3) Amplification and Refinement of 1 st Phage" in Example 1. The titer of the refined solution of the 3rd phage prepared was determined by a plaque assay method using a solution suitably diluted, and was confirmed to be 10⁸ PFU/mL or more.

### (4) Evaluation of Host Range of 3rd Phage

The host range of the 3rd phage was evaluated by the spot test method. The basic operation was in accordance with the method described in "(4) Evaluation of Host Range of 1st Phage" in Example 1.

One example of the results is shown in Figure 3. When the 3rd phage exhibited bacteriolytic ability, only the site where the refined solution of phage was dropped became clear in the bacterial lawn formed on the plate. The 3rd phage obtained in the present invention exhibited bacteriolytic ability against all the bacteria of the *Xanthomonas* genus shown in Table 5. On the other hand, the phage did not exhibit bacteriolytic ability against *Pseudomonas fluorescens.* Generally, the host range of a phage is known to be limited to a local specific strain of a specific bacterial species (Sharma S. et al., Folia Microbiol., 2017, 62: 17-55; Nakayinga R. et al., BMC Microbiology, 2021, 21: 291). As shown by the places of harvest in Table 5, however, the phage isolated in this Example exhibited bacteriolytic ability against each of the bacteria of the *Xanthomonas* genus separated in different places. This suggests that the 3rd phage isolated exhibited bacteriolytic ability against a wide range of bacteria of the *Xanthomonas* genus.

Additionally, these results suggest that the 3rd phage is useful for control of shot hole disease of *Prunus spp.,* which is caused by bacteria of the *Xanthomonas* genus.

### (5) Genomic Analysis of 3rd Phage

The genomic DNA sequence of the 3rd phage was determined and analyzed.

### (i) Preparation and Sequencing of Genomic DNA of 3rd Phage

The genome of the 3rd phage was extracted using a TURBO DNA-free^{™} kit (Thermo Fisher Scientific Inc.). The basic operation was in accordance with the method described in "(5) Genomic Analysis of 1st Phage, (i) Preparation and Sequencing of Genomic DNA of 1st Phage" in Example 1.

### (ii) Bioinformatics Analysis based on Genomic Sequence Information

On the basis of the genomic nucleotide sequence (SEQ ID NO: 14) of the 3rd phage obtained in (i) above, and using the NCBI-provided BLAST server (the same as the above), similar DNA sequences were searched for, and the sequence identities were verified. As a result of the search, several kinds of *Pseudomonas* phage complete genomes (the access codes: KX129925.1, MN504636.1, KX898399.1, NC_041953.1, and MW835180.1) had the highest analogous score. However, all of the sequences only had a sequence identity of approximately 75% over the very small range of from 6% to 7% of the full length of the genome. Moreover, for these phages, the host bacteria are different from bacteria of the *Xanthomonas* genus. This result suggests that the 3rd phage is a phage having a novel genomic sequence, the analogous genomic sequence of which is not known at all.

### <Example 4: Isolation of Novel Bacteriophage, and Bacteriolytic Ability Thereof (4)>

### (Purpose)

The purpose is to isolate a novel bacteriophage having bacteriolytic ability against pathogenic bacteria of a plant disease, and verify the bacteriolytic ability against the plant pathogenic bacteria.

### (Materials and Method)

### (1) Obtainment and Culture of Plant Pathogenic Bacteria

In this Example, the plant pathogenic bacteria as target bacteria were bacteria of the *Xanthomonas* genus, and all the bacterial strains were obtained from the National Agriculture and Food Research Organization (NARO). Each of the bacteria used in this Example is listed in Table 6, together with the deposition numbering (MAFF No.) by the National Agriculture and Food Research Organization.

**[Table 6]**

| ID | Species | Source of Isolation | Place of Harvest |
|---|---|---|---|
| MAFF No. 311282 | *Xanthomonas arboricola* pv. *pruni* | Peach | Fukushima |
| MAFF No. 311351 | *Xanthomonas arboricola* pv. *pruni* | Peach | Fukushima |
| MAFF No. 311417 | *Xanthomonas arboricola* pv. *pruni* | Peach | Fukushima |
| MAFF No. 311579 | *Xanthomonas arboricola* pv. *pruni* | Peach | Fukushima |
| MAFF No. 311584 | *Xanthomonas arboricola* pv. *pruni* | Peach | Fukushima |
| MAFF No. 311586 | *Xanthomonas arboricola* pv. *pruni* | Peach | Nagano |
| MAFF No. 311618 | *Xanthomonas arboricola* pv. *pruni* | Peach | Wakayama |
| MAFF No. 311641 | *Xanthomonas arboricola* pv. *pruni* | Peach | Fukushima |
| NCIMB-ID:10460 | *Pseudomonas fluorescens* | Egg | U.S.A. |

As a control, a *Pseudomonas fluorescens* bacterial strain (NCIMB-ID: 10460), which is of *Pseudomonas* genus, was obtained from NCIMB, a laboratory in The United Kingdom National Culture Collection, UKNCC.

Bacteria of the *Xanthomonas* genus and *Pseudomonas fluorescens* were cultured in accordance with the method described in "(1) Obtainment and Culture of Plant Pathogenic Bacteria" in Example 1.

### (2) Isolation and Purification of 4th Phage

A novel phage was isolated from natural dirty water or soil obtained in Japan. A method for isolation and a method for purification were in accordance with the methods described in "(2) Isolation and Purification of 1st Phage" in Example 1. Consequently, one kind of new phage was isolated from natural dirty water and soil. This novel phage isolated in Example 4 is referred to as a "4th phage".

The isolated phage was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to purify the phage.

### (3) Amplification and Refinement of 4th Phage

The 4th phage isolated and purified was amplified and refined by the plate lysate (PL) method that is an amplifying method using a plaque assay method. Specific method was in accordance with the method described in "(3) Amplification and Refinement of 1st Phage" in Example 1. The titer of the refined solution of the 4th phage prepared was determined by a plaque assay method using a solution suitably diluted, and was confirmed to be 10⁸ PFU/mL or more.

### (4) Evaluation of Host Range of 4th Phage

The host range of the 4th phage was evaluated by the spot test method. The basic operation was in accordance with the method described in "(4) Evaluation of Host Range of 1st Phage" in Example 1. One example of the results is shown in Figure 4. When the 4th phage exhibited bacteriolytic ability, only the site where the refined solution of phage was dropped became clear in the bacterial lawn formed on the plate. The 4th phage obtained in the present invention exhibited bacteriolytic ability against all bacterial strains of the bacteria of the *Xanthomonas* genus shown in Table 6. As with Example 3, the phage exhibited bacteriolytic ability against each of the bacteria of the *Xanthomonas* genus separated in different places. This suggests that the isolated phage exhibited bacteriolytic ability against a wide range of bacteria of the *Xanthomonas* genus.

Additionally, these results suggest that the 4th phage is useful for control of shot hole disease of *Prunus spp.,* which is caused by bacteria of the *Xanthomonas* genus.

### (5) Genomic Analysis of 4th Phage

The genomic DNA sequence of the 4th phage was determined and analyzed.

### (i) Preparation and Sequencing of Genomic DNA of 4th Phage

The genome of the phage was extracted using a TURBO DNA-free^{™} kit (Thermo Fisher Scientific Inc.). The basic operation was in accordance with the method described in "(5) Genomic Analysis of 1 st Phage, (i) Preparation and Sequencing of Genomic DNA of 1 st Phage" in Example 1.

### (ii) Bioinformatics Analysis based on Genomic Sequence Information

On the basis of the genomic nucleotide sequence (SEQ ID NO: 17) of the phage obtained in (i) above, and using the NCBI-provided BLAST server (the same as the above), similar DNA sequences were searched for, and the sequence identities were verified. As a result of the search, several phages separated in Belgium and designated as FoX as described in Nakayinga (Nakayinga R. et al., BMC Microbiology, 2021, 21: 291) (the access codes: NC_055835.1, NC_055836.1, NC_055837.1, and NC_055838.1) had the highest analogous score. However, all of the sequences only had a sequence identity as small as approximately 77% over the range of 10% of the full length. This result suggests that the phage isolated in Example 1 is a phage having a novel genomic sequence, the analogous sequence of which has basically not been reported.

Generally, it is considered that the characteristics related to the host specificity and host range of a phage depend largely on the role of a tail fiber protein (Nobrega F.L. et al., Nat. Rev. Microbiol., 2018, 16: 760-773). Accordingly, the genomic sequence information on the 4th phage was searched for tail fiber genes. Utilizing the RAST server (https://rast.nmpdr.org/) and PHASTER (https://phaster.ca/), the nucleotide sequence of SEQ ID NO: 16 was identified as an estimated tail fiber gene from the genomic sequence of the 4th phage. This gene sequence had a nucleotide sequence identity of 58% to the tail fiber gene of a known phage Fox. The host bacterial species reported for the phage Fox is *Xanthomonas campestris* (Nakayinga R. et al., BMC Microbiology, 2021, 21: 291). Accordingly, it has a different host range from the 4th phage.

Additionally, this result suggests that it is difficult to predict the host range of a phage having a tail fiber gene of a different nucleotide sequence, on the basis of the relationship between information on a known tail fiber gene and information on the host bacterial species of a phage having the gene.

From the above-described results, it has been estimated that the 4th phage has a conventionally unreported novel host range in the *Xanthomonas* genus, and that the characteristics of the phage rely on the novel tail fiber gene in particular.

### <Example 5: Isolation of Novel Bacteriophage, and Bacteriolytic Ability Thereof (5)>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In this Example, the plant pathogenic bacteria as target bacteria were bacteria of the *Xanthomonas* genus, and all the bacterial strains were obtained from the National Agriculture and Food Research Organization (NARO). Each of the bacteria used in this Example is listed in Table 7, together with the deposition numbering (MAFF No.) by the National Agriculture and Food Research Organization.

**[Table 7]**

| ID. | Species | Source of Isolation | Place of Harvest |
|---|---|---|---|
| MAFF No.211971 | *Xanthomonas arboricola* pv. *pruni* | Peach | Yamanashi |
| MAFF No.311351 | *Xanthomonas arboricola* pv. *pruni* | Peach | Fukushima |
| MAFF No.311622 | *Xanthomonas arboricola* pv. *pruni* | Peach | Yamanashi |
| NCIMB-ID:10460 | *Pseudomonas fluorescens* | Egg | U.S.A. |

As a control, a *Pseudomonas fluorescens* bacterial strain (NCIMB-ID: 10460), which is of *Pseudomonas* genus, was obtained from NCIMB, a laboratory in The United Kingdom National Culture Collection, UKNCC.

Bacteria of the *Xanthomonas* genus and *Pseudomonas fluorescens* were cultured in accordance with the method described in "(1) Obtainment and Culture of Plant Pathogenic Bacteria" in Example 1.

### (2) Isolation and Purification of 5th Phage

A novel phage was isolated from natural dirty water or soil obtained in Japan. A method for isolation and a method for purification were in accordance with the methods described in "(2) Isolation and Purification of 1st Phage" in Example 1. Consequently, two kinds of new phages were isolated from natural dirty water and soil. These novel phages isolated in Example 5 are referred to as a "5th phage".

The isolated phage was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to purify the phage.

### (3) Amplification and Refinement of 5th Phage

The 5th phage isolated and purified was amplified and refined by the plate lysate (PL) method. Specific method was in accordance with the method described in "(3) Amplification and Refinement of 1 st Phage" in Example 1. The titer of the refined solution of the 5th phage prepared was determined by a plaque assay method using a solution suitably diluted, and was confirmed to be 10⁸ PFU/mL or more.

### (4) Evaluation of Host Range of 5th Phage

The host range of the 5th phage was evaluated by the spot test method. The basic operation was in accordance with the method described in "(4) Evaluation of Host Range of 1st Phage" in Example 1.

One example of the results is shown in Figure 5. When the 5th phage exhibited bacteriolytic ability, only the site where the refined solution of phage was dropped became clear in the bacterial lawn formed on the plate. The 5th phage obtained in the present invention exhibited bacteriolytic ability against all the bacteria of the *Xanthomonas* genus shown in Table 7. On the other hand, the phage did not exhibit bacteriolytic ability against *Pseudomonas fluorescens.* As with Example 3, the phage exhibited bacteriolytic ability against each of the bacteria of the *Xanthomonas* genus separated in different places. This suggests that the 5th phage isolated exhibited bacteriolytic ability against a wide range of bacteria of the *Xanthomonas* genus.

Additionally, these results suggest that the 5th phage is useful for control of shot hole disease of *Prunus spp.,* which is caused by bacteria of the *Xanthomonas* genus.

### (5) Genomic Analysis of 5th Phage

The genomic DNA sequence of the 5th phage was determined and analyzed.

### (i) Preparation and Sequencing of Genomic DNA of 5th Phage

The genome of the 5th phage was extracted using a TURBO DNA-free^{™} kit (Thermo Fisher Scientific Inc.). The basic operation was in accordance with the method described in "(5) Genomic Analysis of 1st Phage, (i) Preparation and Sequencing of Genomic DNA of 1st Phage" in Example 1.

### (ii) Bioinformatics Analysis based on Genomic Sequence Information

On the basis of the genomic nucleotide sequences (SEQ ID NOs: 18 and 19) of the 5th phage obtained in (i) above, and using the NCBI-provided BLAST server (the same as the above), similar DNA sequences were searched for, and the sequence identities were verified. The search resulted in many hits for the related sequences. Over the range of 90% or more of the full length, the sequence having the highest value of sequence identity to SEQ ID NO: 18 was *Pseudomonas* phage vB_PaeS (the access code: LC552830.1), and the sequence having the highest sequence identity to SEQ ID NO: 19 was P1940 (the access code: MX298177.1), as described in the U.S. Patent Application Publication No. 2017-0319637. Each sequence exhibited the same degree of sequence identity to both of SEQ ID NOs: 18 and 19, as shown in Table 8 below.

**[Table 8]**

| | Access Code | SEQ ID No.18 | | SEQ ID No.19 | |
|---|---|---|---|---|---|
| | | Query Cover | Identity | Query Cover | Identity |
| vB_PaeS | LC552830.1 | 90% | 98.28 % | 87% | 98.05% |
| P1940 | MX298177.1 | 92 % | 97.45 % | 94% | 98.61% |

In the Table, the sequence identity is a value for the range automatically aligned by an analysis server with respect to the genomic full length of SEQ ID NO: 18 or 19. The range is shown as the value of Query Cover. For example, the sequence identity of 98.28% for vB_PaeS is a value calculated over the region limited to 90% of the full length of SEQ ID NO: 18. Accordingly, the sequence identity to the full length is estimated , although it is difficult to be calculated, to be at least a value lower than 98.28% and to correspond to a value lower than 90%. In the case of P1940, the sequence identity is estimated to correspond to a value lower than 95%.

The result of this analysis has revealed that the target bacteria of a phage having the genome of the above-described known sequence are bacteria of *Pseudomonas* genus. Accordingly, the 5th phage in this Example had an extremely high sequence identity to the genomic sequence of a known phage, but it was difficult to identify the target bacteria of the 5th phage. Simultaneously, it is highly plausible that a phage having a higher sequence identity to the 5th phage over a wider range than vB_PaeS and P1940 has the same host range, i.e., bacteria of the *Xanthomonas* genus, as for the 5th phage in this Example.

From the above-described results, it has been estimated that the 5th phage has a conventionally unreported novel host range in the *Xanthomonas* genus.

### <Example 6: Isolation of Novel Bacteriophage, and Bacteriolytic Ability Thereof (6)>

### (Purpose)

The purpose is to isolate a novel bacteriophage having bacteriolytic ability against pathogenic bacteria of a plant disease, and verify the bacteriolytic ability against the plant pathogenic bacteria.

### (Method and Results)

### (1) Obtainment and Culture of Plant Pathogenic Bacteria

In the present invention, the plant pathogenic bacteria as target bacteria were bacteria of the *Xanthomonas* genus, and all the bacterial strains were obtained from the National Agriculture and Food Research Organization (NARO). Each of the bacteria used in this Example is listed in Table 9, together with the deposition numbering (MAFF No.) by the National Agriculture and Food Research Organization.

**[Table 9]**

| ID | Species | Source of Isolation | Place of Harvest |
|---|---|---|---|
| MAFF No. 211971 | *Xanthomonas arboricola* pv. *pruni* | Peach | Yamanashi |
| MAFF No. 311351 | *Xanthomonas arboricola* pv. *pruni* | Peach | Fukushima |
| MAFF No. 311622 | *Xanthomonas arboricola* pv. *pruni* | Peach | Yamanashi |
| NCIMB-ID:10460 | *Pseudomonas fluorescens* | Egg | U.S.A. |

As a control, a *Pseudomonas fluorescens* bacterial strain (NCIMB-ID: 10460), which is of *Pseudomonas* genus, was obtained from NCIMB, a laboratory in The United Kingdom National Culture Collection, UKNCC.

Bacteria of the *Xanthomonas* genus and *Pseudomonas fluorescens* were cultured in accordance with the method described in "(1) Obtainment and Culture of Plant Pathogenic Bacteria" in Example 1.

### (2) Isolation and Purification of 6th Phage

A novel phage was isolated from natural dirty water or soil obtained in Japan. A method for isolation and a method for purification were in accordance with the methods described in "(2) Isolation and Purification of 1st Phage" in Example 1. Consequently, one kind of new phage was isolated from natural dirty water and soil. This novel phage isolated in Example 6 is referred to as a "6th phage".

The isolated phage was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to purify the phage.

### (3) Amplification and Refinement of 6th Phage

The 6th phage isolated and purified was amplified and refined by the plate lysate (PL) method. Specific method was in accordance with the method described in "(3) Amplification and Refinement of 1 st Phage" in Example 1. The titer of the refined solution of the 6th phage prepared was determined by a plaque assay method using a solution suitably diluted, and was confirmed to be 10⁸ PFU/mL or more.

### (4) Evaluation of Host Range of 6th Phage

The host range of the 6th phage was evaluated by the spot test method. The basic operation was in accordance with the method described in "(4) Evaluation of Host Range of 1st Phage" in Example 1.

One example of the results is shown in Figure 6. When the 6th phage exhibited bacteriolytic ability, only the site where the refined solution of phage was dropped became clear in the bacterial lawn formed on the plate. The 6th phage obtained in the present invention exhibited bacteriolytic ability against all the bacteria of the *Xanthomonas* genus shown in Table 9. On the other hand, the phage did not exhibit bacteriolytic ability against *Pseudomonas fluorescens.* As with Example 3, the phage exhibited bacteriolytic ability against each of the bacteria of the *Xanthomonas* genus separated in different places. This suggests that the 6th phage isolated exhibited bacteriolytic ability against a wide range of bacteria of the *Xanthomonas* genus.

Additionally, these results suggest that the 6th phage is useful for control of shot hole disease of *Prunus spp.,* which is caused by bacteria of the *Xanthomonas* genus.

### (5) Genomic Analysis of 6th Phage

The genomic DNA sequence of the 6th phage was determined and analyzed.

### (i) Preparation and Sequencing of Genomic DNA of 6th Phage

The genome of the 6th phage was extracted using a TURBO DNA-free^{™} kit (Thermo Fisher Scientific Inc.). The basic operation was in accordance with the method described in "(5) Genomic Analysis of 1st Phage, (i) Preparation and Sequencing of Genomic DNA of 1st Phage" in Example 1.

### (ii) Bioinformatics Analysis based on Genomic Sequence Information

On the basis of the genomic nucleotide sequence (SEQ ID NO: 23) of the 6th phage obtained in (i) above, and using the NCBI-provided BLAST server (the same as the above), similar DNA sequences were searched for, and the sequence identities were verified. As a result of the search, four phages (the access codes: NC_017864.1, NC_007806.1, NC_029019.1, and NC_024381.1) exhibited a sequence identity of 96.5 to 98.5% over the range of approximately 90% of the full length of the genome. The sequence identity to the full length is estimated to correspond to a value of approximately 85%. Despite such a high sequence identity, however, the target bacteria of these known phages are not *Xanthomonas* genus but *Pseudomonas* genus or *Stenotrophomonas* genus. Accordingly, this suggests that the 6th phage is a completely novel phage as a phage having bacteria of the *Xanthomonas* genus as the target bacteria.

Accordingly, to identify a gene related to target bacteria, the genomic sequence of the 6th phage and the above-described four known phages were compared. Furthermore, an ORF was identified in a region with the low sequence identity to the known phages, which was discovered by the comparison. The ORF was identified utilizing RAST server (https://rast.nmpdr.org/) and PHASTER (https://phaster.ca/).

The comparison has revealed that the ranges from position 8062 to position 9473 and from position 31995 to position 34230 in the genomic nucleotide sequence of SEQ ID NO: 23 are regions having a low sequence identity to the known phages. In these regions, a total of five ORFs were detected. Specifically, the ORFs were the DNA regions of the nucleotide sequences from position 8082 to position 8573, from position 8645 to position 8842, from position 9223 to position 9477, from position 31881 to position 32789, and from position 32792 to position 33694 in the genomic nucleotide sequence of SEQ ID NO: 23, respectively.

Each ORF was scrutinized to reveal the gene important for determining a host range. Consequently, in the 6th phage isolated in Example 6, the nucleotide sequence (SEQ ID NO: 22) from position 9223 to position 9477 in the genomic nucleotide sequence of SEQ ID NO: 23 has been estimated to be an important gene region with a high sequence identity, because it had the highest number of hits of nucleotide sequences with a sequence identity of 30% or more to known bacteriophages that target bacteria of the *Xanthomonas* genus.

To reveal whether this gene is a known gene, the NCBI-provided BLAST server was extensively searched for amino acid sequences similar to the amino acid sequence (SEQ ID NO: 21), which is a translation product, without limitation to a bacteriophage targeting bacteria of the *Xanthomonas* genus. Consequently, even a sequence that had the highest analogous score had a sequence identity as small as approximately 50%, and no sequence exhibiting a high sequence identity was detected.

### <Example 7: Isolation of Novel Bacteriophage, and Bacteriolytic Ability Thereof (7)>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In the present invention, the plant pathogenic bacteria as target bacteria were bacteria of the *Xanthomonas* genus, and all the bacterial strains were obtained from the National Agriculture and Food Research Organization (NARO). Each of the bacteria used in this Example is listed in Table 10, together with the deposition numbering (MAFF No.) by the National Agriculture and Food Research Organization.

**[Table 10]**

| ID | Species | Source of Isolation | Place of Harvest |
|---|---|---|---|
| MAFF No. 211971 | *Xanthomonas arboricola pv. pruni* | Peach | Yamanashi |
| MAFF No. 311351 | *Xanthomonas arboricola pv. pruni* | Peach | Fukushima |
| MAFF No. 311622 | *Xanthomonas arboricola pv. pruni* | Peach | Yamanashi |
| MAFF No. 106765 | *Xanthomonas campestris pv. campestris* | Broccoli | Ibaraki |
| MAFF No. 106642 | *Xanthomonas campestris pv. campestris* | Fescue | Ibaraki |
| NCIMB-ID:10460 | *Pseudomonas fluorescens* | Egg | U.S.A. |

As a control, a *Pseudomonas fluorescens* bacterial strain (NCIMB-ID: 10460), which is of *Pseudomonas* genus, was obtained from NCIMB, a laboratory in The United Kingdom National Culture Collection, UKNCC.

Bacteria of the *Xanthomonas* genus and Pseudomonas fluorescens were cultured in accordance with the method described in "(1) Obtainment and Culture of Plant Pathogenic Bacteria" in Example 1.

### (2) Isolation and Purification of 7th Phage

A novel phage was isolated from natural dirty water or soil obtained in Japan. A method for isolation and a method for purification were in accordance with the methods described in "(2) Isolation and Purification of 1st Phage" in Example 1. Consequently, two kinds of new phages were isolated from natural dirty water and soil. These novel phages isolated in Example 7 are referred to as "7th phage".

The isolated phage was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to purify the phage.

### (3) Amplification and Refinement of 7th Phage

The 7th phage isolated and purified was amplified and refined by the plate lysate (PL) method. Specific method was in accordance with the method described in "(3) Amplification and Refinement of 1 st Phage" in Example 1. The titer of the refined solution of the 7th phage prepared was determined by a plaque assay method using a solution suitably diluted, and was confirmed to be 10⁸ PFU/mL or more.

### (4) Evaluation of Host Range of 7th Phage

The host range of the 7th phage was evaluated by the spot test method. The basic operation was in accordance with the method described in "(4) Evaluation of Host Range of 1st Phage" in Example 1.

One example of the results is shown in Figure 7. When the 7th phage exhibited bacteriolytic ability, only the site where the refined solution of phage was dropped became clear in the bacterial lawn formed on the plate. The 7th phage obtained in the present invention exhibited bacteriolytic ability against all the bacteria of the *Xanthomonas* genus shown in Table 10. On the other hand, the phage did not exhibit bacteriolytic ability against *Pseudomonas fluorescens.* As with Example 1, the phage exhibited bacteriolytic ability against each of the diverse bacterial species, and exhibited bacteriolytic ability against each of the bacterial strains separated in different places. This suggests that the 7th phage isolated exhibited bacteriolytic ability against a wide range of bacteria of the *Xanthomonas* genus.

Additionally, these results suggest that the 7th phage is useful for control of shot hole disease of *Prunus spp.* and black rot disease of broccoli, which are caused by bacteria of the *Xanthomonas* genus.

### (5) Genomic Analysis of 7th Phage

The genomic DNA sequence of the 7th phage was determined and analyzed.

### (i) Preparation and Sequencing of Genomic DNA of 7th Phage

The genome of the 7th phage was extracted using a TURBO DNA-free^{™} kit (Thermo Fisher Scientific Inc.). The basic operation was in accordance with the method described in "(5) Genomic Analysis of 1st Phage, (i) Preparation and Sequencing of Genomic DNA of 1st Phage" in Example 1.

### (ii) Bioinformatics Analysis based on Genomic Sequence Information

On the basis of the genomic nucleotide sequences (SEQ ID NOs: 28 and 29) of the 7th phage obtained in (i) above, and using the NCBI-provided BLAST server (the same as the above), similar DNA sequences were searched for, and the sequence identities were verified. As a result of the search, the genomic sequences of the phages described in JP 2021-102635 (SEQ ID NOs: 19 and 20 in the literature (herein, SEQ ID NOs: 30 and 31 respectively)) had the highest analogous score. Both sequences had a sequence identity of approximately 92% over the range of 90% of the full length. From this result, the sequence identity to the full length is estimated to correspond to a value of approximately 85%. Additionally, the genomic nucleotide sequence of ΦXc10 (Nakayinga R. et al., BMC Microbiology, 2021, 21: 291) had the next highest analogous score. The genomic nucleotide sequence of the ΦXc10 (the access code: NC_047840.1) had a sequence identity of approximately 92% over the range of 87% of the full length. From this result, the sequence identity to the full length is estimated to correspond to a value of approximately 80%. Furthermore, the genomic nucleotide sequences of f30-Xaj and f20-Xaj (Nakayinga R. *et al.,* 2021, ibid.) had a high analogous score in the same manner. The genomic nucleotide sequences of the f30-Xaj and f20-Xaj (the access code: NC_030937.1 and NC_030928.1) had a sequence identity of approximately 91% over the range of 74% of the full length. From this result, the sequence identity to the full length is estimated to correspond to a value of approximately 70%. However, none of those phages targets the bacteria *Xanthomonas arboricola pv. pruni.*

To seek the cause of the difference in the target bacteria, the genomic nucleotide sequence information (SEQ ID NO: 28) of the 7th phage was searched for a novel gene. The gene region was estimated utilizing a RAST server (https://rast.nmpdr.org/) and PHASTER (https://phaster.ca/). As a result of the search, a group of genes encoding novel tail tubular proteins (tail tubular proteins) were identified as genes related to the host range of the 7th phage. These gene regions were located in the region with the nucleotide sequence that is the same between the genomic nucleotide sequences of the two kinds of 7th phages obtained in (i).

SEQ ID NO: 26 shows the nucleotide sequence of the tail tubular protein A gene comprised in the genomic nucleotide sequence (SEQ ID NO: 28) of the 7th phage obtained in this Example, and SEQ ID NO: 24 shows the amino acid sequence of the tail tubular protein A. On the other hand, SEQ ID NO: 27 (correctly SEQ ID NO: 58) shows the nucleotide sequence of the tail tubular protein B gene, and SEQ ID NO: 25 (correctly SEQ ID NO: 57) shows the amino acid sequence of the tail tubular protein B.

The tail tubular protein A and the tail tubular protein B are reported to be involved in the specific attachment of a phage to bacteria (Maozhi Hu, *et al.,* 2020, 9: 1, 855-867), and hence, there is a high possibility that the proteins are also related to the host range of the 7th phage. However, when the NCBI-provided BLAST server was searched on the basis of the amino acid sequence (SEQ ID NO: 24) of the tail tubular protein A and the amino acid sequence (SEQ ID NO: 25 (correctly SEQ ID NO: 57)) of the tail tubular protein B, there was no amino acid sequence having a sequence identity of 97% or more to either of the sequences. The sequence having the highest sequence identity of approximately 96% was the amino acid sequence of each of the tail tubular proteins of the above-described ΦXc10 and f20-Xaj.

The above-described facts suggest that these proteins are novel tail tubular proteins that mediate the bacteriolytic action against bacteria of the *Xanthomonas* genus.

### <Example 8: Isolation of Novel Bacteriophage, and Bacteriolytic Ability Thereof (8)>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In the present invention, the plant pathogenic bacteria as target bacteria were bacteria of the *Xanthomonas* genus, and all the bacterial strains were obtained from the National Agriculture and Food Research Organization (NARO). Each of the bacteria used in this Example is listed in Table 11, together with the deposition numbering (MAFF No.) by the National Agriculture and Food Research Organization.

**[Table 11]**

| ID | Species | Source of Isolation | Place of Harvest |
|---|---|---|---|
| MAFF No. 211971 | *Xanthomonas arboricola* pv. *pruni* | Peach | Yamanashi |
| MAFF No. 311282 | *Xanthomonas arboricola* pv. *pruni* | Peach | Fukushima |
| MAFF No. 301420 | *Xanthomonas arboricola* pv. *pruni* | Peach | Nagano |
| MAFF No. 301426 | *Xanthomonas arboricola* pv. *pruni* | Peach | Fukushima |
| MAFF No. 311351 | *Xanthomonas arboricola* pv. *pruni* | Peach | Fukushima |
| MAFF No. 311414 | *Xanthomonas arboricola* pv. *pruni* | Peach | Fukushima |
| MAFF No. 311417 | *Xanthomonas arboricola* pv. *pruni* | Peach | Fukushima |
| MAFF No. 311562 | *Xanthomonas arboricola* pv. *pruni* | Peach | Yamanashi |
| MAFF No. 311571 | *Xanthomonas arboricola* pv. *pruni* | Peach | Fukushima |
| MAFF No. 311618 | *Xanthomonas arboricola* pv. *pruni* | Peach | Wakayama |
| MAFF No. 311622 | *Xanthomonas arboricola* pv. *pruni* | Peach | Yamanashi |
| NCIMB-ID:10460 | *Pseudomonas fluorescens* | Egg | U.S.A. |

As a control, a *Pseudomonas fluorescens* bacterial strain (NCIMB-ID: 10460), which is of *Pseudomonas* genus, was obtained from NCIMB, a laboratory in The United Kingdom National Culture Collection, UKNCC.

Bacteria of the *Xanthomonas* genus and Pseudomonas fluorescens were cultured in accordance with the method described in "(1) Obtainment and Culture of Plant Pathogenic Bacteria" in Example 1.

### (2) Isolation and Purification of 8th Phage

A novel phage was isolated from natural dirty water, soil, or part of a plant (leaf) obtained in Japan. A method for isolation and a method for purification were in accordance with the methods described in "(2) Isolation and Purification of 1st Phage" in Example 1. Consequently, five kinds of new phages were isolated from natural dirty water or soil or water having part of a plant immersed therein. These novel phages isolated in Example 8 are referred to as an "8th phage".

The isolated phage was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to purify the phage.

### (3) Amplification and Refinement of 8th Phage

The 8th phage isolated and purified was amplified and refined by the plate lysate (PL) method. Specific method was in accordance with the method described in "(3) Amplification and Refinement of 1 st Phage" in Example 1. The titer of the refined solution of the 8th phage prepared was determined by a plaque assay method using a solution suitably diluted, and was confirmed to be 10⁸ PFU/mL or more.

### (4) Evaluation of Host Range of 8th Phage

The host range of the 8th phage was evaluated by the spot test method. The basic operation was in accordance with the method described in "(4) Evaluation of Host Range of 1st Phage" in Example 1.

One example of the results is shown in Figure 8 and Figure 9. When the 8th phage exhibited bacteriolytic ability, only the site where the refined solution of phage was dropped became clear in the bacterial lawn formed on the plate. The 8th phage obtained in the present invention exhibited bacteriolytic ability against all the bacteria of the *Xanthomonas* genus shown in Table 11. On the other hand, the phage did not exhibit bacteriolytic ability against *Pseudomonas fluorescens.* As with Example 3, the phages exhibited bacteriolytic ability against each of the bacteria of the *Xanthomonas* genus separated in different places. This suggests that the 8th phage isolated exhibited bacteriolytic ability against a wide range of bacteria of the *Xanthomonas* genus.

Additionally, these results suggest that the 8th phage is useful for control of shot hole disease of *Prunus spp.,* which is caused by bacteria of the *Xanthomonas* genus.

### (5) Genomic Analysis of 8th Phage

The genomic DNA sequence of the 8th phage was determined and analyzed.

### (i) Preparation and Sequencing of Genomic DNA of 8th Phage

The genome of the 8th phage was extracted using a TURBO DNA-free^{™} kit (Thermo Fisher Scientific Inc.). The basic operation was in accordance with the method described in "(5) Genomic Analysis of 1st Phage, (i) Preparation and Sequencing of Genomic DNA of 1st Phage" in Example 1.

### (ii) Bioinformatics Analysis based on Genomic Sequence Information

On the basis of the genomic nucleotide sequences (SEQ ID NOs: 18 and 19) of the 8th phage obtained in (i) above, and using the NCBI-provided BLAST server (the same as the above), similar DNA sequences were searched for, and the sequence identities were verified. As a result of the search, there was no hit for sequence with a sequence identity of 80% or more to any of SEQ ID NOs: 32 to 36 over the range of 50% or more of the full length. Furthermore, there was no hit for sequence with a sequence identity of 90% or more to any of SEQ ID NOs: 32 to 36 over the range of 20% or more of the full length, as well. This result suggests that each of the 8th phages isolated in Example 8 is a phage having a basically novel genomic sequence, the analogous sequence of which has not been reported.

Additionally, a sequence identity between the genomic DNA sequences of each phage of SEQ ID NOs: 32 to 36 was analyzed with BLAST using GENETYX-NGS packaged in the genetic information processing software GENETYX (https://www.genetyx.co.jp/). The sequence identities based on the average nucleotide identity (ANI) as the index are as shown in Table 12 below.

**[Table 12]**

| | SEQ ID NO: 32 | SEQ ID NO: 33 | SEQ ID NO: 34 | SEQ ID NO: 35 | SEQ ID NO: 36 |
|---|---|---|---|---|---|
| SEQ ID NO: 32 | | 99.98/99.33 | 98.41/98.96 | 100/99.86 | 99.97/97.57 |
| SEQ ID NO: 33 | 99.98/97.72 | | 98.47/99.03 | 99.97/99.79 | 99.91/97.79 |
| SEQ ID NO: 34 | 99.52/93.22 | 99.55/93.12 | | 99.52/90.98 | 99.54/90.83 |
| SEQ ID NO: 35 | 100/97.70 | 99.97/97.70 | 99.15/94.81 | | 99.89/99.65 |
| SEQ ID NO: 36 | 99.88/99.70 | 99.91/99.69 | 99.44/93.11 | 99.88/97.72 | |

In Table 12, each of the ANI value and range to be compared of the genomic DNA sequence of the row with respect to the genomic DNA sequence of the column is shown in the form of "ANI value (%) / range to be compared (%)". Here, the range to be compared (%) in Table 12 is the ratio of a region aligned with respect to the genomic DNA sequence of the corresponding row out of the genomic DNA sequence of the corresponding column. For example, when the ANI value (%) / the range to be compared (%) is "90/90", the genomic DNA sequence of the corresponding row has a sequence identity of 90% to the genomic DNA sequence of the corresponding column over the range of 90% of the full length.

Each of the phages of SEQ ID NOs: 32 to 36 has different genomic DNA sequence lengths, and thus, in Table 12, the ANI value and range to be compared of the genomic DNA sequence of each row with respect to the genomic DNA sequence of each column may be subtly different from the ANI value and range to be compared when the row and the column are interchanged. For example, when the ANI value and range to be compared for the row of SEQ ID NO: 32 and the column of SEQ ID NO: 35 and the ANI value and range to be compared for the row of SEQ ID NO: 35 in the column of SEQ ID NO: 32 are compared, the ANI value is 100% for both, but the values of the range to be compared are different because SEQ ID NOs: 32 and 35 have different genome lengths.

As shown in Table 12, the nucleotide sequences of SEQ ID NOs: 32 to 36 have a sequence identity of 98 to 100% to one another over the range of 90% to 100%, and have been revealed to be very similar to one another. The sequence identity of SEQ ID NO: 35 to SEQ ID NO: 36 in the whole range was the lowest at 90%, when the sequence identity in the whole range was estimated by multiplying the ANI value and range to be compared in Table 12.

The results of comparison of the genomic DNA sequences of the above-described phages have revealed that a phage having a genomic DNA sequence comprising a nucleotide sequence having a sequence identity of 90% or more to the nucleotide sequence of any of SEQ ID NOs: 32 to 36, i.e., a phage having a genomic DNA sequence comprising the nucleotide sequence with addition, deletion, and/or substitution of approximately 10% of the nucleotides in the nucleotide sequence of any of SEQ ID NOs: 32 to 36, also has essentially the same host range and bacteriolytic ability as a phage having the genomic DNA sequence of any of SEQ ID NOs: 32 to 36.

### <Example 9: Isolation of Novel Bacteriophage, and Bacteriolytic Ability Thereof (9)>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In the present invention, the plant pathogenic bacteria as target bacteria were bacteria of the *Xanthomonas* genus, and all the bacterial strains were obtained from the National Agriculture and Food Research Organization (NARO). Each of the bacteria used in this Example is listed in Table 13, together with the deposition numbering (MAFF No.) by the National Agriculture and Food Research Organization.

**[Table 13]**

| ID | Species | Source of Isolation | Place of Harvest |
|---|---|---|---|
| MAFF No. 311622 | *Xanthomonas arboricola* pv. *pruni* | Peach | Yamanashi |
| MAFF No. 212146 | *Xanthomonas arboricola* pv. *juglandis* | Walnut | Nagano |
| MAFF No. 301078 | *Xanthomonas citri* subsp. *citri* | Orange | Wakayama |
| MAFF No. 301260 | *Xanthomonas campestris* pv. *vesicatoria* | Pepper | Tokushima |
| MAFF No. 301352 | *Xanthomonas campestris* pv. *vitians* | Lettuce | Wakayama |
| MAFF No. 301354 | *Xanthomonas campestris* pv. *vitians* | Lettuce | Chiba |
| MAFF No. 106783 | *Xanthomonas campestris* pv. *campestris* | Chinese cabbage | Shizuoka |
| MAFF No. 106181 | *Xanthomonas campestris* pv. *raphani* | Radish | Aomori |
| NCIMB-ID 10460 | *Pseudomonas fluorescens* | Egg | U.S.A. |

As a control, a *Pseudomonas fluorescens* bacterial strain (NCIMB-ID: 10460) that is reported to have a plant growth promoting action and the like, and is beneficial to the environment was obtained from NCIMB, a laboratory in The United Kingdom National Culture Collection, UKNCC.

Each of bacteria of the *Xanthomonas* genus and *Pseudomonas fluorescens* were cultured in accordance with the method described in "(1) Obtainment and Culture of Plant Pathogenic Bacteria" in Example 1.

### (2) Isolation and Purification of 9th Phage

A novel phage was isolated from natural dirty water, soil, or part of a plant (leaf) obtained in Japan. A method for isolation and a method for purification were in accordance with the methods described in "(2) Isolation and Purification of 1st Phage" in Example 1. Consequently, five kinds of new phages were isolated from natural dirty water or soil or water having part of a plant immersed therein. These novel phages isolated in Example 9 are referred to as a "9th phage".

The isolated phage was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to purify the phage.

### (3) Amplification and Refinement of 9th Phage

The 9th phage isolated and purified was amplified and refined by the plate lysate (PL) method. Specific method was in accordance with the method described in "(3) Amplification and Refinement of 1 st Phage" in Example 1. The titer of the refined solution of the 9th phage prepared was determined by a plaque assay method using a solution suitably diluted, and was confirmed to be 10⁸ PFU/mL or more.

### (4) Evaluation of Host Range of 9th Phage

The host range of the 9th phage was evaluated by the spot test method. The basic operation was in accordance with the method described in "(4) Evaluation of Host Range of 1st Phage" in Example 1.

One example of the results is shown in Figure 10. When the phage exhibited bacteriolytic ability, only the site where the refined solution of phage was dropped became clear in the bacterial lawn formed on the plate. The phages obtained in the present invention exhibited bacteriolytic ability against all the bacterial strains belonging to the three species of bacteria: *Xanthomonas arboricola, Xanthomonas citri,* and *Xanthomonas campestris,* which are shown in Table 13. Additionally, in the case of *Xanthomonas arboricola* and *Xanthomonas campestris,* the phages exhibited bacteriolytic ability against the bacterial strains of different pathovars. On the other hand, it has also been verified that the phages do not exhibit bacteriolytic ability against *Pseudomonas fluorescens.* There is an example of a report that a phage exhibits the ability against two or more species of bacteria of the *Xanthomonas* genus, but such a pattern as described above, including a pathovar, has not been reported (Nakayinga R. et al., BMC Microbiology, 2021, 21: 291). This result suggests the possibility that a phage of the present invention can be applied to various plant diseases. For example, this suggests the usefulness for control of shot hole disease of *Prunus spp.,* orange bacterial canker, black rot disease of broccoli, and the like, which are caused by bacteria of the *Xanthomonas* genus, and is expected to be very valuable in industrial uses.

### (5) Genomic Analysis of 9th Phage

The genomic DNA sequence of the 9th phage was determined and analyzed.

### (i) Preparation and Sequencing of Genomic DNA of 9th Phage

The genome of the 9th phage was extracted using a TURBO DNA-free^{™} kit (Thermo Fisher Scientific Inc.). The basic operation was in accordance with the method described in "(5) Genomic Analysis of 1st Phage, (i) Preparation and Sequencing of Genomic DNA of 1st Phage" in Example 1.

### (ii) Bioinformatics Analysis based on Genomic Sequence Information

On the basis of the genomic nucleotide sequence (SEQ ID NO: 41) of the 9th phage obtained in (i) above, and using the NCBI-provided BLAST server (the same as the above), similar DNA sequences were searched for, and the sequence identities were verified. As a result of the search, the genomic sequence (the NCBI access code: MT951568.1) of a phage *"Xanthomonas* phage Xaa_vB_phi31" listed in the NCBI database had the highest analogous score. The sequence had a sequence identity of approximately 85% over the range of 80% of the full length. From this result, the sequence identity to the full length is estimated to correspond to a value of approximately 68%. As a host of Xaa _vB_phi31, only *Xanthomonas euvesicatoria pv. allii* XaaBL11 was described. There was no information suggesting that other phages that made a high analogous score also exhibit bacteriolytic ability against a wide range of various species of bacteria of the *Xanthomonas* genus as for the 9th phage.

To seek the cause of the difference in the target bacteria, the genomic nucleotide sequence information (SEQ ID NO: 41) on the 9th phage was searched for a novel gene. The gene region was estimated utilizing a RAST server (https://rast.nmpdr.org/) and PHASTER (https://phaster.ca/). As a result of the search, a group of genes encoding novel tail tubular proteins (tail tubular proteins) were identified as genes related to the host range of the phage.

SEQ ID NO: 39 shows the nucleotide sequence of the tail tubular protein A gene comprised in the genomic nucleotide sequence (SEQ ID NO: 41) of the 9th phage obtained in this Example, and SEQ ID NO: 37 shows the amino acid sequence of the tail tubular protein A. On the other hand, SEQ ID NO: 40 shows the nucleotide sequence of the tail tubular protein B gene, and SEQ ID NO: 38 shows the amino acid sequence of the tail tubular protein B.

The tail tubular protein A and the tail tubular protein B are reported to be involved in the specific attachment of a phage to bacteria (Maozhi Hu, *et al.,* 2020, *9*: 1, 855-867), and hence, there is a high possibility that the proteins are also related to the host range of the 9th phage. However, when the NCBI-provided BLAST server was searched on the basis of the amino acid sequence (SEQ ID NO: 37) of the tail tubular protein A and the amino acid sequence (SEQ ID NO: 38) of the tail tubular protein B, there was no amino acid sequence having a sequence identity of 97% or more to either of the sequences. As to the amino acid sequence of a tail tubular protein A, the amino acid sequence of the tail tubular protein A of the above-described *Xanthomonas* phage Xaa_vB_phi31 exhibited the highest sequence identity of approximately 95%. The sequence identity between the nucleotide sequence of SEQ ID NO: 39 and the nucleotide sequence encoding the tail tubular protein A of Xaa _vB_phi31 was approximately 89%. As to the amino acid sequence of a tail tubular protein B, the amino acid sequence of the tail tubular protein B of Xaa _vB_phi31 also exhibited the highest sequence identity of approximately 91%. Additionally, the sequence identity of the nucleotide sequence of the gene to the nucleotide sequence of SEQ ID NO: 40 was approximately 86%.

The above-described facts suggest that these proteins are novel tail tubular proteins that mediate the bacteriolytic action against bacteria of the *Xanthomonas* genus.

### <Example 10: Isolation of Novel Bacteriophage, and Bacteriolytic Ability Thereof (10)>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In this Example, the plant pathogenic bacteria were bacteria of the *Xanthomonas* genus, and all the bacterial strains were obtained from the National Agriculture and Food Research Organization (NARO). Each of the bacteria used in this Example are the same as the bacteria listed in Table 13 in Example 9.

As a control, a *Pseudomonas fluorescens* bacterial strain (NCIMB-ID: 10460), which is of *Pseudomonas* genus, was obtained from NCIMB, a laboratory in The United Kingdom National Culture Collection, UKNCC.

Bacteria of the *Xanthomonas* genus and Pseudomonas fluorescens were cultured in accordance with the method described in "(1) Obtainment and Culture of Plant Pathogenic Bacteria" in Example 1.

### (2) Isolation and Purification of 10th Phage

A novel phage was isolated from natural dirty water or soil obtained in Japan. A method for isolation and a method for purification were in accordance with the methods described in "(2) Isolation and Purification of 1st Phage" in Example 1. Consequently, one kind of new phage was isolated from natural dirty water and soil. This novel phage isolated in Example 10 is referred to as a "10th phage".

The isolated phage was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to purify the phage.

### (3) Amplification and Refinement of 10th Phage

The 10th phage isolated and purified was amplified and refined by the plate lysate (PL) method. Specific method was in accordance with the method described in "(3) Amplification and Refinement of 1st Phage" in Example 1. The titer of the refined solution of the 10th phage was determined by a plaque assay method using a solution suitably diluted, and was confirmed to be 10⁸ PFU/mL or more.

### (4) Evaluation of Host Range of 10th Phage

The host range of the 10th phage was evaluated by a spot test method. The basic operation was in accordance with the method described in "(4) Evaluation of Host Range of 1st Phage" in Example 1.

One example of the results is shown in Figure 11. When the phage exhibited bacteriolytic ability, only the site where the refined solution of phage was dropped became clear in the bacterial lawn formed on the plate. The 10th phage exhibited bacteriolytic ability against all the bacterial strains belonging to the three species of bacteria: *Xanthomonas arboricola, Xanthomonas citri,* and *Xanthomonas campestris,* which are shown in Table 13. Additionally, in the case of *Xanthomonas arboricola* and *Xanthomonas campestris,* the phages exhibited bacteriolytic ability against the bacterial strains of different pathovars. On the other hand, it has also been verified that the phage does not exhibit bacteriolytic ability against *Pseudomonas fluorescens.* There is an example of a report that a phage exhibits the ability against two or more species of bacteria of the *Xanthomonas* genus, but such a pattern as described above, including a pathovar, has not been reported (Nakayinga R. et al., BMC Microbiology, 2021, 21: 291). This result suggests the possibility that the 10th phage can be applied to various plant diseases. For example, this suggests the usefulness for control of shot hole disease of *Prunus spp.,* orange bacterial canker, black rot disease of broccoli, and the like, which are caused by a bacteria of the *Xanthomonas* genus, and is expected to be very valuable in industrial uses.

### (5) Genomic Analysis of 10th Phage

The genomic DNA sequence of the 10th phage was determined and analyzed.

### (i) Preparation and Sequencing of Genomic DNA of 10th Phage

The genome of the 10th phage was extracted using a TURBO DNA-free^{™} kit (Thermo Fisher Scientific Inc.). The basic operation was in accordance with the method described in "(5) Genomic Analysis of 1st Phage, (i) Preparation and Sequencing of Genomic DNA of 1st Phage" in Example 1.

### (ii) Bioinformatics Analysis based on Genomic Sequence Information

On the basis of the genomic nucleotide sequence (SEQ ID NO: 44) of the 10th phage obtained in (i) above, and using the NCBI-provided BLAST server (the same as the above), similar DNA sequences were searched for, and the sequence identities were verified. As a result of the search, a phage (the name: RiverRider; the NCBI access code: NC_048703.1) separated in the U.S.A. and described in the literature of Miller *et al.* (Miller M. et al., Archives of Virology, 2020, 165: 1481-1484) had the highest analogous score. The sequence identity was approximately 91% to SEQ ID NO: 44 over the range of approximately 80% of the full length, and a sequence identity to the full length of the genomic sequence was 80% or less (corresponding to approximately 73%). This result has revealed that the 10th phage isolated in this Example is a phage having a novel genomic sequence.

Generally, it is considered that the characteristics related to the host specificity and host range of a phage depend largely on the role of a tail fiber protein (Nobrega F.L. et al., Nat. Rev. Microbiol., 2018, 16: 760-773). Accordingly, the genomic sequence information on the phage was searched for tail fiber genes. Utilizing the RAST server (https://rast.nmpdr.org/) and PHASTER (https://phaster.ca/), the nucleotide sequence of SEQ ID NO: 43 was identified as an estimated tail fiber gene from the genomic sequence of the 10th phage. A DNA sequence similar to this gene sequence was searched for, and consequently, the sequence of the tail fiber gene of the above-described phage RiverRider had the highest analogous score. The sequence identity of this nucleotide sequence was approximately 77% in the first half (1st to 890th: corresponding to approximately 52% of the full length) of the nucleotide sequence of SEQ ID NO: 43. As to the amino acid sequence of SEQ ID NO: 42, the sequence having the highest analogous score was the tail fiber protein of RiverRider as well. The sequence identity of the amino acid sequence was approximately 82% over approximately 52% of the full length. This has revealed that the tail fiber protein of the 10th phage is a novel protein to which no other protein is highly analogous, and is a protein characteristic particularly to a phage of the present invention, compared with a known phage.

As to the host bacteria of the phage RiverRider, the above-described literature states that the phage exhibited bacteriolytic ability against *Xanthomonas fragariae*, but exhibited bacteriolytic ability against neither *Xanthomonas arboricola* nor *Xanthomonas campestris.* Accordingly, this phage has a clearly different host range from the 10th phage, suggesting that a large difference in the host range between the phage RiverRider and the phage obtained in this Example is due to a difference in the tail fiber gene.

From the above-described results, it has been estimated that the 10th phage has a conventionally unreported novel host range in the *Xanthomonas* genus, and that the characteristics of the phage rely on the novel tail fiber gene in particular.

### <Example 11: Isolation of Novel 2nd Bacteriophage, and Bacteriolytic Ability Thereof>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In this Example, the plant pathogenic bacteria as target bacteria were bacteria of the *Xanthomonas* genus, and all the bacterial strains were obtained from the National Agriculture and Food Research Organization (NARO). In this Example, the bacteria listed in Table 14 were used in addition to the bacterium described in Table 4.

**[Table 14]**

| ID | Species | Source of Isolation | Place of Harvest |
|---|---|---|---|
| MAFF No. 106765 | *Xanthomonas campestris pv. campestris* | Broccoli | Ibaraki |

Additionally, as a control, a *Pseudomonas fluorescens* bacterial strain (NCIMB-ID: 10460), which is of *Pseudomonas* genus, was obtained from NCIMB, a laboratory in The United Kingdom National Culture Collection, UKNCC.

Bacteria of the *Xanthomonas* genus and Pseudomonas fluorescens were cultured in accordance with the method described in "(1) Obtainment and Culture of Plant Pathogenic Bacteria" in Example 1.

### (2) Isolation and Purification of Phage

A novel phage was isolated from natural dirty water or soil obtained in Japan. A method for isolation and a method for purification were in accordance with the methods described in "(2) Isolation and Purification of 1st Phage" in Example 1. Consequently, two kinds of new phages were isolated from natural dirty water and soil.

The isolated phage was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to purify the phage.

### (3) Amplification and Refinement of Phage

The 10th phage isolated and purified was amplified and refined by the plate lysate (PL) method. Specific method was in accordance with the method described in "(3) Amplification and Refinement of 1st Phage" in Example 1. The titer of the refined solution of phage prepared was determined by a plaque assay method using a solution suitably diluted, and was confirmed to be 10⁸ PFU/mL or more.

### (4) Evaluation of Host Range of Phage

The host range of the phage was evaluated by a spot test method. The basic operation was in accordance with the method described in "(4) Evaluation of Host Range of 1st Phage" in Example 1.

One example of the results is shown in Figures 14 and 15. When the phage exhibited bacteriolytic ability, only the site where the refined solution of phage was dropped became clear in the bacterial lawn formed on the plate. Two kinds of phages obtained in the present invention exhibited bacteriolytic ability against all the bacteria of the *Xanthomonas* genus shown in Table 4, in the same manner as the 2nd phage did (Figure 14). On the other hand, the phages did not exhibit bacteriolytic ability against *Pseudomonas fluorescens.* This suggests the possibility that the two kinds of phages isolated are highly analogous to the 2nd phage. Furthermore, the 2nd phage obtained in Example 2 and the two kinds of phages obtained in this Example were examined for bacteriolytic ability against *Xanthomonas campestris pv. campestris.* Consequently, all three kinds of phages exhibited bacteriolytic ability against this bacterial strain (Figure 15).

Additionally, these results suggest that the phage obtained in this Example is useful for control of shot hole disease of *Prunus spp.,* rice leaf blight, orange bacterial canker, black rot disease of broccoli, and the like, which are caused by bacteria of the *Xanthomonas* genus.

### (5) Genomic Analysis of Phage

The genomic DNA sequence of the phage obtained in this Example was determined and analyzed.

### (i) Preparation and Sequencing of Genomic DNA of Phage

The genome of two kinds of phages was extracted using a TURBO DNA-free^{™} kit (Thermo Fisher Scientific Inc.). The basic operation was in accordance with the method described in "(5) Genomic Analysis of 1st Phage, (i) Preparation and Sequencing of Genomic DNA of 1st Phage" in Example 1.

### (ii) Bioinformatics Analysis based on Genomic Sequence Information

On the basis of the genomic nucleotide sequences (SEQ ID NOs: 47 and 48) of the phages obtained in (i) above, and using GENETYX-NGS, the average nucleotide identity (ANI) to the genomic nucleotide sequence (SEQ ID NO: 13) of the 2nd phage was analyzed. As a result of the analysis, the average nucleotide identity (ANI) to SEQ ID NO: 13 was 98.90% for SEQ ID NO: 47 and 98.92% for SEQ ID NO: 48. Accordingly, the two kinds of phages obtained in this Example, are referred to as a "2nd phage" in addition to the phage obtained in Example 2.

To clarify the cause of the identity in host specificity between the phage isolated in this Example and the phage isolated in Example 2, the sequences of the tail fiber proteins of both phages were compared.

First, a tail fiber gene was identified from the genomic sequence of each of the two kinds of phages in this Example. The gene was identified utilizing a RAST server (https://rast.nmpdr.org/) and a PHASTER server (https://phaster.ca/). Consequently, the nucleotide sequence of SEQ ID NO: 46 was identified as the nucleotide sequence of the tail fiber gene. The nucleotide sequences of the tail fiber genes of the two kinds of phages isolated in this Example were the same.

Furthermore, the amino acid sequence (SEQ ID NO: 45) encoded by the gene consisting of the nucleotide sequence of SEQ ID NO: 46 was compared with the amino acid sequence (SEQ ID NO: 11) encoded by the tail fiber gene of the phage isolated in Example 2. Consequently, only the amino acid at position 154 was different. Specifically, threonine (Thr) at position 154 in SEQ ID NO: 11 was substituted with alanine (Ala).

In the relationship with the phage Xp12 having the highest analogous score to the phage isolated in Example 2, the amino acid sequence was different, in the same manner as with the phage isolated in Example 2, in the amino acid at position 278 and position 350 from the amino acid sequence of this analogous protein. In the phage obtained in this Example, the above-described amino acid at position 154 was additionally different.

As described above in Example 2, *Xanthomonas oryzae* is the only known bacterial species against which the phage Xp12 exhibits bacteriolytic ability, thus suggesting that the bacteriolytic ability against a wide variety of host, as the 2nd phage has, is due to a difference in the amino acid at position 278 and position 350 in the amino acid sequence of the tail fiber protein. Additionally, it has been suggested that even a phage having an additional different amino acid at another position, as the 2nd phage obtained in this Example, does have a wide host range.

### <Example 12: Isolation of Novel 7th Bacteriophage, and Bacteriolytic Ability Thereof>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In the present invention, the plant pathogenic bacteria as target bacteria were bacteria of the *Xanthomonas* genus, and all the bacterial strains were obtained from the National Agriculture and Food Research Organization (NARO). Besides the bacteria of the *Xanthomonas* genus used in Example 7 listed in Table 10, the bacteria used in this Example are listed in Table 15, together with the deposition numbering (MAFF No.) of the National Agriculture and Food Research Organization.

**[Table 15]**

| ID | Species | Source of Isolation | Place of Harvest |
|---|---|---|---|
| MAFF No. 301260 | *Xanthomonas campestris pv. vesicatoria* | Pepper | Tokushima |

As a control, a *Pseudomonas fluorescens* bacterial strain (NCIMB-ID: 10460), which is of *Pseudomonas* genus, was obtained from NCIMB, a laboratory in The United Kingdom National Culture Collection, UKNCC.

Bacteria of the *Xanthomonas* genus and *Pseudomonas fluorescens* were cultured in accordance with the method described in "(1) Obtainment and Culture of Plant Pathogenic Bacteria" in Example 1.

### (2) Isolation and Purification of Phage

A novel phage was isolated from natural dirty water or soil obtained in Japan. A method for isolation and a method for purification were in accordance with the methods described in "(2) Isolation and Purification of 1st Phage" in Example 1. Consequently, one kind of new phage was isolated from natural dirty water and soil.

The phage isolated was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to purify the phage.

### (3) Amplification and Refinement of Phage

The phage isolated and purified was amplified and refined by a plate lysate (PL) method. Specific method was in accordance with the method described in "(3) Amplification and Refinement of 1st Phage" in Example 1. The titer of the refined solution of phage prepared was determined by a plaque assay method using a solution suitably diluted, and was confirmed to be 10⁸ PFU/mL or more.

### (4) Evaluation of Host Range of Phage

The host range of the phage obtained was evaluated by a spot test method. The basic operation was in accordance with the method described in "(4) Evaluation of Host Range of 1st Phage" in Example 1.

One example of the results is shown in Figures 16 and 17. When the phage exhibited bacteriolytic ability, only the site where the refined solution of phage was dropped became clear in the bacterial lawn formed on the plate. The phage obtained in the present invention exhibited bacteriolytic ability against all the bacteria of the *Xanthomonas* genus shown in Table 10 (Figure 16). On the other hand, the phage did not exhibit bacteriolytic ability against *Pseudomonas fluorescens.* This suggests the possibility that the phage isolated are high analogous to the 7th phage. Furthermore, the two kinds of 7th phages obtained in Example 7 and the phage obtained in this Example were examined for bacteriolytic ability against *Xanthomonas campestris pv. vesicatoria.* Consequently, all three kinds of phages exhibited bacteriolytic ability against this bacterial strain (Figure 17).

Additionally, these results suggest that the phage obtained in this Example is useful for control of shot hole disease of *Prunus spp.* and black rot disease of broccoli, which are caused by bacteria of the *Xanthomonas* genus.

### (5) Genomic Analysis of 7th Phage

The genomic DNA sequence of the phage obtained in this Example was determined and analyzed.

### (i) Preparation and Sequencing of Genomic DNA of Phage

The genome of the phage was extracted using a TURBO DNA-free^{™} kit (Thermo Fisher Scientific Inc.). The basic operation was in accordance with the method described in "(5) Genomic Analysis of 1 st Phage, (i) Preparation and Sequencing of Genomic DNA of 1 st Phage" in Example 1.

### (ii) Bioinformatics Analysis based on Genomic Sequence Information

On the basis of the genomic nucleotide sequence (SEQ ID NO: 53) of the phage obtained in (i) above, and using GENETYX-NGS, the average nucleotide identity (ANI) to the genomic nucleotide sequences (SEQ ID NOs: 28 and 29) of the 7th phage was analyzed. Consequently, the nucleotide sequence of SEQ ID NO: 53 had a sequence identity of 92.61% to the nucleotide sequence of SEQ ID NO: 28 in the range of 75.06%. On the other hand, the sequence identity was 92.19% to the nucleotide sequence SEQ ID NO: 29 over the range of 77.05%. It was unexpected that the genomic sequence identity was not extremely high.

To clarify the cause of the identity in host specificity between the phage isolated in this Example and the phage isolated in Example 7, the sequences of the tail tubular proteins of both phages were compared.

First, a gene group encoding a tail tubular protein was identified from the genomic sequence of the phage in this Example. The gene was identified utilizing a BRAST server (https://rast.nmpdr.org/) and a PHASTER server (https://phaster.ca/).

SEQ ID NO: 51 shows the nucleotide sequence of the tail tubular protein A gene comprised in the genomic nucleotide sequence (SEQ ID NO: 53) of the phage obtained in this Example, and SEQ ID NO: 49 shows the amino acid sequence of the tail tubular protein A. On the other hand, SEQ ID NO: 52 shows the nucleotide sequence of the tail tubular protein B gene, and SEQ ID NO: 50 shows the amino acid sequence of the tail tubular protein B.

Furthermore, the amino acid sequences (SEQ ID NOs: 49 and 50) of these tail tubular proteins were compared with the amino acid sequences (SEQ ID NO: 24 and 58) of the tail tubular proteins of the phages isolated in Example 7. Consequently, the sequence identity of the amino acid sequence of the tail tubular protein A was 96.58% and the sequence identity of the amino acid sequence of the tail tubular protein B was 97.60%. Accordingly, the phage obtained in this Example, is referred to as a "7th phage" in addition to the phage obtained in Example 7.

Specifically, as to the amino acid sequence of the tail tubular protein A, seven amino acid substitutions from the amino acid sequence of SEQ ID NO: 24 were observed in the amino acid sequence of SEQ ID NO: 49, as follows: the substitution of glutamic acid (Glu) with aspartic acid (Asp) at position 28, the substitution of serine (Ser) with asparagine (Asn) at position 108, the substitution of glutamine (Gln) with histidine (His) at position 110, the substitution of glutamine (Gln) with lysine (Lys) at position 153, the substitution of aspartic acid (Asp) with asparagine (Asn) at position 157, the substitution of tyrosine (Tyr) with phenyl alanine (Phe) at position 189, and the substitution of valine (Val) with tyrosine (Tyr) at position 201.

On the other hand, as to the amino acid sequence of the tail tubular protein B, 20 amino acid substitutions from the amino acid sequence of SEQ ID NO: 58 were observed in the amino acid sequence of SEQ ID NO: 50, as follows: the substitution of alanine (Ala) with proline (Pro) at position 25, the substitution of alanine (Ala) with serine (Ser) at position 53, the substitution of alanine (Ala) with proline (Pro) at position 216, the substitution of histidine (His) with tyrosine (Tyr) at position 221, the substitution of valine (Val) with (Ile) at position 272, the substitution of alanine (Ala) with glutamic acid (Glu) at position 389, the substitution of serine (Ser) with alanine (Ala) at position 395, the substitution of valine (Val) with isoleucine (Ile) at position 410, the substitution of isoleucine (Ile) with valine (Val) at position 425, the substitution of glycine (Gly) with alanine (Ala) at position 472, the substitution of alanine (Ala) with serine (Ser) at position 607, the substitution of isoleucine (Ile) with valine (Val) at position 623, the substitution of arginine (Arg) with serine (Ser) at position 662, the substitution of leucine (Leu) with glutamine (Gln) at position 670, the substitution of threonine (Thr) with asparagine (Asn) at position 699, the substitution of aspartic acid (Asp) with glycine (Gly) at position 707, the substitution of serine (Ser) with alanine (Ala) at position 757, the substitution of glycine (Gly) with serine (Ser) at position 763, the substitution of serine (Ser) with asparagine (Asn) at position 764, and the substitution of methionine (Met) with valine (Val) at position 765.

The relationship with the phages ΦXc10 and f20-Xaj having the highest analogous score to the amino acid sequences of the tail tubular proteins of the phages isolated in Example 7 was examined. Consequently, as to the tail tubular protein A, SEQ ID NOs: 24 and 49 are encompassed in the amino acid sequence of SEQ ID NO: 60, but there was no known phage encompassed in the amino acid sequence of SEQ ID NO: 60, including ΦXc10 and f20-Xaj. Additionally, as to the tail tubular protein B, SEQ ID NOs: 58 and 50 are encompassed in the amino acid sequence of SEQ ID NO: 61, but there was no known phage encompassed in the amino acid sequence of SEQ ID NO: 61, including ΦXc10 and f20-Xaj.

As described above in Example 7, the phages ΦXc10, f20-Xaj, and the like were not phages exhibiting bacteriolytic ability against *Xanthomonas arboricola pv. pruni* and the like, thus suggesting that the bacteriolytic ability peculiar to the 7th phage is due to a difference in the amino acid sequence of the tail tubular protein. Additionally, it has been suggested that even if the amino acid sequence of the tail tubular protein has a little difference as in the 7th phage obtained in this Example, the phage has the same host range, depending on the position of the difference.

### <Example 13: Isolation of Novel 9th Bacteriophage, and Bacteriolytic Ability Thereof>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In the present invention, the plant pathogenic bacteria as target bacteria were bacteria of the *Xanthomonas* genus, and all the bacterial strains were obtained from the National Agriculture and Food Research Organization (NARO). In this Example, the bacteria of the *Xanthomonas* genus listed in Table 13 were used.

As a control, a *Pseudomonas fluorescens* bacterial strain (NCIMB-ID: 10460) that is reported to have a plant growth promoting action and the like, and is beneficial to the environment was obtained from NCIMB, a laboratory in The United Kingdom National Culture Collection, UKNCC.

Each of bacteria of the *Xanthomonas* genus and *Pseudomonas fluorescens* were cultured in accordance with the method described in "(1) Obtainment and Culture of Plant Pathogenic Bacteria" in Example 1.

### (2) Isolation and Purification of Phage

A novel phage was isolated from natural dirty water, soil, or part of a plant (leaf) obtained in Japan. A method for isolation and a method for purification were in accordance with the methods described in "(2) Isolation and Purification of 1st Phage" in Example 1. Consequently, one kind of new phage was isolated from natural dirty water or soil or water having part of a plant immersed therein.

The phage isolated was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to purify the phage.

### (3) Amplification and Refinement of Phage

The phage isolated and purified was amplified and refined by a plate lysate (PL) method. Specific method was in accordance with the method described in "(3) Amplification and Refinement of 1st Phage" in Example 1. The titer of the refined solution of phage prepared was determined by a plaque assay method using a solution suitably diluted, and was confirmed to be 10⁸ PFU/mL or more.

### (4) Evaluation of Host Range of Phage

The host range of the phage obtained was evaluated by a spot test method. The basic operation was in accordance with the method described in "(4) Evaluation of Host Range of 1st Phage" in Example 1.

One example of the results is shown in Figure 18. When the phage exhibited bacteriolytic ability, only the site where the refined solution of phage was dropped became clear in the bacterial lawn formed on the plate. The phage obtained in the Example exhibited bacteriolytic ability against all the bacterial strains belonging to the three species of bacteria: *Xanthomonas arboricola, Xanthomonas citri,* and *Xanthomonas campestris* as with the phages obtained in Example 9 (Figure 18). Additionally, it has been verified that the phages exhibit bacteriolytic ability against bacterial strains of different pathovars. On the other hand, it has also been verified that the phage does not exhibit bacteriolytic ability against *Pseudomonas fluorescens.* This suggests the possibility that the phage isolated is highly analogous to the 9th phage.

Additionally, these results suggest that the phage obtained in this Example is useful for control of, for example, shot hole disease of *Prunus spp.,* orange bacterial canker, black rot disease of broccoli, and the like, which are caused by bacteria of the *Xanthomonas* genus.

### (5) Genomic Analysis of Phage

The genomic DNA sequence of the phage obtained was determined and analyzed.

### (i) Preparation and Sequencing of Genomic DNA of Phage

The genome of the phage was extracted using a TURBO DNA-free^{™} kit (Thermo Fisher Scientific Inc.). The basic operation was in accordance with the method described in "(5) Genomic Analysis of 1 st Phage, (i) Preparation and Sequencing of Genomic DNA of 1 st Phage" in Example 1.

### (ii) Bioinformatics Analysis based on Genomic Sequence Information

On the basis of the genomic nucleotide sequence (SEQ ID NO: 56) of the phage obtained in (i) above, and using GENETYX-NGS, the average nucleotide identity (ANI) to the genomic nucleotide sequence (SEQ ID NO: 41) of the 9th phage was analyzed. Consequently, the nucleotide sequence of SEQ ID NO: 56 had a sequence identity of 92.61% to the nucleotide sequence of SEQ ID NO: 41 in the range of 75.06%. It was unexpected that the genomic sequence identity was not extremely high.

To clarify the cause of the identity in host specificity between the phage isolated in this Example and the phage isolated in Example 9, the sequences of the tail tubular proteins of both phages were compared.

First, a gene group encoding a tail tubular protein was identified from the genomic sequence of the phage in this Example. The gene was identified utilizing a BRAST server (https://rast.nmpdr.org/) and a PHASTER server (https://phaster.ca/).

The nucleotide sequence of the tail tubular protein A gene, comprised in the genomic nucleotide sequence (SEQ ID NO: 56) of the phage obtained in this Example, was completely the same as the nucleotide sequence (SEQ ID NO: 39) of the tail tubular protein A gene of the phage isolated in Example 9. Accordingly, the amino acid sequence of the tail tubular protein A resulting from the translation was the same as the amino acid sequence (SEQ ID NO: 37) of the tail tubular protein A of the phage isolated in Example 9. Therefore, the phage obtained in this Example, is referred to as a "9th phage" in addition to the phage obtained in Example 9.

On the other hand, SEQ ID NO: 55 shows the nucleotide sequence of the tail tubular protein B gene, and SEQ ID NO: 54 shows the amino acid sequence of the tail tubular protein B.

Furthermore, the amino acid sequence (SEQ ID NO: 54) of the tail tubular protein B was compared with the amino acid sequence (SEQ ID NO: 38) of the tail tubular protein B of the phages isolated in Example 9. The sequence identity of the amino acid sequence of the tail tubular protein B was 99.3%. Specifically, six amino acid substitutions from the amino acid sequence of SEQ ID NO: 38 in the amino acid sequence of SEQ ID NO: 54 were observed as follows: the substitution of threonine (Thr) with alanine (Ala) at position 61, the substitution of glutamine (Gln) with lysine (Lys) at position 108, the substitution of proline (Pro) with glutamine (Gln) at position 404, the substitution of proline (Pro) with serine (Ser) at position 679, the substitution of threonine (Thr) with alanine (Ala) at position 682, and the substitution of isoleucine (Ile) with valine (Val) at position 727.

As described above in Example 9, there was no known phage having a sequence identity of 97% or more to the amino acid sequences of the tail tubular proteins A and B of the 9th phage obtained in Example 9. Additionally, it has not been known that the phage *Xanthomonas* phage Xaa _vB_phi31 and the like that made a high analogous score to the 9th phage exhibit bacteriolytic ability against a wide range of various bacterial species of *Xanthomonas* genus as with the 9th phage. This suggests that the wide bacteriolytic ability of the 9th phage is due to a difference in the amino acid sequence of the tail tubular protein. Additionally, it has been suggested that even if the amino acid sequence of the tail tubular protein has a little difference, as in the 9th phage obtained in this Example, the phage has the same host range.

### <Example 14: Bacteriolytic Ability of Combination of Bacteriophages Obtained>

### (Purpose)

The purpose is to examine how novel bacteriophages shown to have bacteriolytic ability against the pathogenic bacteria of a plant disease when used alone have an effect on the plant disease when used in combination.

### (Method)

### (1) Spot Test Method

A spot test was performed in accordance with the method described in "(4) Evaluation of Host Range of 1st Phage" in Example 1. In this Example, a bacterial strain *of Xanthomonas arboricola pv. pruni* (MAFF No. 311351), against which all the phages tested were confirmed to have bacteriolytic ability, was used.

As a refined solution of phage to be dropped, a solution mixture obtained by mixing equal amounts of the refined solutions of phage prepared in each Example was used. The solution mixture was prepared by being suitably diluted so that the total titer could be the same as when the solution was used alone. The phages used in this Example are as below.

In this Example, each of the following phages is used: the phage having the genomic DNA sequence of SEQ ID NO: 8 as the 1st phage, the phage with the genomic DNA sequence of SEQ ID NO: 13 as the 2nd phage, the phage with the genomic DNA sequence of SEQ ID NO: 14 as the 3rd phage, the phage with the genomic DNA sequence of SEQ ID NO: 17 as the 4th phage, the phage with the genomic DNA sequence of SEQ ID NO: 18 as the 5th phage, the phage with the genomic DNA sequence of SEQ ID NO: 23 as the 6th phage, the phage with the genomic DNA sequence of SEQ ID NO: 28 as the 7th phage, the phage with the genomic DNA sequence of SEQ ID NO: 32 as the 8th phage, the phage with the genomic DNA sequence of SEQ ID NO: 41 as the 9th phage, and the phage with the genomic DNA sequence of SEQ ID NO: 44 as the 10th phage.

### (Results)

One example of the results is shown in Figures 19 and 20. When a combination of the phages exhibited bacteriolytic ability, only the site where the refined solution of phage was dropped became clear in the bacterial lawn formed on the plate. Using any of the combinations of two kinds, five kinds, and eight kinds tested has been revealed to exhibit bacteriolytic ability at least in the same manner as using each phage alone (Figures 19 and 20).

The results have verified that phages of the present invention are useful when used in combination as well as when used alone.

### <Example 15: Disease Control Effect Test on Shot Hole Disease of Prunus spp.>

### (Purpose)

The purpose is to examine how isolated novel bacteriophages with bacteriolytic ability against the pathogenic bacteria of a plant disease have an effect on the plant disease when applied to a plant.

### (Method)

### (1) Preparation of Spreading Solution of Phage

A spreading solution of phage used in this test was prepared in accordance with the following procedure.

First, a bacterial cell culture solution of host bacteria for amplifying phages was prepared in accordance with the following procedure. As the host, a bacterial strain of *Xanthomonas arboricola pv. pruni* (MAFF No. 311351), against which all the phages tested were confirmed to have bacteriolytic ability, was used. The bacterial cell of this strain was inoculated into YPG Broth and incubated overnight with a shaker set at 25°C. After the incubation, OD₆₀₀(a turbidity at a wavelength of 600 nm) was measured, and a bacterial cell culture solution, the turbidity of which became approximately 1.0, was used below.

The bacterial cell culture solution prepared and a refined solution of phage containing one kind of phage (having a titer of approximately 10⁸ PFU/mL) prepared in each of Examples 1 to 13 were mixed in equal amounts. The resulting mixture solution was inoculated into a 100-fold amount of YPG culture solution and incubated for approximately 8 to 12 hours with a shaker set at 25°C. The resulting culture solution was collected as a crude solution of phage. To the crude solution collected, a 1/10 amount of chloroform was added, stirred vigorously, and then centrifuged under 8,000 × g at 20°C for 5 minutes. The supernatant was then collected. The collected supernatant was passed through a 0.2 µm filter, and the resulting filtrate was used as a refined solution of phage.

As a spreading solution of phage comprising one kind of phage, a refined solution of phage diluted with sterile tap water so as to have a titer of approximately 10⁹ PFU/mL was used.

For a spreading solution of phage comprising a plurality of kinds of phages, refined solutions of phage adjusted so as to each have a titer at the same level (order), mixed in equal amounts, and diluted with sterile tap water so as to have a titer of approximately 10⁹ PFU/mL were used. Each of the following phages is used: the phage with the genomic DNA sequence of SEQ ID NO: 8 as the 1st phage, the phage with the genomic DNA sequence of SEQ ID NO: 13 as the 2nd phage, the phage with the genomic DNA sequence of SEQ ID NO: 14 as the 3rd phage, the phage with the genomic DNA sequence of SEQ ID NO: 17 as the 4th phage, the phage with the genomic DNA sequence of SEQ ID NO: 18 as the 5th phage, the phage with the genomic DNA sequence of SEQ ID NO: 23 as the 6th phage, the phage with the genomic DNA sequence of SEQ ID NO: 28 as the 7th phage, and the phage with the genomic DNA sequence of SEQ ID NO: 32 as the 8th phage.

### (2) Preparation of Spreading Solution of Bacteria

To prepare a spreading solution of bacteria used to infect a plant in this test, the bacterial cell culture solution prepared in (1) was used. A bacterial cell culture solution diluted approximately 10,000-fold with sterile tap water was applied to a YPG Agar plate, and incubated for approximately 1 to 3 days in an incubator set at 25°C. A bacterial cell suspension, in which the colony on the YPG Agar plate was collected by suspending in sterile tap water, was diluted with sterile tap water so as a final OD₆₀₀ to be approximately 0.5, and the resulting solution was used as a spreading solution of bacteria.

### (3) Plant Specimen

A commercially available peach seedling (the breed: Kawanakajima, one-year old) was grown in a greenhouse, and the seedling grown to have approximately 100 leaves was used as a specimen for evaluation.

### (4) Application and Infection of Phage

With the phage-applied group, foliar application of the spreading solution of phage to each specimen was performed twice each, twice at intervals of 2 to 3 days, before and after the bacterial infection, and was followed by foliar application of the spreading solution of bacteria 2 to 3 days later, whereby the specimen was infected with the bacteria. Over an approximately 2-day period after the infection treatment, the specimen infected with the bacteria was left in a vinyl house with a high humidity being kept. Then, another foliar application of the spreading solution of phage, twice with an interval of two to three days, was performed again. For a nontreated group, the same procedure as described above was performed except that sterile tap water was used instead of a refined solution of phage.

### (5) Measurement of Incidence Rate

When a certain incidence of disease was verified with the nontreated group after one week or more from the infection treatment, the nontreated group and each phage-applied group were examined for the incidence rate.

A leaf, on the face of which a brown spot characteristic of a shot hole disease of *Prunus spp.* was developed, was judged as a diseased leaf. The ratio of the number of diseased leaves out of the number of all the leaves was calculated as an incidence rate. The relative incidence rate of the phage-applied group was calculated as a relative value, with the incidence rate of the nontreated group assumed to be 100%.

### (Results)

The results are shown in Figures 21 and 22. The incidence rate of the nontreated group was approximately 36%. On the other hand, the relative incidence rate, with the incidence rate of the nontreated group assumed to be 100%, was approximately 50% on average when the spreading solution of phage comprising one kind of phage was applied. When the phage was applied, even the highest relative incidence rate was approximately 61% (with the phage-applied group for the 6th phage and the phage-applied group for the 8th phage). On the other hand, the relative incidence rate was the lowest, approximately 24%, with the phage-applied group for the 7th phage.

Furthermore, when the spreading solution of phage comprising two kinds or more of phages was applied, the incidence rate was further decreased, compared to the case when the spreading solution of phage comprising one kind of phage was applied. Here, the incidence rate of the nontreated group was approximately 15%. When phages were applied in combination (a combination of the 2nd phage, 4th phage, 6th phage, and 8th phage), even the highest relative incidence rate was approximately 41%. On the other hand, the relative incidence rate was the lowest, approximately 34%, with a combination of the 1st phage and the 2nd phage.

The results have revealed that, even when applied to an actual plant, a phage of the present invention can effectively control the plant away from a disease. Additionally, applying the phages in combination has been revealed to afford a higher effect. Furthermore, it has been revealed that the disease control effect of a phage of the present invention is also effective under conditions where the incidence rate in the nontreated group is low.

Common plant protectives comprising antibiotics cause drug poisoning in a leaf, such as the etiolation of a leaf apex, in some cases. However, in the phage-applied group, there was no significant adverse effect found on any plant that could be attributed to the phage, for example, such drug poisoning as appears on the leaf. This has revealed that applying a phage of the present invention is an effective disease control means with a smaller side effect.

### <Example 16: Disease Control Effect Test on Black Rot Disease of Broccoli>

### (Purpose)

The purpose is to examine how isolated novel bacteriophages with bacteriolytic ability against the pathogenic bacteria of a plant disease have an effect on the plant disease when applied to a plant.

### (Method)

### (1) Preparation of Spreading Solution of Phage

A spreading solution of phage was prepared in accordance with Example 15, except that the bacteria used as a bacterial cell was *Xanthomonas campestris pv. campestris* (MAFF No. 106765),and that the below-described phages were used.

The phages used in this Example were as follows: the phage with the genomic DNA sequence of SEQ ID NO: 7 (Φ1 in Figure 23) as the 1st phage, the phage with the genomic DNA sequence of SEQ ID NO: 13 (Φ2-1 in Figure 23) and the phage with the genomic DNA sequence of SEQ ID NO: 47 (Φ2-2 in Figure 23) as the 2nd phages, the phage with the genomic DNA sequence of SEQ ID NO: 28 (Φ7-1 in Figure 23) and the phage with the genomic DNA sequence of SEQ ID NO: 53 (Φ7-2 in Figure 23) as the 7th phages, the phage with the genomic DNA sequence of SEQ ID NO: 41 (Φ9 in Figure 23) as the 9th phage, and the phage with the genomic DNA sequence of SEQ ID NO: 44 (Φ10 in Figure 23) as the 10th phage, respectively.

### (2) Preparation of Spreading Solution of Bacteria

A spreading solution of phage was prepared in accordance with Example 15, except that the bacterial cells were the above-described bacteria.

### (3) Plant Specimen

Commercially available seeds of broccoli were seeded, and grown in a greenhouse. A seedling grown to have five or more leaves was used as a specimen for evaluation.

### (4) Application and Infection of Phage

This was performed in accordance with Example 15.

### (5) Measurement of Incidence Rate

This was performed the same as in Example 15, except that an evaluation was made 16 days after the infection treatment.

### (Results)

The results are shown in Figure 23. The incidence rate of the nontreated group was approximately 52%. On the other hand, the relative incidence rate, with the incidence rate of the nontreated group assumed to be 100%, was approximately 62% on average when the spreading solution of phage comprising one kind of phage was applied. When the phage was applied, even the highest relative incidence rate was approximately 66% (with the group to which the phage with the genomic DNA sequence of SEQ ID NO: 28 (Φ7-1 in Figure 23) was applied as the 7th phage). On the other hand, the relative incidence rate was the lowest, approximately 56%, with the group to which the phage having the genomic DNA sequence of SEQ ID NO: 47 was applied as the 2nd phage (Φ2-2 in Figure 23).

These results have verified that the 9th phage and the 10th phage exhibit bacteriolytic ability even when the specific bacterial strain was different from a bacterial strain belonging to *Xanthomonas campestris pv. campestris,* bacteriolytic ability against which was verified in Examples 9 and 10, respectively.

Furthermore, when the spreading solution of phage comprising two kinds or more of phages was applied, the incidence rate was further decreased to approximately 53% on average. The highest relative incidence rate was approximately 58% when a combination of the 9th phage (Φ9 in Figure 23) and the 10th phage (Φ10 in Figure 23) was used. On the other hand, when a total of four kinds of phages, the 1st phage (Φ1 in Figure 23), two kinds of 2nd phages (Φ2-1 and Φ2-2 in Figure 23), and the 10th phage (Φ10 in Figure 23), were used in combination, the relative incidence rate was the lowest, approximately 47%.

The results have revealed that, even when applied to an actual plant, a phage of the present invention can effectively control the plant away from a disease, regardless of the kind of the plant itself. Additionally, applying the phages of the present invention in combination has been revealed to afford a higher effect.

In the phage-applied group, there was no significant adverse effect found on any plant that could be attributed to the phage, such as drug poisoning found in a leaf. This has revealed that applying a phage of the present invention is an effective disease control means with a smaller side effect.

### <Example 17: Disease Control Effect Test on Bacterial Spot Disease of Tomato>

### (Purpose)

The purpose is to examine how novel isolated bacteriophages with bacteriolytic ability against the pathogenic bacteria of a plant disease have an effect on the plant disease when applied to a plant.

### (Method)

### (1) Preparation of Spreading Solution of Phage

A spreading solution of phage was prepared in accordance with Example 15, except that the bacteria used as a bacterial cell was *Xanthomonas campestris pv. vesicatoria* (MAFF No. 301256), and that the below-described phages were used.

The phages used in this Example were as follows: the phage with the genomic DNA sequence of SEQ ID NO: 7 (Φ1 in Figure 24) as the 1st phage, the phage with the genomic DNA sequence of SEQ ID NO: 13 (Φ2-1 in Figure 24), the phage with the genomic DNA sequence of SEQ ID NO: 47 (Φ2-2 in Figure 24), and the phage with the genomic DNA sequence of SEQ ID NO: 48 (Φ2-3 in Figure 24) as the 2nd phages, the phage with the genomic DNA sequence of SEQ ID NO: 28 (Φ7 in Figure 24) as the 7th phage, and the phage with the genomic DNA sequence of SEQ ID NO: 44 (Φ10 in Figure 24) as the 10th phage, respectively.

### (2) Preparation of Spreading Solution of Bacteria

A spreading solution of phage was prepared in accordance with Example 15, except that the bacterial cells were the above-described bacteria.

### (3) Plant Specimen

Commercially available seeds of tomatoes were seeded and grown in a greenhouse. A seedling grown to have 50 or more leaves was used as a specimen for evaluation.

### (4) Application and Infection of Phage

This was performed in accordance with Example 15.

### (5) Measurement of Incidence Rate

This was performed the same as in Example 15, except that an evaluation was made 16 days after the infection treatment.

### (Results)

The results are shown in Figure 24. The incidence rate of the nontreated group was approximately 25%. On the other hand, the relative incidence rate, with the incidence rate of the nontreated group assumed to be 100%, was approximately 60% on average when the spreading solution of phage comprising one kind of phage was applied. When the phage was applied, even the highest relative incidence rate was approximately 66% (with the group to which the phage with the genomic DNA sequence of SEQ ID NO: 47 (Φ2-2 in Figure 24) was applied as the 2nd phage). On the other hand, the relative incidence rate was the lowest, approximately 53%, with the group to which the 7th phage (Φ7 in Figure 24) was applied.

These results have verified that the 7th phage and the 10th phage exhibit bacteriolytic ability even when the specific bacterial strain was different from a bacterial strain belonging to *Xanthomonas campestris pv. vesicatoria,* bacteriolytic ability against which was verified in Examples 12 and 10, respectively.

Furthermore, when the spreading solution of phage comprising two or more kinds of phages was applied, the incidence rate was further decreased to approximately 49% on average. The highest relative incidence rate was approximately 55% when a combination of the phage with the genomic DNA sequence of SEQ ID NO: 48 (Φ2-3 in Figure 24) as the 2nd phage and the 7th phage (Φ7 in Figure 24) was used. On the other hand, when a total of four kinds of phages, the 1st phage (Φ1 in Figure 24), two kinds of 2nd phages (Φ2-1 and Φ2-2 in Figure 24), and the 10th phage (Φ10 in Figure 24), were used in combination, the relative incidence rate was the lowest, approximately 43%.

The results have revealed that, even when applied to an actual plant, a phage of the present invention can effectively control the plant away from a disease, regardless of the kind of the plant itself. Additionally, applying the phages in the present invention of combination has been revealed to afford a higher effect.

In the applied-phage group, there was no significant adverse effect found on any plant that could be attributed to the phage, such as drug poisoning found in a leaf. This has revealed that applying a phage of the present invention is an effective disease control means with a smaller side effect.

All publications, patents, and patent applications cited herein are incorporated herein by reference in their entirety.

## Claims

1. A bacteriolytic agent consisting of a bacteriophage comprising, in a genomic DNA, a gene encoding a tail fiber protein having a recognizing ability for target bacteria, wherein the tail fiber protein consists of an amino acid sequence of any one of the following (a) to (d):
(a) an amino acid sequence of any one selected from the group consisting of SEQ ID NOs: 11, 45, 42, and 1;
(b) an amino acid sequence having addition, deletion, and/or substitution of one or a plurality of amino acids in the amino acid sequence of SEQ ID NO: 42 or 1;
(c) an amino acid sequence having a sequence identity of 90% or more to the amino acid sequence of SEQ ID NO: 42 or 1; or
(d) an amino acid sequence having substitution of one amino acid other than at positions 278 and 350 in the amino acid sequence of SEQ ID NO: 11.

2. A bacteriolytic agent consisting of a bacteriophage comprising, in a genomic DNA, the following: a gene consisting of an amino acid sequence of any one of the following (e) to (g); and a gene consisting of an amino acid sequence of any one of the following (h) to (j),
wherein the genes are respectively a gene encoding a tail tubular protein A and a gene encoding a tail tubular protein B, both proteins having a recognizing ability for target bacteria:
(e) an amino acid sequence of SEQ ID NO: 24, 49, or 60;
(f) an amino acid sequence having addition, deletion, and/or substitution of one or a plurality of amino acids in the amino acid sequence of SEQ ID NO: 24 or 49;
(g) an amino acid sequence having a sequence identity of 97% or more to the amino acid sequence of SEQ ID NO: 24 or 49;
(h) an amino acid sequence of SEQ ID NO: 57, 50, or 61;
(i) an amino acid sequence having addition, deletion, and/or substitution of one or a plurality of amino acids in the amino acid sequence of SEQ ID NO: 57 or 50; or
(j) an amino acid sequence having a sequence identity of 97% or more to the amino acid sequence of SEQ ID NO: 57 or 50.

3. A bacteriolytic agent consisting of a bacteriophage comprising, in a genomic DNA, the following: a gene consisting of an amino acid sequence of any one of the following (k) to (m); and
a gene consisting of an amino acid sequence of any one of the following (n) to (p), wherein the genes are respectively a gene encoding a tail tubular protein A and a gene encoding a tail tubular protein B, both proteins having a recognizing ability for target bacteria:
(k) an amino acid sequence of SEQ ID NO: 37;
(1) an amino acid sequence having addition, deletion, and/or substitution of one or a plurality of amino acids in the amino acid sequence of SEQ ID NO: 37;
(m) an amino acid sequence having a sequence identity of 92% or more to the amino acid sequence of SEQ ID NO: 37;
(n) an amino acid sequence of SEQ ID NO: 38, 54, or 59;
(o) an amino acid sequence having addition, deletion, and/or substitution of one or a plurality of amino acids in the amino acid sequence of SEQ ID NO: 38 or 54; or
(p) an amino acid sequence having a sequence identity of 92% or more to the amino acid sequence of SEQ ID NO: 38 or 54.

4. The bacteriolytic agent according to claim 1, wherein the gene consists of a nucleotide sequence of any one of the following (1) to (3):
(1) a nucleotide sequence of any one selected from the group consisting of SEQ ID NOs: 12, 46, 43, and 5 to 7;
(2) a nucleotide sequence having addition, deletion, and/or substitution of one or a plurality of nucleotides in the nucleotide sequence of any one selected from the group consisting of SEQ ID NOs: 43 and 5 to 7; or
(3) a nucleotide sequence having a sequence identity of 90% or more to the nucleotide sequence of any one selected from the group consisting of SEQ ID NOs: 43 and 5 to 7.

5. The bacteriolytic agent according to claim 4, wherein a nucleotide sequence of the genomic DNA consists of the nucleotide sequence of any one of the following (1') to (7'):
(1') a nucleotide sequence of any one selected from the group consisting of SEQ ID NOs: 13, 47, 48, 44, and 8 to 10;
(2') a nucleotide sequence having addition, deletion, and/or substitution of one or a plurality of nucleotides in the nucleotide sequence other than the gene according to claim 4 within the nucleotide sequence of any one selected from the group consisting of SEQ ID NOs: 13, 47, 48, 44, and 8 to 10;
(3') a nucleotide sequence having a sequence identity of 80% or more to the nucleotide sequence other than the gene according to claim 4 within the nucleotide sequence of any one selected from the group consisting of SEQ ID NOs: 44 and 8 to 10;
(4') a nucleotide sequence having a sequence identity of 98.5% or more to the nucleotide sequence other than the gene according to claim 4 within the nucleotide sequence of SEQ ID NO: 13, 47, or 48;
(5') a nucleotide sequence having addition, deletion, and/or substitution of one or a plurality of nucleotides in the nucleotide sequence of any one selected from the group consisting of SEQ ID NOs: 13, 47, 48, 44, and 8 to 10;
(6') a nucleotide sequence having a sequence identity of 90% or more to the nucleotide sequence of any one selected from the group consisting of SEQ ID NOs: 44 and 8 to 10;
(7') a nucleotide sequence having a sequence identity of 98.5% or more to the nucleotide sequence of SEQ ID NO: 13, 47, or 48.

6. The bacteriolytic agent according to claim 2, wherein a nucleotide sequence of the genomic DNA consists of the nucleotide sequence of any one of the following (8') to (12'):
(8') a nucleotide sequence of SEQ ID NO: 28, 29, or 53;
(9') a nucleotide sequence having addition, deletion, and/or substitution of one or a plurality of nucleotides in the nucleotide sequence other than the gene according to claim 2 within the nucleotide sequence of SEQ ID NO: 28, 29, or 53;
(10') a nucleotide sequence having a sequence identity of 95% or more to the nucleotide sequence other than the gene according to claim 2 within the nucleotide sequence of SEQ ID NO: 28, 29, or 53;
(11') a nucleotide sequence having addition, deletion, and/or substitution of one or a plurality of nucleotides in the nucleotide sequence of SEQ ID NO: 28, 29, or 53; or
(12') a nucleotide sequence having a sequence identity of 95% or more to the nucleotide sequence of SEQ ID NO: 28, 29, or 53.

7. The bacteriolytic agent according to claim 3, wherein a nucleotide sequence of the genomic DNA consists of the nucleotide sequence of any one of the following (13') to (17'):
(13') a nucleotide sequence of SEQ ID NO: 41 or 56;
(14') a nucleotide sequence having addition, deletion, and/or substitution of one or a plurality of nucleotides in the nucleotide sequence other than the gene according to claim 3 within the nucleotide sequence of SEQ ID NO: 41 or 56;
(15') a nucleotide sequence having a sequence identity of 80% or more to the nucleotide sequence other than the gene according to claim 3 within the nucleotide sequence of SEQ ID NO: 41 or 56;
(16') a nucleotide sequence having addition, deletion, and/or substitution of one or a plurality of nucleotides in the nucleotide sequence of SEQ ID NO: 41 or 56; or
(17') a nucleotide sequence having a sequence identity of 90% or more to the nucleotide sequence of SEQ ID NO: 41 or 56.

8. The bacteriolytic agent according to any one of claims 1 to 7, wherein the bacteriolytic agent exhibits bacteriolytic ability against bacteria of *Xanthomonas* genus.

9. A composition comprising one or more of the bacteriolytic agents according to any of claims 1 to 8 as an active component(s).

10. A composition for plant disease control, comprising the composition according to claim 9.

11. The composition for plant disease control according to claim 10, wherein the plant disease is caused by bacteria *of Xanthomonas* genus.

12. A method for controlling plant disease, comprising a contacting step of contacting the composition for plant disease control according to claim 10 or 11 to a target plant.

13. A method for identifying bacteria *of Xanthomonas* genus, comprising:
a culturing step of culturing subject bacteria isolated from a plant tissue affected by a plant disease, to obtain a culture preparation;
a mixing step of mixing the culture preparation and the bacteriolytic agent according to any one of claims 1 to 8, to obtain a mixture;
a mixture culturing step of culturing the mixture under predetermined conditions; and
a determining step of determining that the subject bacteria is a bacteria *of Xanthomonas* genus, when the subject bacteria are lysed after the mixture culturing step.

14. The method according to claim 13, wherein, in the mixture culturing step, the mixture further comprises a soft agar containing liquid medium, and the mixture is cultured on a solid medium.

15. The method according to claim 13, wherein, in the culturing step, the culture preparation comprises a soft agar containing liquid medium, and the culture preparation is cultured on a solid medium.
